(19) **Europäisches Patentamt** / **European Patent Office** / **Office européen des brevets**

(11) **EP 4 300 500 B1**

(12) **EUROPEAN PATENT SPECIFICATION**

(45) Date of publication and mention
of the grant of the patent:
**09.04.2025   Bulletin 2025/15**

(21) Application number: **23205094.8**

(22) Date of filing: **19.12.2019**

(51) International Patent Classification (IPC):
$C12Q\ 1/68^{(2018.01)}$         $G01N\ 33/574^{(2006.01)}$
$C12Q\ 1/6886^{(2018.01)}$      $C12Q\ 1/6883^{(2018.01)}$
$G16B\ 20/00^{(2019.01)}$

(52) Cooperative Patent Classification (CPC):
**C12Q 1/6886; C12Q 1/6883; G16B 20/00;**
G16H 10/40; G16H 50/30

(54) **CELL-FREE DNA END CHARACTERISTICS**

ZELLFREIE DNA-ENDCHARAKTERISTIKA

CARACTÉRISTIQUES D'EXTRÉMITÉ D'ADN ACELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2018   US 201862782316 P**

(43) Date of publication of application:
**03.01.2024   Bulletin 2024/01**

(60) Divisional application:
**25161060.6**

(62) Document number(s) of the earlier application(s) in accordance with Art. 76 EPC:
**19898588.9 / 3 899 018**

(73) Proprietors:
• **The Chinese University of Hong Kong**
  **Shatin, New Territories  999077 (HK)**
• **Grail, Inc.**
  **Menlo Park, California 94025 (US)**

(72) Inventors:
• **LO, Yuk-Ming Dennis**
  **Shatin, New Territories (HK)**
• **CHIU, Rossa Wai Kwun**
  **Shatin, New Territories (HK)**
• **CHAN, Kwan Chee**
  **Shatin, New Territories (HK)**
• **JIANG, Peiyong**
  **Shatin, New Territories (HK)**
• **CHAN, Wing Yan**
  **Shatin, New Territories (HK)**
• **SUN, Kun**
  **Shatin, New Territories (HK)**

(74) Representative: **J A Kemp LLP**
**80 Turnmill Street**
**London EC1M 5QU (GB)**

(56) References cited:
**WO-A1-2018/009723      WO-A1-2018/081130
WO-A2-2016/015058**

**Description**

This application is a PCT of and claims the benefit of U.S. Provisional Patent Application No. 62/782,316, entitled "CELL-FREE DNA END CHARACTERISTICS," filed on December 19, 2018.

Plasma DNA is believed to consist of cell-free DNA shed from multiple tissues in the body, including but not limited to, hematopoietic tissues, brain, liver, lung, colon, pancreas and so on (Sun et al, Proc Natl Acad Sci USA. 2015;112:E5503-12; Lehmann-Werman et al, Proc Natl Acad Sci USA. 2016; 113: E1826-34; Moss et al, Nat Commun. 2018; 9: 5068). Plasma DNA molecules (a type of cell-free DNA molecules) have been demonstrated to be generated through a non-random process, for example, its size profile showing 166-bp major peaks and 10-bp periodicities occurring in the smaller peaks (Lo et al, Sci Transl Med. 2010;2:61ra91; Jiang et al, Proc Natl Acad Sci USA. 2015;112:E1317-25).

Most recently, it was reported that a subset of human genomic locations (e.g., positions on a reference genome) are preferentially cut, thereby generating plasma DNA fragment having end positions that bear a relationship with the tissue of origin (Chan et al, Proc Natl Acad Sci USA. 2016;113:E8159-8168; Jiang et al, Proc Natl Acad Sci USA. 2018; doi: 10.1073/pnas.1814616115). Chandrananda et al (BMC Med Genomics. 2015; 8: 29) used the *de novo* discovery software DREME (Bailey, Bioinformatics. 2011;27:1653-9) to mine the cell-free DNA data for motifs related to nuclease cleavage, irrespective of tissue type.

WO2018081130 describes a method for screening tumor in a subject by sequencing the cfDNA from a sample of the subject and determine the number of sequencing reads according to size. The document also refers to determining the end position of the reads on the genomic sequences with the aim to determine the pathology level in an individual. ¶
According to the present invention, there is provided a method of estimating a fractional concentration of clinically-relevant DNA in a biological sample of a subject as defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

The present disclosure describes techniques for measuring quantities (e.g., relative frequencies) of sequence end motifs of cell-free DNA fragments in a biological sample of an organism for measuring a property of the sample (e.g., fractional concentration of clinically-relevant DNA) and/or determining a condition of the organism based on such measurements. Different tissue types exhibit different patterns for the relative frequencies of the sequence end motifs. The present disclosure provides various uses for measures of the relative frequencies of sequence end motifs of cell-free DNA, e.g., in mixtures of cell-free DNA from various tissues. DNA from one of such tissue may be referred to as clinically-relevant DNA.

Various examples can quantify amounts of sequence motifs (end motifs) representing an end sequence of DNA fragments. For example, embodiments can determine relative frequencies of a set of sequence motifs for ending sequences of DNA fragments. In various implementations, preferred sets of end motifs and/or patterns of end motifs can be determined using a genotypic (e.g., a tissue-specific allele) or a phenotypic approach (e.g., using samples that have a same condition). The relative frequencies of a preferred set or having a particular pattern can be used to measure a classification of a property (e.g., fractional concentration of clinically-relevant DNA) of a new sample or a condition (e.g., a gestational age of a fetus or a level of pathology) of the organism. Accordingly, embodiments can provide measurements to inform physiological alterations, including cancers, autoimmune diseases, transplantation, and pregnancy.

As further examples, sequence end motifs can be used in a physical enrichment and/or an in silico enrichment of a biological sample for cell-free DNA fragments that are clinically-relevant. The enrichment can use sequence end motifs that are preferred for a clinically-relevant tissue, such as fetal, tumor, or transplant. The physical enrichment can use one or more probe molecules that detect a particular set of sequence end motifs such that the biological sample is enriched for clinically-relevant DNA fragments. For the in silico enrichment, a group of sequence reads of cell-free DNA fragments having one of a set of preferred ending sequences for clinically-relevant DNA can be identified. Certain sequence reads can be stored based on a likelihood of corresponding to clinically-relevant DNA, where the likelihood accounts for the sequence reads including the preferred sequence end motifs. The stored sequence reads can be analyzed to determine a property of the clinically-relevant DNA the biological sample.

These and other embodiments of the disclosure are described in detail below. For example, other embodiments are directed to systems, devices, and computer readable media associated with methods described herein.

A better understanding of the nature and advantages of embodiments of the present disclosure may be gained with reference to the following detailed description and the accompanying drawings.

BRIEF DESCRIPTION OF THE DRAWINGS

FIG. 1 shows examples for end motifs according to embodiments of the present disclosure.
FIG. 2 shows a schematic of genotypic difference based approach for analyzing the differential end motif patterns between fetal and maternal DNA molecules according to embodiments of the present disclosure.

FIG. 3 shows a bar plot of end motif frequencies between fetal and maternal DNA molecules according to embodiments of the present disclosure.

FIG. 4 shows the top 10 end motifs from FIG. 3 for fetal and shared (i.e., fetal plus maternal) sequences according to embodiments of the present disclosure.

FIGS. 5A and 5B show box plots of entropy between fetal and maternal DNA molecules in pregnant women according to embodiments of the present invention.

FIGS. 6A and 6B show a hierarchical clustering analysis for fetal and maternal DNA molecules according to embodiments of the present disclosure.

FIGS. 7A and 7B show entropy distributions using all motifs for pregnant women across different trimesters according to embodiments of the present disclosure. FIGS. 7C and 7D show entropy distributions using 10 motifs for pregnant women across different trimesters according to embodiments of the present disclosure.

FIG. 8A shows the entropy for all fragments across different gestational ages. The entropy of plasma DNA fragments in subjects with the $3^{rd}$ trimester was shown to be lower (p-value=0.06) than those with the $1^{st}$ and $2^{nd}$ trimester. FIG. 8B shows the entropy for Y chromosome derived fragments across different gestational ages. The entropy of Y chromosome derived fragments in subjects with the $3^{rd}$ trimester was shown to be lower (p-value=0.01) than those with the $1^{st}$ and $2^{nd}$ trimester.

FIGS. 9 and 10 show the distribution of the top 10 ranked end motifs between fetal and maternal DNA molecules across different trimesters according to embodiments of the present disclosure.

FIG. 11 shows a combined frequency of top 10 ranked motifs between fetal and shared molecules across different trimester according to embodiments of the present disclosure.

FIG. 12 shows a schematic of genotypic difference based approach to analyze the differential end motif patterns between mutant and shared molecules in the plasma DNA of a cancer patient according to embodiments of the present disclosure.

FIG. 13 shows the landscape of plasma DNA end motifs of cancer-associated mutant and shared molecules in hepatocellular carcinoma according to embodiments of the present disclosure.

FIG. 14 shows a radial landscape of plasma DNA end motifs of cancer-associated mutant and shared molecules in hepatocellular carcinoma according to embodiments of the present disclosure.

FIG. 15A shows the top 10 end motifs in the ranked difference of end motif frequency between mutant and shared sequences in the plasma DNA of an HCC patient according to embodiments of the present disclosure.

FIG. 15B shows a combined frequency for 8 end motifs for an HCC patient and a pregnant female according to embodiments of the present disclosure.

FIGS. 16A and 16B show entropy values for shared and mutant fragments for different sets of end motifs for an HCC case according to embodiments of the present disclosure.

FIG. 17 is a plot of a motif diversity score (entropy) against a measured circulating tumor DNA fraction according to embodiments of the present disclosure.

FIG. 18A shows an entropy analysis using donor-specific fragments according to embodiments of the present disclosure. FIG. 18B shows a hierarchical clustering analysis using donor-specific fragments.

FIG. 19 is a flowchart illustrating a method of estimating a fractional concentration of clinically-relevant DNA in a biological sample of a subject according to embodiments of the present disclosure.

FIG. 20 is a flowchart illustrating a method of determining a gestational age of a fetus by analyzing a biological sample from a female subject pregnant with a fetus according to embodiments of the present disclosure.

FIG. 21 shows a schematic of a phenotypic approach for plasma DNA end motif analysis according to embodiments of the present disclosure.

FIG. 22 shows an example for the frequency profile of 4-mer end motifs between HCC and HBV subjects with the use of all plasma DNA molecules according to embodiments of the present disclosure.

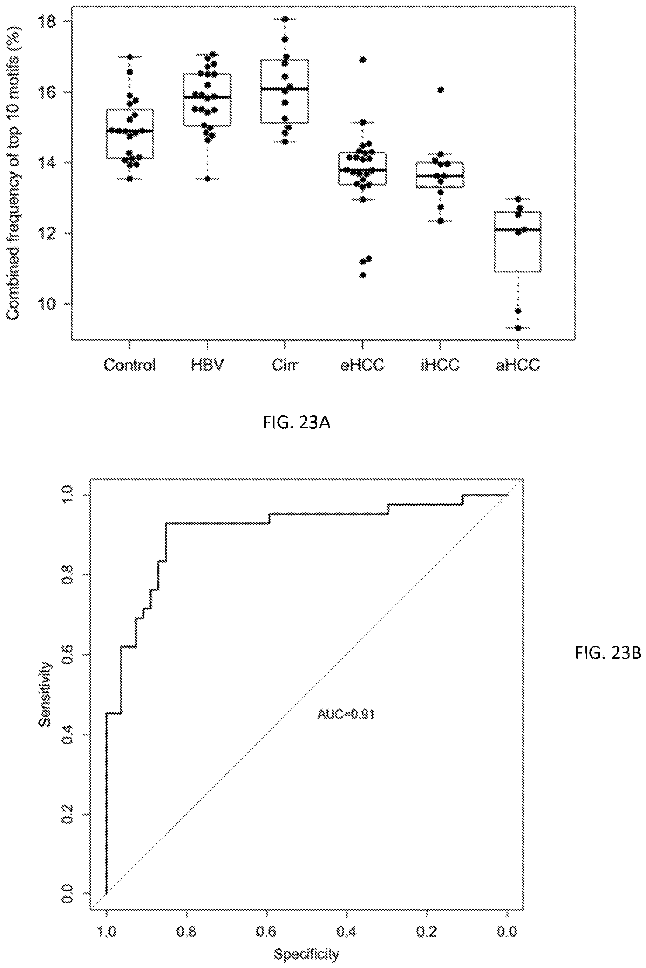

FIG. 23A shows a boxplot for the combined frequency of top 10 plasma DNA 4-mer end motifs for various subjects having different levels of cancer according to embodiments of the present disclosure. The levels are control: healthy control subjects; HBV: chronic hepatitis B carriers; Cirr: cirrhosis subjects; eHCC: early-stage HCC; iHCC: immediate-stage HCC; and aHCC: advanced-stage HCC. FIG. 23B shows a Receiver Operating Characteristic (ROC) curve of the combined frequency of top 10 plasma DNA 4-mer end motifs between HCC and non-cancer subjects according to embodiments of the present disclosure.

FIG. 24A shows a boxplot of the frequency of CCA motif across different groups according to embodiments of the present disclosure. FIG. 24B shows an ROC curve between non-HCC and HCC groups using the most frequent 3-mer motif (CCA) present in non-HCC subjects according to embodiments of the present disclosure.

FIG. 25A shows a boxplot of entropy values across different groups using 256 4-mer end motifs according to embodiments of the present disclosure. FIG. 25B shows a boxplot of entropy values across different groups using 10 4-mer end motifs according to embodiments of the present disclosure.

FIG. 26A shows a boxplot of entropy values using 3-mer motifs across different groups according to embodiments of the present disclosure. The entropy of HCC subjects using 3-mer motifs (a total of 64 motifs) was found to be significantly higher (p-value < 0.0001) than that of non-HCC subjects. FIG. 26B shows an ROC curve using the entropy of 64 3-mer motifs between non-HCC and HCC groups according to embodiments of the present disclosure. The AUC was found to be 0.872.

FIGS. 27A and 27B show boxplots of motif diversity (entropy) scores using 4-mers across different groups according to embodiments of the present disclosure.

FIG. 28 shows a receiver operating curve for various techniques of discriminating healthy controls from cancer according to embodiments of the present disclosure.

FIG. 29 shows a receiver operating curve for an MDS analysis using various k-mers according to embodiments of the present disclosure.

FIG. 30 shows performance of an MDS-based cancer detection for various tumor DNA fractions according to embodiments of the present disclosure.

FIG. 31 shows a receiver operating curve for MDS, SVM, and logistic regression analyses according to embodiments of the present disclosure.

FIG. 32 shows a hierarchical clustering analysis for top 10 ranked end motifs across different groups having different levels of cancer according to embodiments of the present disclosure. The different groups include control: healthy control subjects; HBV: chronic hepatitis B carriers; Cirr: cirrhosis subjects; eHCC: early-stage HCC; iHCC: immediate-stage HCC; and aHCC: advanced-stage HCC.

FIGS. 33A-33C shows a hierarchical clustering analysis using all plasma DNA molecules across different groups having different levels of cancer according to embodiments of the present disclosure.

FIG. 34 shows a hierarchical clustering analysis based on 3-mer motifs using all plasma DNA molecules across different groups having different levels of cancer according to embodiments of the present disclosure.

FIG. 35A shows an entropy analysis using all plasma DNA molecules between healthy control subjects and SLE patient according to embodiments of the present disclosure. FIG. 35B shows a hierarchical clustering analysis using all plasma DNA molecules between healthy control subjects and SLE patients according to embodiments of the present disclosure.

FIG. 36 shows an entropy analysis using plasma DNA molecules having 10 selected end motifs between healthy control subjects and SLE patient according to embodiments of the present disclosure.

FIG. 37 shows an ROC curve for a combined analysis that include end motifs and copy number or methylation according to embodiments of the present disclosure.

FIG. 38A shows an entropy analysis based on the 4-mer jointly constructed from the ends of sequenced plasma DNA fragments and their adjacent genomic sequences in HCC and non-HCC subjects according to embodiments of the present disclosure. FIG. 38B shows a clustering analysis based on the 4-mer jointly constructed from the ends of sequenced plasma DNA fragments and their adjacent genomic sequences in HCC and non-HCC subjects according to embodiments of the present disclosure.

FIG. 39 shows an ROC comparison for techniques 140 and 160 of FIG. 1 used to define the end motif of plasma DNA according to embodiments of the present disclosure.

FIG. 40 shows a comparison of accuracies that shows tissue-specific open chromatin regions improves the discriminative power of plasma DNA end motif according to embodiments of the present disclosure.

FIG. 41 shows a size-band based plasma DNA end motif analysis according to embodiments of the present disclosure.

FIG. 42 is a flowchart illustrating a method of classifying a level of pathology in a biological sample of a subject according to embodiments of the present disclosure.

FIG. 43 is a flowchart illustrating a method of enriching a biological sample for clinically-relevant DNA according to embodiments of the present disclosure.

FIG. 44 is a flowchart illustrating a method 3700 of enriching a biological sample for clinically-relevant DNA according to embodiments of the present disclosure.

FIG. 45 shows an example plot illustrating an increase in fetal DNA fraction using the CCCA end motif according to embodiments of the present disclosure.

FIG. 46 illustrates a measurement system according to an embodiment of the present invention.

FIG. 47 shows a block diagram of an example computer system usable with systems and methods according to embodiments of the present invention.

TERMS

A *"tissue"* corresponds to a group of cells that group together as a functional unit. More than one type of cells can be found in a single tissue. Different types of tissue may consist of different types of cells (e.g., hepatocytes, alveolar cells or blood cells), but also may correspond to tissue from different organisms (mother vs. fetus) or to healthy cells vs. tumor cells. "Reference tissues" can correspond to tissues used to determine tissue-specific methylation levels. Multiple samples of a same tissue type from different individuals may be used to determine a tissue-specific methylation level for that tissue type.

A *"biological sample"* refers to any sample that is taken from a subject *(e.g.,* a human (or other animal), such as a pregnant woman, a person with cancer, or a person suspected of having cancer, an organ transplant recipient or a subject suspected of having a disease process involving an organ (e.g., the heart in myocardial infarction, or the brain in stroke, or the hematopoietic system in anemia) and contains one or more nucleic acid molecule(s) of interest. The biological sample can be a bodily fluid, such as blood, plasma, serum, urine, vaginal fluid, fluid from a hydrocele (e.g. of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (e.g. thyroid, breast), intraocular fluids (e.g. the aqueous humor), etc. Stool samples can also be used. In various embodiments, the majority of DNA in a biological sample that has been enriched for cell-free DNA (e.g., a plasma sample obtained via a centrifugation protocol) can be cell-free, e.g., greater than 50%, 60%, 70%, 80%, 90%, 95%, or 99% of the DNA can be cell-free. The centrifugation protocol can include, for example, 3,000 g x 10 minutes, obtaining the fluid part, and re-centrifuging at for example, 30,000 g for another 10 minutes to remove residual cells. As part of an analysis of a biological sample, at least 1,000 cell-free DNA molecules can be analyzed. As other examples, at least 10,000 or 50,000 or 100,000 or 500,000 or 1,000,000 or 5,000,000 cell-free DNA molecules, or more, can be analyzed.

*"Clinically-relevant DNA"* can refer to DNA of a particular tissue source that is to be measured, e.g., to determine a fractional concentration of such DNA or to classify a phenotype of a sample (e.g., plasma). Examples of clinically-relevant DNA are fetal DNA in maternal plasma or tumor DNA in a patient's plasma or other sample with cell-free DNA. Another example includes the measurement of the amount of graft-associated DNA in the plasma, serum, or urine of a transplant patient. A further example includes the measurement of the fractional concentrations of hematopoietic and nonhematopoietic DNA in the plasma of a subject, or fractional concentration of a liver DNA fragments (or other tissue) in a sample or fractional concentration of brain DNA fragments in cerebrospinal fluid.

A *"sequence read'* refers to a string of nucleotides sequenced from any part or all of a nucleic acid molecule. For example, a sequence read may be a short string of nucleotides (e.g., 20-150 nucleotides) sequenced from a nucleic acid fragment, a short string of nucleotides at one or both ends of a nucleic acid fragment, or the sequencing of the entire nucleic acid fragment that exists in the biological sample. A sequence read may be obtained in a variety of ways, e.g., using sequencing techniques or using probes, e.g., in hybridization arrays or capture probes, or amplification techniques, such as the polymerase chain reaction (PCR) or linear amplification using a single primer or isothermal amplification. As part of an analysis of a biological sample, at least 1,000 sequence reads can be analyzed. As other examples, at least 10,000 or 50,000 or 100,000 or 500,000 or 1,000,000 or 5,000,000 sequence reads, or more, can be analyzed.

A sequence read can include an *"ending sequence"* associated with an end of a fragment. The ending sequence can correspond to the outermost N bases of the fragment, e.g., 2-30 bases at the end of the fragment. If a sequence read corresponds to an entire fragment, then the sequence read can include two ending sequences. When paired-end sequencing provides two sequence reads that correspond to the ends of the fragments, each sequence read can include one ending sequence.

A *"sequence motif"* may refer to a short, recurring pattern of bases in DNA fragments (e.g., cell-free DNA fragments). A sequence motif can occur at an end of a fragment, and thus be part of or include an ending sequence. An "end motif" can refer to a sequence motif for an ending sequence that preferentially occurs at ends of DNA fragments, potentially for a particular type of tissue. An end motif may also occur just before or just after ends of a fragment, thereby still corresponding to an ending sequence.

The term *"alleles"* refers to alternative DNA sequences at the same physical genomic locus, which may or may not result in different phenotypic traits. In any particular diploid organism, with two copies of each chromosome (except the sex chromosomes in a male human subject), the genotype for each gene comprises the pair of alleles present at that locus, which are the same in homozygotes and different in heterozygotes. A population or species of organisms typically include multiple alleles at each locus among various individuals. A genomic locus where more than one allele is found in the population is termed a polymorphic site. Allelic variation at a locus is measurable as the number of alleles (i.e., the degree of polymorphism) present, or the proportion of heterozygotes (i.e., the heterozygosity rate) in the population. As used herein, the term *"polymorphism"* refers to any inter-individual variation in the human genome, regardless of its frequency. Examples of such variations include, but are not limited to, single nucleotide polymorphism, simple tandem repeat polymorphisms, insertion-deletion polymorphisms, mutations (which may be disease causing) and copy number variations. The term *"haplotype"* as used herein refers to a combination of alleles at multiple loci that are transmitted together on the same chromosome or chromosomal region. A haplotype may refer to as few as one pair of loci or to a chromosomal region, or to an entire chromosome or chromosome arm.

The term *"fractional fetal DNA concentration"* is used interchangeably with the terms "*fetal DNA proportion"* and "*fetal DNA fraction,"* and refers to the proportion of fetal DNA molecules that are present in a biological sample (e.g., maternal plasma or serum sample) that is derived from the fetus (Lo et al, Am J Hum Genet. 1998;62:768-775; Lun et al, Clin Chem.

2008;54:1664-1672). Similarly, tumor fraction or tumor DNA fraction can refer to the fractional concentration of tumor DNA in a biological sample.

A *"relative frequency"* may refer to a proportion (e.g., a percentage, fraction, or concentration). In particular, a relative frequency of a particular end motif (e.g., CCGA) can provide a proportion of cell-free DNA fragments that are associated with the end motif CCGA, e.g., by having an ending sequence of CCGA.

An *"aggregate value"* may refer to a collective property, e.g., of relative frequencies of a set of end motifs. Examples include a mean, a median, a sum of relative frequencies, a variation among the relative frequencies (e.g., entropy, standard deviation (SD), the coefficient of variation (CV), interquartile range (IQR) or a certain percentile cutoff (e.g. 95th or 99th percentile) among different relative frequencies), or a difference (e.g., a distance) from a reference pattern of relative frequencies, as may be implemented in clustering.

A *"calibration sample"* can correspond to a biological sample whose fractional concentration of clinically-relevant DNA (e.g., tissue-specific DNA fraction) is known or determined via a calibration method, e.g., using an allele specific to the tissue, such as in transplantation whereby an allele present in the donor's genome but absent in the recipient's genome can be used as a marker for the transplanted organ. As another example, a calibration sample can correspond to a sample from which end motifs can be determined. A calibration sample can be used for both purposes.

A *"calibration data point"* includes a *"calibration value"* and a measured or known fractional concentration of the clinically-relevant DNA (e.g., DNA of particular tissue type). The calibration value can be determined from relative frequencies (e.g., an aggregate value) as determined for a calibration sample, for which the fractional concentration of the clinically-relevant DNA is known. The calibration data points may be defined in a variety of ways, e.g., as discrete points or as a calibration function (also called a calibration curve or calibration surface). The calibration function could be derived from additional mathematical transformation of the calibration data points.

A *"site"* (also called a *"genomic site")* corresponds to a single site, which may be a single base position or a group of correlated base positions, e.g., a CpG site or larger group of correlated base positions. A "locus" may correspond to a region that includes multiple sites. A locus can include just one site, which would make the locus equivalent to a site in that context.

The *"methylation index"* for each genomic site (e.g., a CpG site) can refer to the proportion of DNA fragments (e.g., as determined from sequence reads or probes) showing methylation at the site over the total number of reads covering that site. A "read" can correspond to information (e.g., methylation status at a site) obtained from a DNA fragment. A read can be obtained using reagents (e.g. primers or probes) that preferentially hybridize to DNA fragments of a particular methylation status. Typically, such reagents are applied after treatment with a process that differentially modifies or differentially recognizes DNA molecules depending of their methylation status, e.g. bisulfite conversion, or methylation-sensitive restriction enzyme, or methylation binding proteins, or anti-methylcytosine antibodies, or single molecule sequencing techniques that recognize, for example, methylcytosines and hydroxymethylcytosines.

The *"methylation density"* of a region can refer to the number of reads at sites within the region showing methylation divided by the total number of reads covering the sites in the region. The sites may have specific characteristics, e.g., being CpG sites. Thus, the "CpG methylation density" of a region can refer to the number of reads showing CpG methylation divided by the total number of reads covering CpG sites in the region (e.g., a particular CpG site, CpG sites within a CpG island, or a larger region). For example, the methylation density for each 100-kb bin in the human genome can be determined from the total number of cytosines not converted after bisulfite treatment (which corresponds to methylated cytosine) at CpG sites as a proportion of all CpG sites covered by sequence reads mapped to the 100-kb region. This analysis can also be performed for other bin sizes, e.g. 500 bp, 5 kb, 10 kb, 50-kb or 1-Mb, etc. A region could be the entire genome or a chromosome or part of a chromosome (e.g. a chromosomal arm). The methylation index of a CpG site is the same as the methylation density for a region when the region only includes that CpG site. The "proportion of methylated cytosines" can refer the number of cytosine sites, "C's", that are shown to be methylated (for example unconverted after bisulfite conversion) over the total number of analyzed cytosine residues, i.e. including cytosines outside of the CpG context, in the region. The methylation index, methylation density and proportion of methylated cytosines are examples of "methylation levels." Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al, Proc Natl Acad Sci USA. 2013; 110: 18910-18915) and by the Pacific Biosciences single molecule real time analysis (Flusberg et al, Nat Methods. 2010; 7: 461-465)). A methylation metric of a DNA molecule can correspond to a percentage of sites (e.g., CpG sites) that are methylated. The methylation metric can be specified as an absolute number or a percentage, which may be referred to as a methylation density of a molecule.

The term *"sequencing depth"* refers to the number of times a locus is covered by a sequence read aligned to the locus. The locus could be as small as a nucleotide, or as large as a chromosome arm, or as large as the entire genome. Sequencing depth can be expressed as 50x, 100x, etc., where "x" refers to the number of times a locus is covered with a sequence read. Sequencing depth can also be applied to multiple loci, or the whole genome, in which case x can refer to the mean number of times the loci or the haploid genome, or the whole genome, respectively, is sequenced. Ultra-deep sequencing can refer to at least 100x in sequencing depth.

A *"separation value"* corresponds to a difference or a ratio involving two values, e.g., two fractional contributions or two methylation levels. The separation value could be a simple difference or ratio. As examples, a direct ratio of x/y is a separation value, as well as x/(x+y). The separation value can include other factors, e.g., multiplicative factors. As other examples, a difference or ratio of functions of the values can be used, e.g., a difference or ratio of the natural logarithms (ln) of the two values. A separation value can include a difference and a ratio.

A *"separation value"* and an *"aggregate value"* (e.g., of relative frequencies) are two examples of a parameter (also called a metric) that provides a measure of a sample that varies between different classifications (states), and thus can be used to determine different classifications. An aggregate value can be a separation value, e.g., when a difference is taken between a set of relative frequencies of a sample and a reference set of relative frequencies, as may be done in clustering.

The term *"classification"* as used herein refers to any number(s) or other characters(s) that are associated with a particular property of a sample. For example, a "+" symbol (or the word "positive") could signify that a sample is classified as having deletions or amplifications. The classification can be binary (e.g., positive or negative) or have more levels of classification (e.g., a scale from 1 to 10 or 0 to 1).

The terms "*cutoff*" and *"threshold"* refer to predetermined numbers used in an operation. For example, a cutoff size can refer to a size above which fragments are excluded. A threshold value may be a value above or below which a particular classification applies. Either of these terms can be used in either of these contexts. A cutoff or threshold may be "a reference value" or derived from a reference value that is representative of a particular classification or discriminates between two or more classifications. Such a reference value can be determined in various ways, as will be appreciated by the skilled person. For example, metrics can be determined for two different cohorts of subjects with different known classifications, and a reference value can be selected as representative of one classification (e.g., a mean) or a value that is between two clusters of the metrics (e.g., chosen to obtain a desired sensitivity and specificity). As another example, a reference value can be determined based on statistical simulations of samples.

The term *"level of cancer"* can refer to whether cancer exists (i.e., presence or absence), a stage of a cancer, a size of tumor, whether there is metastasis, the total tumor burden of the body, the cancer's response to treatment, and/or other measure of a severity of a cancer (e.g. recurrence of cancer). The level of cancer may be a number or other indicia, such as symbols, alphabet letters, and colors. The level may be zero. The level of cancer may also include premalignant or precancerous conditions (states). The level of cancer can be used in various ways. For example, screening can check if cancer is present in someone who is not previously known to have cancer. Assessment can investigate someone who has been diagnosed with cancer to monitor the progress of cancer over time, study the effectiveness of therapies or to determine the prognosis. In one embodiment, the prognosis can be expressed as the chance of a patient dying of cancer, or the chance of the cancer progressing after a specific duration or time, or the chance or extent of cancer metastasizing. Detection can mean 'screening' or can mean checking if someone, with suggestive features of cancer (e.g. symptoms or other positive tests), has cancer.

A *"level of pathology"* can refer to the amount, degree, or severity of pathology associated with an organism, where the level can be as described above for cancer. Another example of pathology is a rejection of a transplanted organ. Other example pathologies can include autoimmune attack (e.g., lupus nephritis damaging the kidney or multiple sclerosis), inflammatory diseases (e.g., hepatitis), fibrotic processes (e.g. cirrhosis), fatty infiltration (e.g. fatty liver diseases), degenerative processes (e.g. Alzheimer's disease) and ischemic tissue damage (e.g., myocardial infarction or stroke). A heathy state of a subject can be considered a classification of no pathology.

The term "about" or "approximately" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term "about" or "approximately" can mean within an order of magnitude, within 5-fold, and more preferably within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to $\pm$ 10%. The term "about" can refer to $\pm$5%.

## DETAILED DESCRIPTION

The present disclosure describes techniques for measuring quantities (e.g., relative frequencies) of end motifs of cell-free DNA fragments in a biological sample of an organism for measuring a property of the sample and/or determining a condition of the organism based on such measurements. Different tissue types exhibit different patterns for the relative frequencies of the sequence motifs. The present disclosure provides various uses for measures of the relative frequencies of end motifs of cell-free DNA, e.g., in mixtures of cell-free DNA from various tissues. DNA from one of such tissues may be referred to as clinically-relevant DNA.

Clinically-relevant DNA of a particular tissue (e.g., of a fetus, a tumor, or a transplanted organ) exhibit a particular pattern of relative frequencies, which can be measured as an aggregate value. Other DNA in a sample can exhibit a different pattern, thereby allowing a measurement of an amount of clinically-relevant DNA in the sample. Accordingly, in one example, a fractional concentration (e.g., a percentage) of clinically relevant DNA can be determined based on relative frequencies of end motifs. The fractional concentration can be a number, a numerical range, or other classification, e.g., high, medium, or low, or whether the fractional concentration exceeds a threshold. In various implementations, the aggregate value could be a sum of relative frequencies for a set of end motifs, a variance (e.g., entropy, also called a motif diversity score) in relative frequencies in all or a set of end motifs, or a difference (e.g., total distance) from a reference pattern, e.g., an array (vector) of relative frequencies for calibration sample(s) with a known fractional concentration. Such an array can be considered a reference set of relative frequencies. Such a difference can be used in a classifier of which hierarchal clustering, support vector machines, and logistic regression are examples. As examples, the clinically relevant DNA can be fetal, tumor, transplanted organ, or other tissue (e.g. hematopoietic or liver) DNA.

In another example, a level of pathology can be determined using motif relative frequencies. An organism having different phenotypes can exhibit different patterns of motif relative frequencies of cell-free DNA fragments. An aggregate value of relative frequencies of end motifs can be compared to a reference value to classify the phenotype. In various implementations, the aggregate value can be a sum of relative frequencies, a variance in relative frequencies, or a difference from a reference set of relative frequencies. Examples pathologies include cancer and autoimmune diseases, such as SLE.

In another example, motif relative frequencies can be used to determine a gestational age of a fetus. The aggregate value of relative frequencies of end motifs changes in a maternal sample as a result of the longer gestational age of the fetus. Such an aggregate value can be determined as described above and elsewhere.

Given that cell-free DNA fragments from a certain tissue have a particular set of end motifs that are preferred, the preferred end motifs can be used to enrich a sample for DNA from the certain tissue (clinically-relevant DNA). Such enrichment can be performed via physical operations to enrich the physical sample. Some embodiments can capture and/or amplify cell-free DNA fragments having ending sequences matching a set of preferred end motifs, e.g., using primers or adapters. Other examples are described herein.

In some embodiments, the enrichment can be performed in silico. For example, a system can receive sequence reads and then filter the reads based on end motifs to obtain a subset of sequence reads that have a higher concentration of corresponding DNA fragments from the clinically-relevant DNA. If a DNA fragment has an ending sequence that includes a preferred end motif, it can be identified as having a higher likelihood of being from the tissue of interest. The likelihood can be further determined based on methylation and size of the DNA fragments, as is described herein.

Such uses of end motifs can obviate a need for a reference genome, as may be needed when using end positions (Chan et al, Proc Natl Acad Sci USA. 2016;113 :E8159-8168; Jiang et al, Proc Natl Acad Sci USA. 2018; doi: 10.1073/pnas.1814616115)). Further, as the number of end motifs may be smaller than the number of preferred end positions in a reference genome, greater statistics can be gathered for each end motif, potentially increasing accuracy.

Such an ability to use end motifs in the manner described above is surprising, e.g., as Chandrananda et al. found that there was high similarity between maternal and fetal fragments in terms of position-specific nucleotide patterns concerning mononucleotide frequencies for the region of 51 bp (up-/down-stream 20 bp) around fragment start sites (Chandrananda et al, BMC Med Genomics. 2015; 8:29), implying that the use of their method based on mononucleotide frequencies around ends was unable to inform the tissue of origin of the cell-free DNA fragments.

## I. CELL-FREE DNA END MOTIFS

An end motif relates to the ending sequence of a cell-free DNA fragment, e.g., the sequence for the K bases at either end of the fragment. The ending sequence can be a k-mer having various numbers of bases, e.g., 1, 2, 3, 4, 5, 6, 7, etc. The end motif (or "sequence motif") relates to the sequence itself as opposed to a particular position in a reference genome. Thus, a same end motif may occur at numerous positions throughout a reference genome. The end motif may be determined using a reference genome, e.g., to identify bases just before a start position or just after an end position. Such bases will still correspond to ends of cell-free DNA fragments, e.g., as they are identified based on the ending sequences of the fragments.

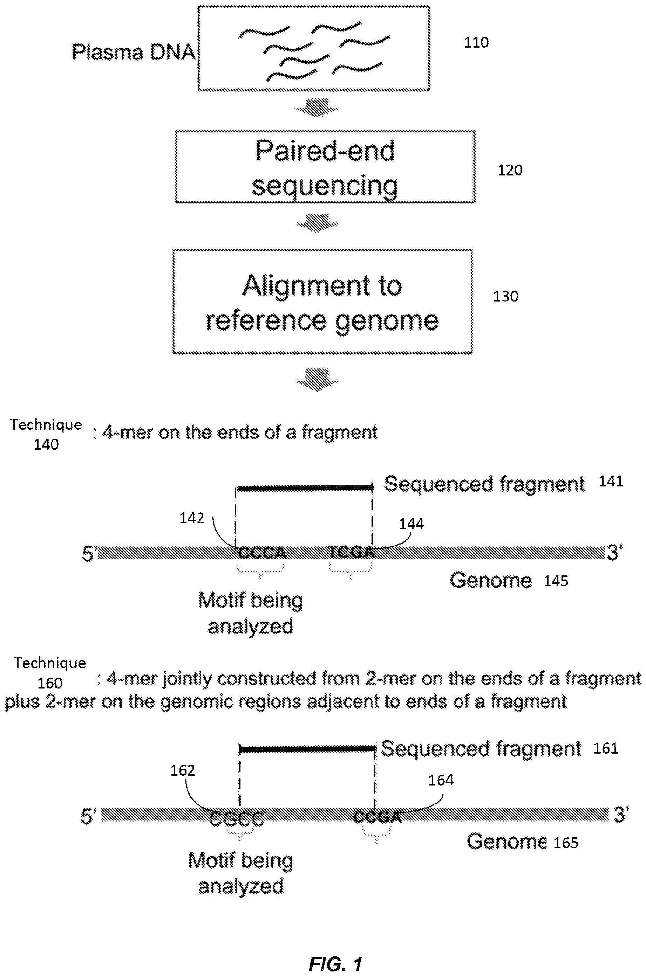

FIG. 1 shows examples for end motifs according to embodiments of the present disclosure. FIG. 1 depicts two ways to define 4-mer end motifs to be analyzed. In technique 140, the 4-mer end motifs are directly constructed from the first 4-bp sequence on each end of a plasma DNA molecule. For example, the first 4 nucleotides or the last 4 nucleotides of a sequenced fragment could be used. In technique 160, the 4-mer end motifs are jointly constructed by making use of the 2-mer sequence from the sequenced ends of fragments and the other 2-mer sequence from the genomic regions adjacent to the ends of that fragment. In other embodiments, other types of motifs can be used, e.g., 1-mer, 2-mer, 3-mer, 5-mer, 6-mer, and 7-mer end motifs.

As shown in FIG. 1, cell-free DNA fragments 110 are obtained, e.g., using a purification process on a blood sample, such as by centrifuging. Besides plasma DNA fragments, other types of cell-free DNA molecules can be used, e.g., from serum, urine, saliva, and other such cell-free samples mentions herein. In one embodiment, the DNA fragments may be blunt-ended.

At block 120, the DNA fragments are subjected to paired-end sequencing. In some embodiments, the paired-end sequencing can produce two sequence reads from the two ends of a DNA fragment, e.g., 30-120 bases per sequence read. These two sequence reads can form a pair of reads for the DNA fragment (molecule), where each sequence read includes an ending sequence of a respective end of the DNA fragment. In other embodiments, the entire DNA fragment can be sequenced, thereby providing a single sequence read, which includes the ending sequences of both ends of the DNA fragment.

At block 130, the sequence reads can be aligned to a reference genome. This alignment is to illustrate different ways to define a sequence motif, and may not be used in some embodiments. The alignment procedure can be performed using various software packages, such as BLAST, FASTA, Bowtie, BWA, BFAST, SHRiMP, SSAHA2, NovoAlign and SOAP.

Technique 140 shows a sequence read of a sequenced fragment 141, with an alignment to a genome 145. With the 5' end viewed as the start, a first end motif 142 (CCCA) is at the start of sequenced fragment 141. A second end motif 144 (TCGA) is at the tail of the sequenced fragment 141. Such end motifs might, in one embodiment, occur when an enzyme recognizes CCCA and then makes a cut just before the first C. If that is the case, CCCA will preferentially be at the end of the plasma DNA fragment. For TCGA, an enzyme might recognize it, and then make a cut after the A.

Technique 160 shows a sequence read of a sequenced fragment 161, with an alignment to a genome 165. With the 5' end viewed as the start, a first end motif 162 (CGCC) has a first portion (CG) that occurs just before the start of sequenced fragment 161 and a second portion (CC) that is part of the ending sequence for the start of sequenced fragment 161. A second end motif 164 (CCGA) has a first portion (GA) that occurs just after the tail of sequenced fragment 161 and a second portion (CC) that is part of the ending sequence for the tail of sequenced fragment 161. Such end motifs might, in one embodiment, occur when an enzyme recognizes CGCC and then makes a cut in between the G and the C. If that is the case, CC will preferentially be at the end of the plasma DNA fragment with CG occurring just before it, thereby providing an end motif of CGCC. As for the second end motif 164 (CCGA), an enzyme can cut between C and G. If that is the case, CC will preferentially be at the end of the plasma DNA fragment. For technique 160, the number of bases from the adjacent genome regions and sequenced plasma DNA fragments can be varied and are not necessarily restricted to a fixed ratio, e.g., instead of 2:2, the ratio can be 2:3, 3:2, 4:4, 2:4, etc.

The higher the number of nucleotides included in the cell-free DNA end signature, the higher the specificity of the motif because the probability of having 6 bases ordered in an exact configuration in the genome is lower than the probability of having 2 bases ordered in an exact configuration in the genome. Thus, the choice of the length of the end motif can be governed by the needed sensitivity and/or specificity of the intended use application.

As the ending sequence is used to align the sequence read to the reference genome, any sequence motif determined from the ending sequence or just before/after is still determined from the ending sequence. Thus, technique 160 makes an association of an ending sequence to other bases, where the reference is used as a mechanism to make that association. A difference between techniques 140 and 160 would be to which two end motif a particular DNA fragment is assigned, which affects the particular values for the relative frequencies. But, the overall result (e.g., fractional concentration of clinically-relevant DNA, classification of a level of pathology, etc.) would not be affected by how the a DNA fragment is assigned to an end motif, as long as a consistent technique is used for the training data as used in production.

The counted numbers of DNA fragments having an ending sequence corresponding to a particular end motif may be counted (e.g., stored in an array in memory) to determine relative frequencies. As described in more detail below, a relative frequency of end motifs for cell-free DNA fragments can be analyzed. Differences in relative frequencies of end motifs have been detected for different types of tissue and for different phenotypes, e.g., different levels of pathology. The differences can be quantified by an amount of DNA fragments having specific end motifs or an overall pattern, e.g., a variance (such as entropy, also called a motif diversity score), across a set of end motifs (e.g., all possible combinations of the k-mers corresponding to the length used).

## II. APPROACHES BASED ON GENOTYPIC DIFFERENCES

We have identified that different tissue types have different end motifs. Herein, we describe how the end motifs can be used to determine a fractional concentration of clinically-relevant DNA, e.g., fetal DNA, tumor DNA, DNA from a transplanted organ, or DNA from a particular organ.

To identify end motifs that are preferential to a particular type of clinically-relevant DNA, genotypic differences can be used to identify a DNA fragment as being from the clinically-relevant tissue. Once a DNA fragment is detected as being from the clinically-relevant tissue, an end motif of the DNA fragment can be determined. Our analysis of a relative frequency of end motifs reveals that the relative frequency of end motifs varies for different tissues. As explained below, the quantification of the difference in relative frequencies can be used in conjunction with calibration sample(s), whose fractional concentration of clinically-relevant DNA are known (e.g., measured by a separate technique, such a tissue-specific allele), to determine a classification of the fractional concentration of clinically-relevant DNA in the biological sample.

Although measurement of the fractional concentration of clinically-relevant DNA in the calibration samples may be needed, the resulting calibration values (e.g., as part of a calibration function) can be used to determine a fractional concentration for a new sample without having to identify alleles that are specific to the clinically-relevant DNA. In this manner, the fractional concentration can be determined in a more robust manner.

*A. Pregnancy*

The genotypic difference between the maternal and fetal genomes can be used to distinguish the fetal and maternal DNA molecules. For example, we can make use of the informative single nucleotide polymorphism (SNP) sites for which the mother is homozygous (AA) and the fetus is heterozygous (AB).

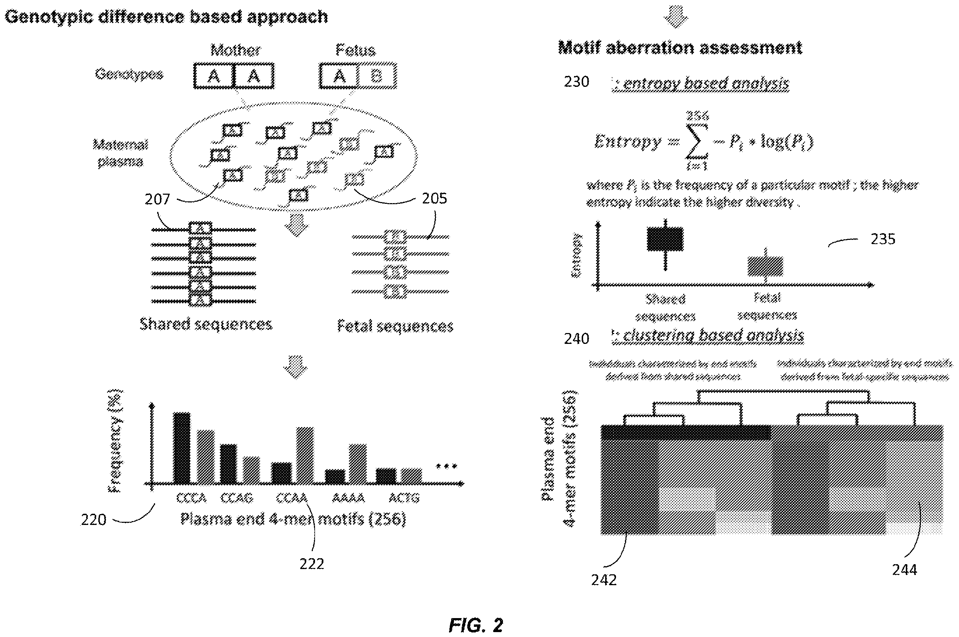

FIG. 2 shows a schematic of genotypic difference based approach for analyzing the differential end motif patterns between fetal and maternal DNA molecules according to embodiments of the present disclosure. As illustrated in FIG. 2, the fetal-specific molecules 205 that carry the fetal-specific alleles (B) can be determined. On the other hand, the shared molecules 207 that carry the shared allele (A) can be determined, which would represent the predominantly maternally-derived DNA molecules because the fetal DNA molecules generally would be the minority in the maternal plasma DNA pool. Therefore, any molecular properties derived from shared molecules would reflect the characteristics of maternal background DNA molecules (i.e. hematopoietically derived DNA molecules). Besides alleles, other fetal-specific markers (e.g., epigenetic markers) can be used.

We analyzed 4-mer end motifs using technique 140 in FIG. 1. 256 end motifs were analyzed. We calculated the proportion of each 4-mer motif and compared the frequencies across 256 motifs using a bar plot, depicted as bar plot 220. Such a bar plot provides a relative frequency (%) that each 4-mer occurs as an end motif. For ease of illustration, only a few 4-mers are shown. A relative frequency (also sometimes referred to as just "frequency") can be determined by (# of DNA fragments having the end motif) / a total number of DNA fragments analyzed, potentially with a factor of 2 in the denominator, to account for both ends. Such a percentage can be considered a relative frequency as it relates to a ratio of one amount (e.g., count) for a first end motif relative to an amount for one or more other motifs (potentially including the first end motif). As one can see, an end motif 222 has a significant difference of relative frequencies between DNA fragments of the different tissue types. Such a difference can be used for various purposes, e.g., to enrich a sample for fetal DNA or to determine a fetal DNA concentration.

The values of the relative frequencies shown in bar plot 220 can be stored values in an array having 256 values. Counters can exist for each end motif of a set of end motifs, where a counter for a particular end motif is incremented each time a new DNA fragment has an end motif corresponding to that counter. The set of motifs can be selected in various ways, e.g., as all end motifs or a smaller set, such as those occurring the most in a reference sample or those showing a largest separation in a reference sample.

Various quantification techniques can be used to provide a measure for the relative frequencies of a sample, and such quantification techniques can be used to classify an amount of cell-free DNA from the clinically-relevant DNA. One example quantification technique includes a sum of the relative frequencies of a set of end motifs, also called a combined frequency herein. As example, such a set may be end motifs that occur most frequently in a particular tissue type or that are identified as having a largest separation between two tissue types. A weighted sum could also be used. The weights can be predetermined or variable, e.g., a weight for a given frequency can depend on the frequency itself. An entropy is such an example.

In another embodiment, to capture the landscape difference in end motifs between fetal and maternal DNA molecules, an entropy-based analysis 230 can be used. Entropy is an example of a variance/diversity. To analyze the distribution of frequencies of motifs (e.g. for a total of 256 motifs), one definition of entropy uses the following equation:

$$Entropy = \sum_{i=1}^{256} - P_i * \log(P_i)$$

where $P_i$ is the frequency of a particular motif; a higher entropy value indicates a higher diversity (i.e. a higher degree of randomness).

In this example, when the 256 motifs are equally present in terms of their frequencies, the entropy would achieve the maximal value (i.e. 5.55). In contrast, when the 256 motifs have a skewed distribution in their frequencies, the entropy would decrease. For example, if one particular motif accounts for 99% and the other motifs constitute the remaining 1%, the entropy would decrease to 0.11 in this formulation, although other formulations may be used, such as without the log or just using the log). Therefore, the decreasing entropy of motif frequencies would imply the increasing skewness in the frequency distribution across end motifs. The increasing entropy of motif frequencies would suggest that the frequencies across motifs would shift toward equal probabilities for those motifs. Accordingly, the entropy of motif frequencies measures how evenly the end motif abundances are present in the plasma DNA. The higher the degree of evenness in motif frequencies, the higher entropy values would be expected. In other words, the decreased entropy of motif frequencies would imply the increased skewness across end motifs in terms of its frequency.

In various other examples, the standard deviation (SD), the coefficient of variation (CV), interquartile range (IQR) or a certain percentile cutoff (e.g. 95th or 99th percentile) among different motif frequencies can be used for assessing the landscape changes of end motif patterns between fetal and maternal DNA molecules. Such various examples provide measures of a variance/diversity in the relative frequencies for a set of end motifs. Given the definition of entropy in FIG. 2, the entropy will have a minimal value if only one end motif has a non-zero count. If other end motifs do appear in some DNA fragments, the entropy will be increased. If there is no selection (random distribution for all end motifs, e.g., in one hypothetical scenario of all having the same frequency), then the entropy will go to the maximum value. In this manner, entropy quantifies a global selectivity of the ending sequences of cell-free DNA fragments for end motifs.

Plot 235 shows entropy values for the shared sequences (predominantly maternal) and the fetal sequences. The shared sequences comprise less fetal DNA (potentially around 5% if the original sample had 10% fetal DNA) than the fetal sequences, which would have nearly 100% fetal DNA, within an error tolerance for the genotyping measurements. Given this separation, the greater the concentration of fetal DNA in a sample, the larger the difference the entropy value will be. This relationship between fetal DNA concentration and entropy can be used to determine a fetal DNA concentration, e.g., as measured using one or more calibration values. For example, a concentration of clinically-relevant DNA can be measured for a calibration sample via another technique (resulting in a calibration value), which might not be generally applicable, such as using Y chromosome DNA for male fetuses or a previously identified mutation for tumor tissue. Given an entropy measurement for the calibration sample, a comparison of the two entropy values (one for the test sample and one for the calibration sample) can provide a fractional concentration for the test sample, using the measured concentration in the calibration sample. Further details of such use of calibration values and calibration functions are described later.

In yet another embodiment, a clustering-based analysis 240 can be employed. The vertical axis corresponds to the 4-mer motifs, and the horizontal axis corresponds to the different samples, e.g., having different classification for the concentration of fetal DNA. The color corresponds to a relative frequency of a particular 4-mer motif for a particular samples, e.g., with red calibration samples 242 having a higher concentration than green calibration samples 244, which have a lower value.

The clustering-based analysis can take advantage of the assumption that the similarity of frequency profile of 256 4-mer end motifs would be relatively higher within either fetal DNA molecules or within maternal DNA molecules (i.e. within-group molecular properties) compared to the similarity between fetal and maternal DNA molecules (i.e. between-group molecular properties). Thus, the calibration samples of individuals characterized with the end motifs derived from shared sequences (e.g., a higher concentration of shared sequences) were expected to be different from the calibration samples of individuals characterized with the end motifs derived from fetal-specific sequences (e.g., a lower concentration of shared sequences, and thus higher fetal). Each individual corresponded to a vector comprising 256 end motifs and their corresponding frequencies (i.e. a 256-dimensional vector). Example clustering techniques include, but not limited to, hierarchical clustering, centroid-based clustering, distribution-based clustering, density-based clustering. The different clusters can correspond to differing amounts of the fetal DNA in the sample, as those will have different patterns of relative frequencies, due to the differences in frequency of end motifs between maternal and fetal DNA fragments.

To assess the difference of end motifs between fetal and maternal DNA molecules, we genotyped, respectively, the maternal buffy coat and fetal samples using a microarray platform (Human Omni2.5, Illumina) and sequenced the matched plasma DNA samples. We obtained peripheral blood samples from 10 pregnant women from each of the first (12-14 weeks), second (20-23 weeks), and third (38-40 weeks) trimesters and harvested the plasma and maternal buffy coat samples from each case. We obtained a median of 195,331 informative SNPs (range: 146,428-202,800) where the mother was homozygous and the fetus was heterozygous. Plasma DNA molecules that carried the fetal-specific alleles were identified as fetal-specific DNA molecules. Plasma DNA molecules carrying the shared alleles were identified and believed to be predominantly maternal-derived DNA molecules. The median fetal DNA fraction among those samples was 17.1% (range: 7.0%-46.8%). A median of 103 million (range: 52-186 million) mapped paired-end reads was obtained for each case. The end motif for each plasma DNA molecule was determined by bioinformatically investigating 4-mer sequences nearest to the fragment end. The results from the analysis of this sample set are provided below.

### 1. *Differences in relative frequencies in ranked order*

We reasoned that the top end motifs in the ranked difference of motif frequency between fetal and maternal DNA molecules would be useful for the detecting or enriching fetal and maternal DNA molecules. Thus, we ranked end motifs in terms of their frequency differences between fetal and maternal DNA molecules in one pregnant woman with a sequencing depth of 270x. The fetal and shared sequences were identified according to informative SNPs using the similar way as mentioned above.

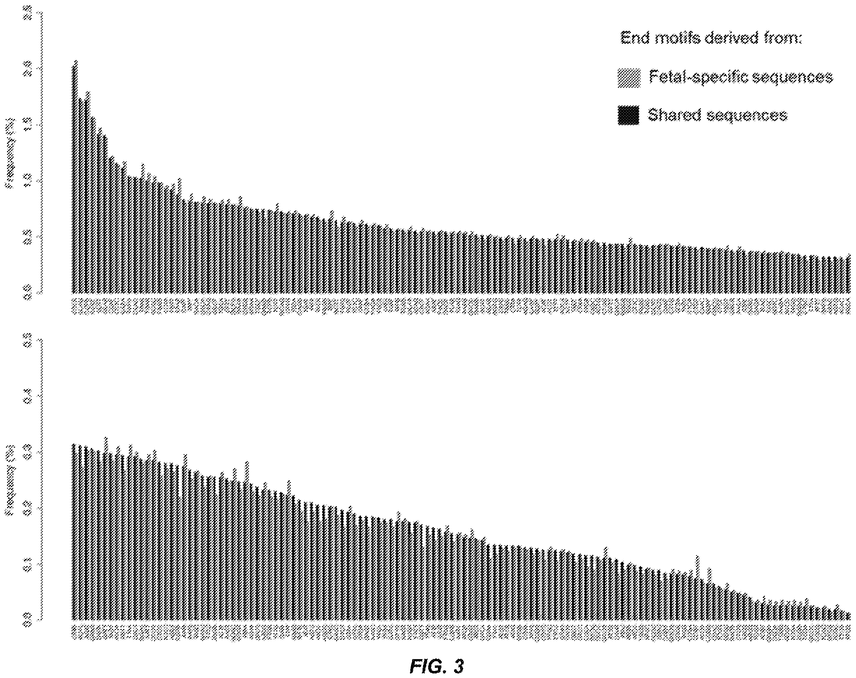

FIG. 3 shows a bar plot of end motif frequencies between fetal and maternal DNA molecules according to embodiments of the present disclosure. The data was obtained from the one pregnant woman with a sequencing depth of 270x. The vertical axis corresponds to the frequency percentage for a given 4-mer motif as determined from the number of DNA fragments (as determined from the sequence reads) that had a given 4-mer motif divided by the total number of ending sequences of the DNA fragments analyzed (e.g., two times the number of DNA fragments). The horizontal axis corresponds to the 256 different 4-mers. The 4-mers are sorted in decreasing frequency for the shared sequences, with FIG. 3 split in two parts with a different scale used for the vertical axis. A difference in the frequencies of end motifs could be observed between fetal DNA molecules (those having a fetal-specific allele) and maternal DNA molecules (those having the shared allele).

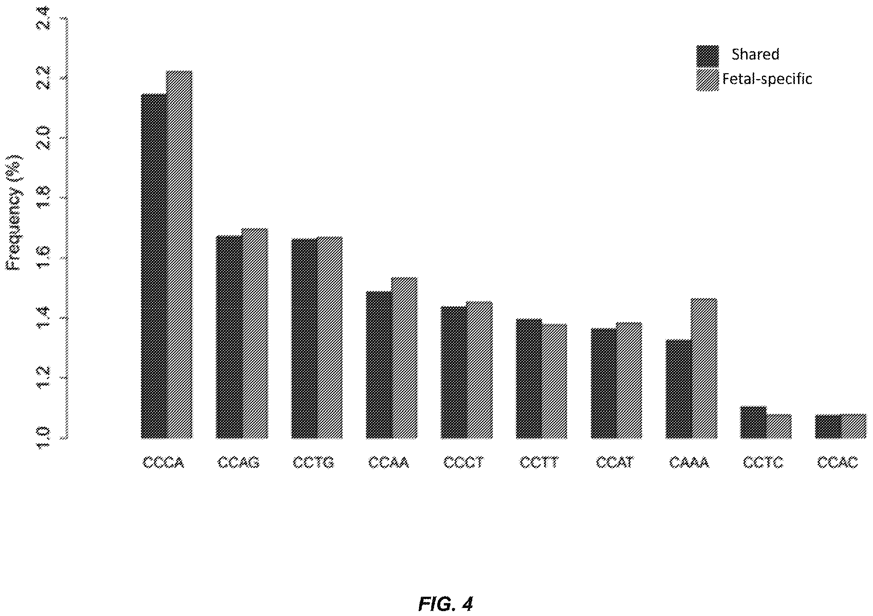

FIG. 4 shows the top 10 end motifs from FIG. 3 for fetal and shared (i.e., fetal plus maternal) sequences according to embodiments of the present disclosure. The vertical axis is shifted and starts with a frequency of 1%. The top 10 end motifs are CCCA, CCAG, CCTG, CCAA, CCCT, CCTT, CCAT, CAAA, CCTC, and CCAC. As one can see, some end motifs have a larger difference between the shared sequences and the fetal-specific sequences than others. Thus, to differentiate between maternal and fetal DNA, one may want to use the end motifs that have the largest differences as opposed to the end motifs that simply have the highest frequency.

### 2. *Use of entropy*

For various samples, the entropy of DNA molecules having the shared allele, and the entropy of DNA molecules having the fetal-specific allele were then analyzed. The former are identified as maternal, and the latter are identified as fetal. For each sample, two data points are obtained: entropy for fetal DNA molecules and entropy for shared DNA molecules (labeled as "maternal").

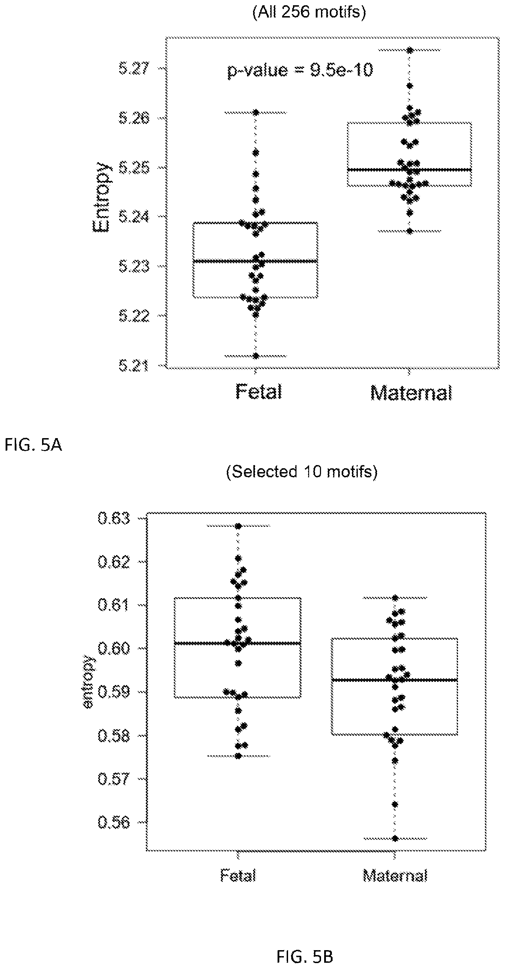

FIG. 5A shows that the entropy of end motifs in fetal DNA molecules is lower than that in maternal DNA molecules (p-value < 0.0001), suggesting that there is higher skewness in the distribution of end motifs originating from the maternal DNA molecules. The entropy in FIG. 5A is determined using all 256 motifs, as a 4-mer was used in these examples, for a given sample and for a given pool of fetal DNA or maternal DNA molecules.

Similar to plot 235 of FIG. 2, the difference in entropy for the two tissue types shows that entropy can be used to determine a fractional concentration of fetal DNA in a mixture (e.g., plasma or serum) of cell-free DNA fragments. As explained above, the pools identified as fetal DNA have a higher percentage (e.g., near 100%) of fetal DNA than the maternal pools. The entropy values determined for the types of pools are different. Thus, there is a relationship between entropy and fetal DNA concentration. This relationship can be determined as a calibration function based on measurements (calibration values) of fetal DNA concentration of calibration samples and the corresponding entropy values (example of relative frequencies), where a calibration value and a relative frequency can form a calibration data point. Calibration samples with different fetal DNA concentration will have different entropy values. A calibration function can be fit to the calibration data points such that a newly measured relative frequency (e.g., entropy) can be input to the calibration function to provide an output of the fetal DNA concentration.

FIG. 5B shows entropy when the relative frequencies of the 10 motifs from FIG. 4 are used. As shown, the relationship changes with fetal sequences having a higher entropy for this given set of 10 end motifs. The fractional concentration of fetal DNA can still be determined, but a different calibration function would be used. Thus, the set of motifs used for calibration should be the same as used later, i.e., when measuring the fractional concentration based on entropy, or other aggregate value of the relative frequencies for the set.

### 3. *Clustering*

We further carried out a hierarchical clustering analysis for pregnant women, each of whom was characterized by a 256-dimensional vector comprising all 4-mer end motif frequencies. Indeed, the individuals characterized with end motifs derived from fetal-specific sequences and maternal DNA molecules can be clustered into two groups.

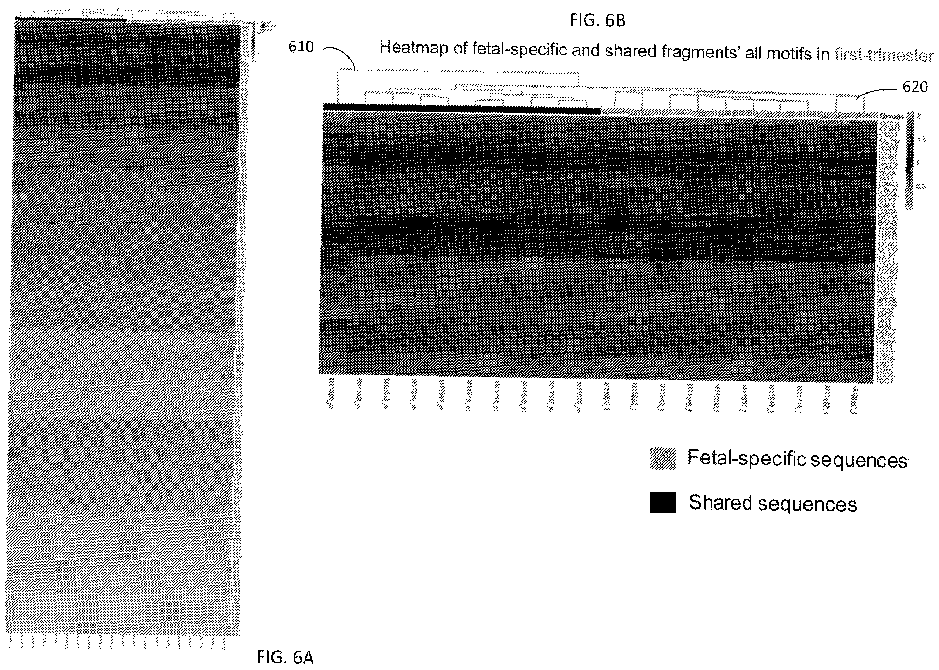

FIGS. 6A and 6B show a hierarchical clustering analysis for fetal and maternal DNA molecules for a first trimester pregnancy according to embodiments of the present disclosure. FIG. 6A shows a hierarchical clustering analysis based on 256 4-mer end motif frequencies. The vertical axis corresponds to the 4-mer motifs, and the horizontal axis corresponds to different portions (i.e., the fetal-specific 620 (yellow) and shared 610 (blue) sequences) of various samples. The color corresponds to a relative frequency of a particular 4-mer motif for a particular portions of samples.

The different portions (fetal-specific and shared) have different fetal DNA concentrations, and thus would have different classifications for the concentration of fetal DNA. When such clustering is performed using calibration samples, the fetal DNA concentration can be measured, e.g., as described in the entropy section above. Each calibration sample would have a corresponding vector of length equal to the number of motifs used (e.g., 256 for all 4-mers or potentially just a subset of 4-mers, as may have a largest difference between fetal and shared sequences, although other k-mers can be used).

FIG. 6B shows a zoomed-in visualization for hierarchical clustering analysis based on 256 4-mer end motif frequencies. Each row represents one type of end motif (i.e., a different end motif). Each column represents a pregnant subject. The gradient colors indicate the frequencies of end motifs. Red represents the highest frequency and green represents the lowest frequency. As one can see, the two portions (fetal and shared) representing samples with different fetal DNA concentrations are cleanly clustered into two separate clusters, showing good accuracy for being able to differentiate samples with differing levels of fetal DNA concentration.

### 4. Samples at different trimesters

Besides being able to differentiate samples with differing fractional concentrations, some embodiments can different samples from pregnant subjects at differing gestational ages (e.g., which trimester, or just whether is in the 3rd trimester).

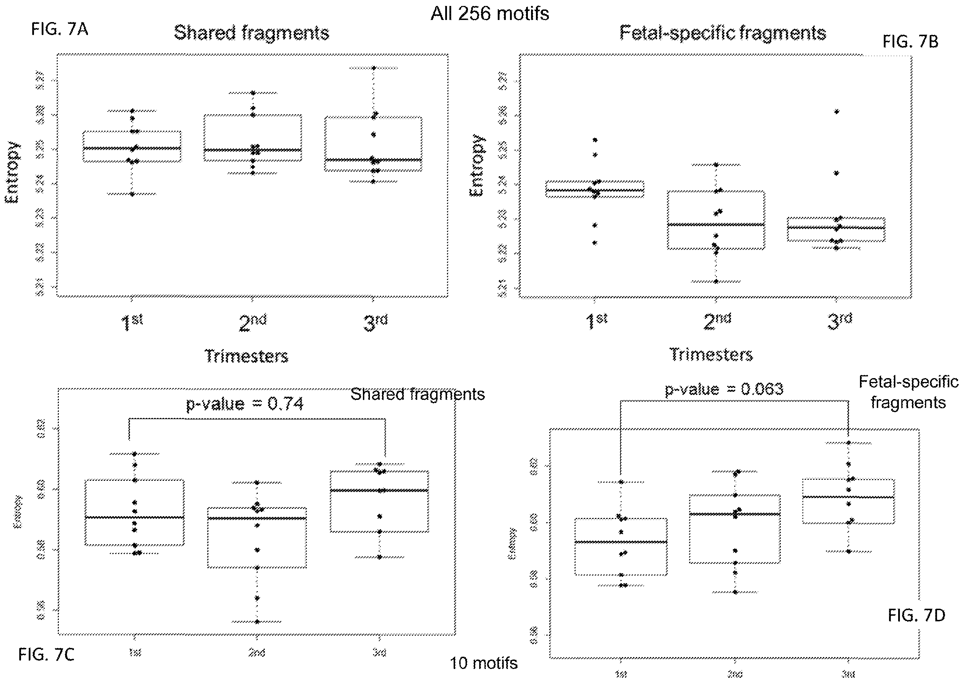

FIG. 7A and 7B show entropy distributions using all motifs for pregnant women across different trimesters according to embodiments of the present disclosure. Interestingly, the entropy values of numbers of end motifs determined using fetal-specific fragments appeared to be associated with gestational ages (p-value: 0.024, 1st trimester data versus pooled data from 2nd & 3rd trimesters), but those from shared fragments (predominantly maternal DNA) appeared not to be associated with gestational ages (p-value: 1, 1st trimester data versus pooled data from 2nd & 3rd trimesters). Later gestation, generally has higher fetal DNA concentrations. Thus, there can be a correlation between concentration and gestational age.

For the fetal-specific fragments, compared to the first trimester, the second and third trimester have a reduced entropy. Thus, the fetal fragments can convey gestational age. And, since the shared fragments have essentially a constant entropy (e.g., due to being mostly maternal fragments and/or maternal physiology-associated changes in end motifs canceling out such fetal signals), a change in entropy for all fragments will reflect the gestational age due to the change in the fetal fragments. Such a relationship of the entropy among the different trimesters will show less change due to the existence of the maternal fragments, but the relationship will still exist But, when fetal-specific alleles can be identified (e.g., a male fetus or by identifying alleles that occur at a percentage similar to an expected fetal DNA concentration, or using paternal genotype information), then a more pronounced relationship would exist (e.g., as shown in FIG. 7B).

FIGS. 7C and 7D show entropy distributions using 10 motifs for pregnant women across different trimesters according to embodiments of the present disclosure. The 10 motifs were selected via a ranking determined from the shared fragments. These figures show that the entropy still changes for different trimesters for the fetal-specific fragments, even if the relationship may be a decrease (as opposed to the increase in FIG. 7B), due to the specific selection of motifs.

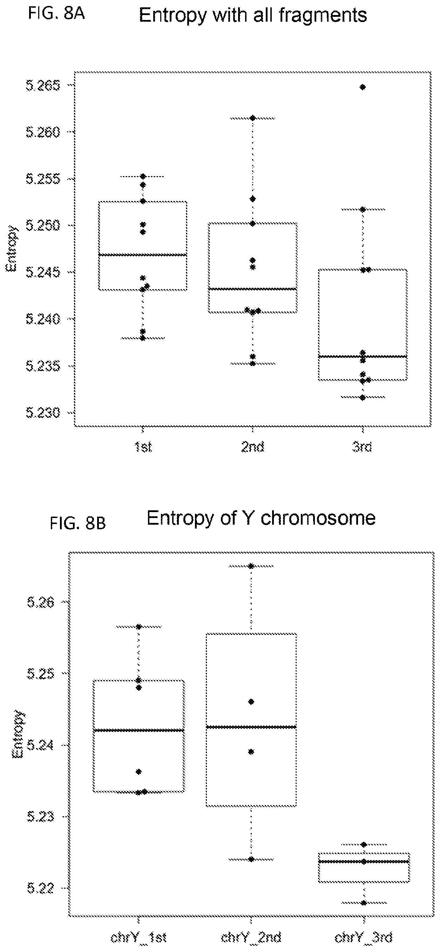

FIG. 8A shows the entropy for all fragments across different gestational ages according to embodiments of the present disclosure. The entropy is determined using all 256 4-mer end motifs. The entropy of plasma DNA fragments in subjects with the 3rd trimester was shown to be lower (p-value=0.06) than those with the 1st and 2nd trimester. And, the average for the 2nd trimester is lower than the 1st trimester. Thus, when all of the fetal fragments are included (as opposed to shared fragments in FIG. 7A), the entropy does provide a gestational age.

FIG. 8B shows the entropy for Y chromosome derived fragments across different gestational ages. The entropy of Y chromosome derived fragments in subjects with the 3rd trimester was shown to be lower (p-value=0.01) than those with the 1st and 2nd trimester. These samples that are filtered for fetal molecules (using the fetal-specific sequences from the Y chromosome) show are larger separation between the 3rd trimester and the 2nd trimester.

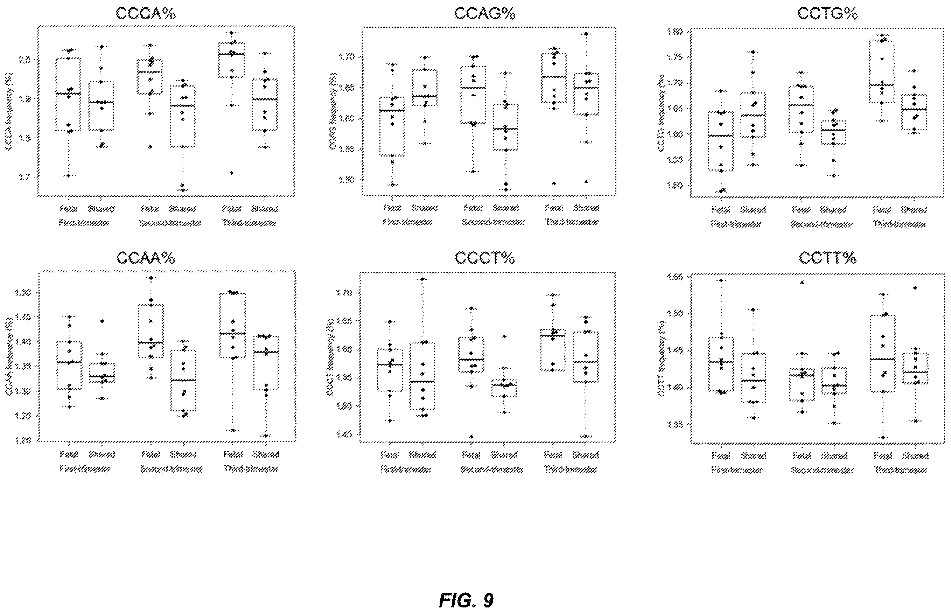

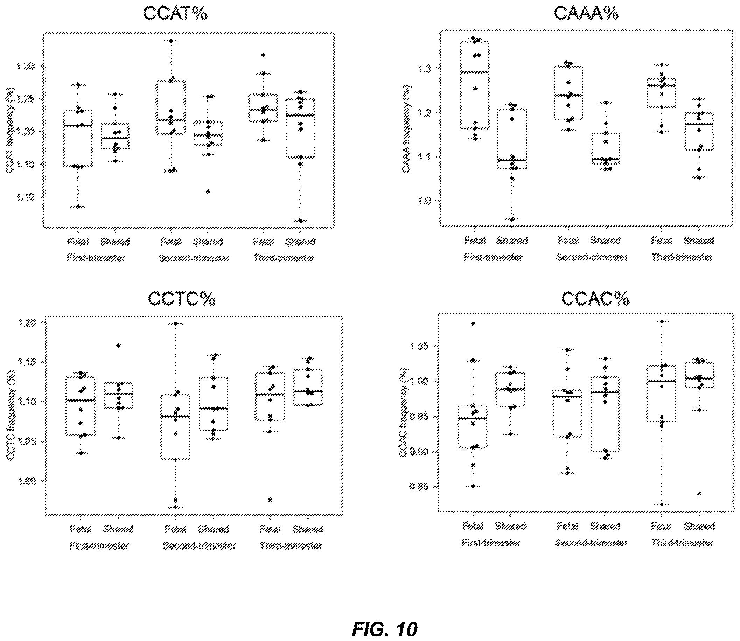

FIGS. 9 and 10 show the distribution of the top 10 ranked end motifs between fetal and maternal DNA molecules across different trimesters according to embodiments of the present disclosure. The top 10 end motifs in the ranked difference in motif frequency between fetal and maternal DNA molecules were mined from one single deep sequencing pregnant case. These top 10 end motifs were then used to analyze each of the samples.

The proportions of fetal and shared DNA molecules carrying these end motifs of interest were calculated in an independent cohort comprising 10 pregnant women from each of the first (12-14 weeks), second (20-23 weeks), and third (38-40 weeks) trimesters, respectively. There were a number of end motifs that were found to be higher in fetal DNA molecules compared with shared molecules, suggesting that those end motifs bear a certain relationship with the tissue of origin. For example, the median of CAAA% was found to be consistently higher in fetal DNA molecules than that in shared molecules (mainly maternal) across the first (1.26% versus 1.11 %), second (1.24% versus 1.11 %), and third (1.24% versus 1.15 %) trimesters. Thus, an ending motif CAAA can be identified as a marker that indicates an increased likelihood that a particular DNA fragment having an ending sequence of CAAA is from the fetus.

Certain end motifs show a more pronounced relationship to gestational age. For example, the fetal DNA molecules having an end motif CCCA shows a continual (monotonic) increase with gestational age, as also do CCAG, CCTG, CCAA, CCCT, and CCAC. However, CCTT does not show a continual increase as the median dips for the 2nd trimester, and then increases for the 3rd trimester.

In another embodiment, one could combine the top 10 ranked end motifs to see the difference between fetal and maternal DNA molecules across different trimesters.

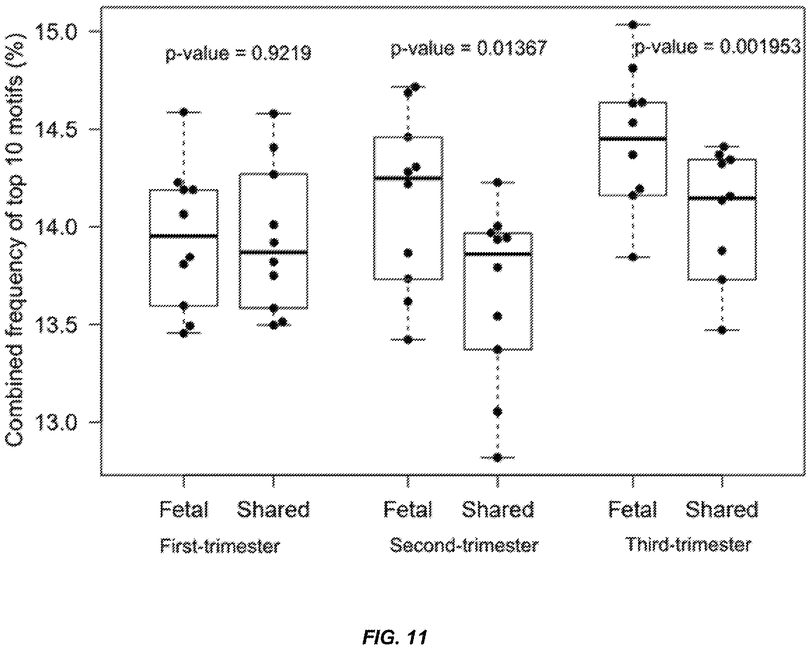

FIG. 11 shows a combined frequency of top 10 ranked motifs between fetal and shared molecules across different trimesters according to embodiments of the present disclosure. As shown in FIG. 11, we found that the difference in combined frequency of top 10 ranked end motifs between fetal and maternal DNA molecules was relatively larger in both the 2nd trimester (p-value: 0.013) and 3rd trimester (p-value: 0.0019) in comparison with the 1st trimester (p-value: 0.92). The frequency for the fetal molecules increases continually 1st trimester to 2nd trimester to 3rd trimester, while this continual relationship is not shown for the shared molecules. This shows that different physiological conditions (e.g. gestational ages) would affect the end motifs derived from different tissue of origins.

*B. Oncology*

The genotypic means devised in the context of pregnancy could be also applied in the context of oncology.

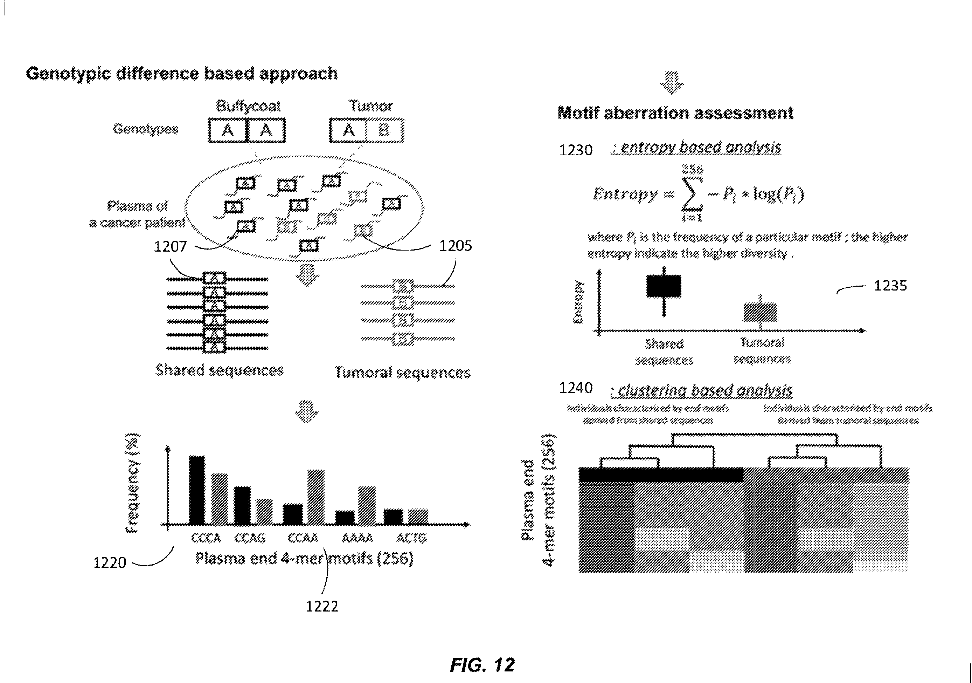

FIG. 12 shows a schematic of genotypic difference based approach to analyze the differential end motif patterns between mutant and shared molecules in the plasma DNA of a cancer patient according to embodiments of the present disclosure. As illustrated in FIG. 12, the tumor-specific molecules 1205 that carry the tumor-specific alleles (B) can be determined. On the other hand, the shared molecules 1207 that carry the shared allele (A) can be determined, which would represent the predominantly healthy-derived DNA molecules because the tumor DNA molecules generally would be the minority in the plasma DNA pool.

As an example, one could identify the mutant sequences (i.e. plasma DNA carrying cancer-associated mutations) and shared sequences (mainly hematopoietically derived DNA). The cancer-associated mutations could be defined as variants present in tumor tissues (hepatocellular carcinoma, HCC) but absent in normal cells (e.g. buffy coat). For example, in an HCC patient, assuming the genotype of tumor tissues was "AG" in a particular genomic locus and the genotype of buffy coat cells was "AA", the "G" specifically present in tumor tissues would be deemed as cancer-associated mutations, and "A" would be deemed as shared wildtype allele. In various implementations, the mutant sequence can be obtained by sequencing a tissue biopsy from the tumor or by analyzing a cell-free sample such as plasma or serum, e.g., as described in U.S. Patent Publication 2014/0100121.

The frequency profile of end motifs between mutant sequences and shared sequences was determined in an HCC patient whose plasma DNA was sequenced with a depth of 220x. Bar plot 1220 provides a relative frequency (%) that each 4-mer occurs as an end motif for mutant and shared sequences. Such relative frequencies can be determined as described above for bar plot 220 of FIG. 2. As one can see, an end motif 1222 has a significant difference of relative frequencies between DNA fragments of the different tissue types. Such a difference can be used for various purposes, e.g., to enrich a sample for tumor DNA or to determine a tumor DNA concentration.

In another embodiment, to capture the landscape difference in end motifs between tumor and shared DNA molecules, an entropy-based analysis 1230 can be used, similar to FIG. 2. Plot 1235 shows entropy values for the shared sequences and the tumor sequences. The difference in the entropy or other variance metric can provide a tumor fractional concentration, e.g., using a calibration function.

In yet another embodiment, a clustering-based analysis 1240 can be performed, similar to the fetal-analysis in FIG. 2. A classification for an amount of tumor sequences in a sample can be determined based on a new sample belonging to a reference cluster whose classification of tumor fraction is known.

### 1. Differences in relative frequencies in ranked order

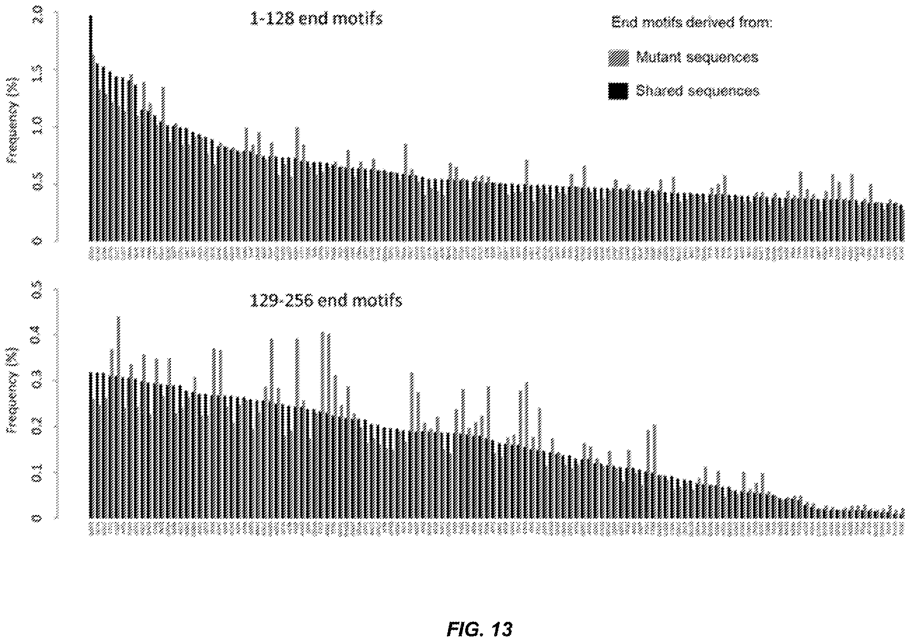

FIG. 13 shows the landscape of plasma DNA end motifs of cancer-associated mutant and shared molecules in hepatocellular carcinoma according to embodiments of the present disclosure. There were a number of end motifs that were observed to be altered between mutant and shared sequences, for example, but not limited to be, CCCA, CCAG, CCAA, CCTG, CCTT, CCCT, CAAA, CCAT, TAAA, AAAA motifs. FIG. 13 shows similar information as FIG. 3, but for the clinically-relevant DNA being tumor DNA as opposed to fetal DNA.

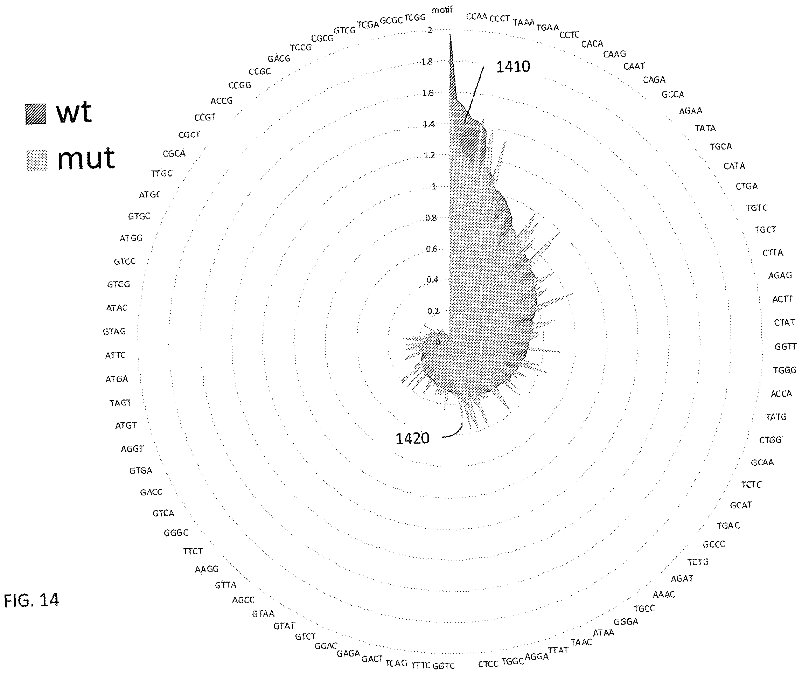

FIG. 14 shows a radial landscape of plasma DNA end motifs of cancer-associated mutant and shared molecules in hepatocellular carcinoma according to embodiments of the present disclosure. Different end motifs are listed on the outer circumference, and the frequency of an end motif is shown at different radial lengths. The end motifs are sorted by frequency of the wildtype (wt) allele of non-tumor (e.g., healthy) cells. The frequency values 1410 correspond to the wt alleles, and the frequency values 1420 correspond to the mutant (mut) allele. This radial view shows significant differences in the relative frequencies of the end motifs for the mutant sequences compared to the wildtype (shared) sequences.

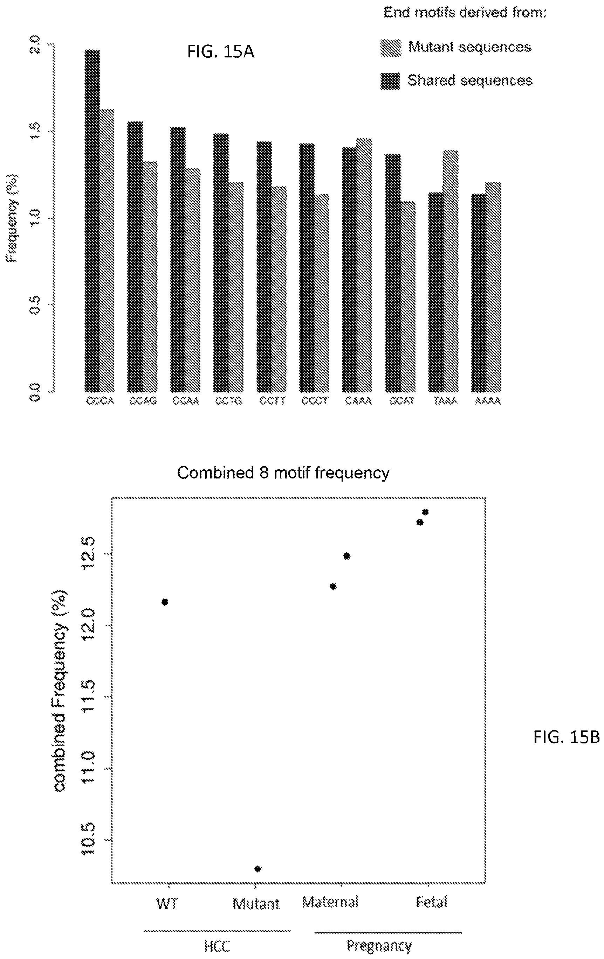

FIG. 15A shows the top 10 end motifs in the ranked difference of end motif frequencies between mutant and shared sequences in the plasma DNA of an HCC patient according to embodiments of the present disclosure. The top end motifs are determined for the shared sequences in a reference sample. As shown, the top end motifs are CCCA, CCAG, CCAA, CCTG, CCTT, CCCT, CAAA, CCAT, TAAA, and AAAA. The differential in the relative frequencies varies among the end motifs. For example, the motif (CCCA) showing most differences between mutant and shared sequences was found to be 1.9% and 1.6%, respectively, suggesting a 15% reduction in mutant sequences for such a motif relative to shared sequences (mainly blood cell-derived wildtype sequences).

FIG. 15B shows a combined frequency for 8 end motifs for an HCC patient and a pregnant female according to embodiments of the present disclosure. The combined frequency is an example aggregate value, e.g., as a sum of relative frequencies of a set of end motifs. As can be seen, there is a separation in the combined frequency for the two classes of sequences in each of these two scenarios: between the wildtype (WT) and mutant, and the maternal and fetal sequences. The separation for the combined frequency between the wildtype (WT) and mutant is larger than the separation for the maternal and fetal sequences.

This combined frequency shows a similar behavior as the entropy plots for the fetal analysis. Thus, FIG. 15B shows another example of an aggregate value of relative frequencies that can be used to determine a fractional concentration of clinically-relevant DNA. And, the wt vs mutant relationship in FIG. 15B shows that a fractional concentration of other clinically-relevant DNA (e.g., tumor DNA) can also be determined.

### 2. Use of entropy

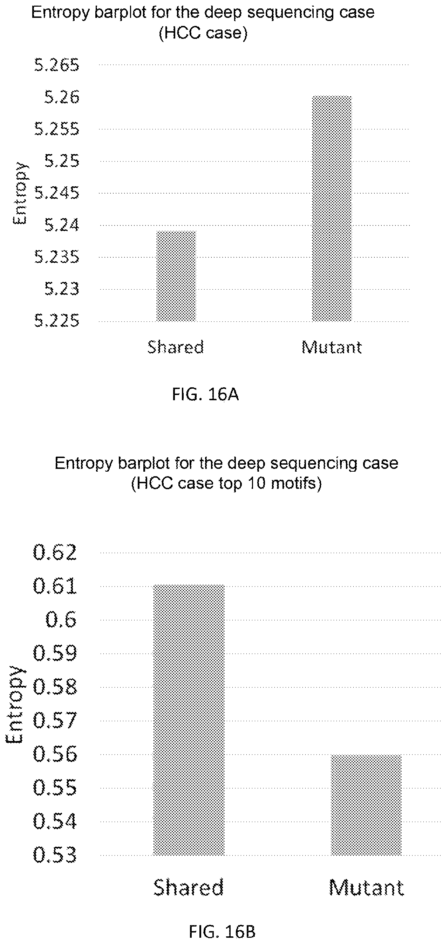

FIGS. 16A and 16B show entropy values for shared and mutant fragments for different sets of end motifs for an HCC case according to embodiments of the present disclosure. As with the fetal sequences, the relationship between the entropies for the two types of sequences can vary depending on the set of end motifs used. FIG. 16A uses all 256 end motifs for 4-mers. Due to a more uniform frequency distribution (e.g., more flat) for the mutant fragments, the entropy is higher for the mutant fragments. And, due to a higher skewness frequency distribution, the entropy for the shared fragments is lower.

FIG. 16B uses the top 10 end motifs for 4-mers that occur in the HCC subject for shared fragments. The relationship for the entropies are opposite for the top 10 motifs. Both FIG. 16A and 16B show that the calibration analysis for determining a fetal DNA concentration can also be used to determine a tumor DNA concentration.

As explained above, a higher entropy value indicates a higher diversity in the end motif. A motif diversity score (MDS) can be used to estimate a fractional concentration of clinically-relevant DNA (e.g., fetal, transplant, or tumor) in a biological sample of circulating cell-free DNA.

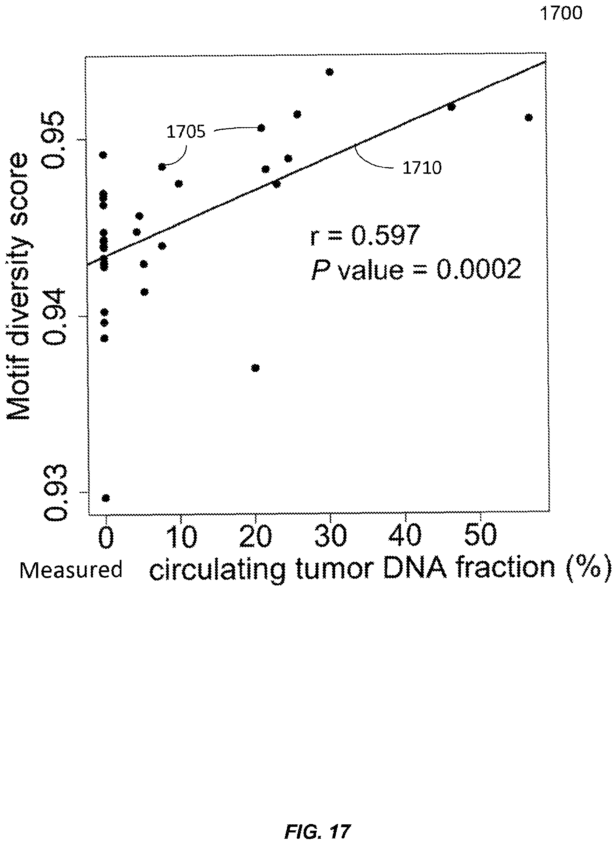

FIG. 17 is a plot of a motif diversity score against a measured circulating tumor DNA fraction according to embodiments of the present disclosure. For each of a plurality of calibration samples, calibration data points 1705 were measured. A calibration data point comprises a motif diversity score for the sample and a fractional concentration of clinically-relevant DNA, in this case a tumor DNA fraction. The tumor DNA fraction was estimated based on ichorCNA, a software package that measured the tumor DNA fraction in plasma DNA by taking advantage of cancer-associated copy number aberrations (Adalsteinsson et al. 2017).

A given sample may be a healthy control sample with no tumor DNA or a sample from a patient who has a tumor, where the tumor DNA fraction is non-zero, i.e., there is tumor DNA and other (e.g., healthy) DNA. The MDS values of plasma DNA of patients with HCC were found to be positively correlated with the tumor DNA fractions (Spearman's $\rho$: 0.597; p-value: 0.0002). This is shown with the calibration function 1710 (a linear function in this example).

Calibration function 1710 can be used to determine a tumor DNA fraction in new test samples for which a motif diversity score has been measured. Calibration function 1710 can be determined by a functional fit to the calibration data points 1705, e.g., using regression.

In some examples, a calculated value X of MDS for a new sample can be used as input into a function F(X), where F is the calibration function (curve). The output of F(X) is the fractional concentration. An error range can be provided, which may be different for each X value, thereby providing a range of values as an output of F(X). In other examples, the fractional concentration corresponding to a measurement of 0.95 for MDS in a new sample can be determined as the average concentration calculated from the calibration data points at an MDS of 0.95. As another example, the calibration data points 1705 may be used to provide a range of fractional DNA concentration for a particular calibration value, where the range can be used to determine if the fractional concentration is above a threshold amount.

### C. *Transplantation*

The genotypic technique can also be applied to monitor transplantation, for example, liver transplantation. The SNP sites where the recipient is homozygous and the donor is heterozygous would allow for determining the donor-specific DNA molecules and the predominantly hematopoietic DNA in plasma of a transplant patient.

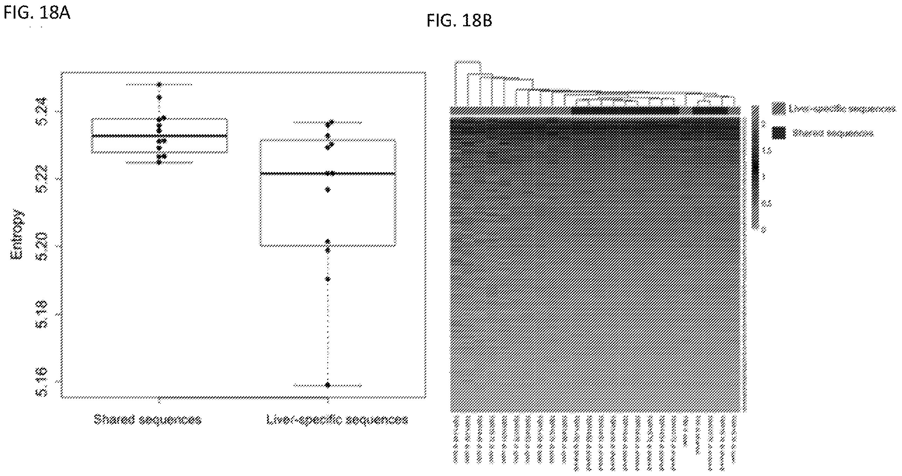

FIG. 18A shows an entropy analysis using donor-specific fragments according to embodiments of the present disclosure. FIG. 18B shows a hierarchical clustering analysis using donor-specific fragments. As shown in FIGS. 18A and 18B, in the context of liver transplantation, the liver-specific DNA molecules were observed to have different properties from the shared sequences (mainly blood-derived DNA). The entropy of plasma DNA end motifs was generally found to be lower in donor-specific DNA molecules (liver DNA) in comparison with shared sequences (FIG. 18A). The individuals characterized with end motifs derived from liver-specific DNA molecules were clustered together while the individuals characterized with end motifs derived from shared DNA molecules were clustered into another group.

*D. Classifying fractional concentration*

As described above, the relative frequencies of a set of one or more end motifs can be used to determine a classification of fractional concentration of clinically-relevant DNA.

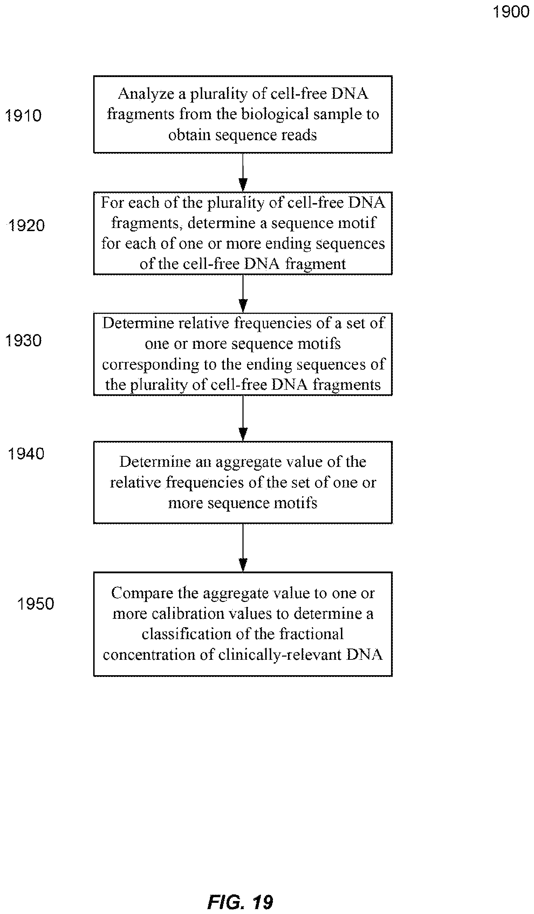

FIG. 19 is a flowchart illustrating a method 1900 of estimating a fractional concentration of clinically-relevant DNA in a biological sample of a subject according to embodiments of the present disclosure. The biological sample may include the clinically-relevant DNA and other DNA that are cell-free. In other examples, a biological sample may not include the clinically-relevant DNA, and the estimated fractional concentration may indicate zero or a low percentage of the clinically-relevant DNA. Aspects of method 1900 and any other methods described herein may be performed by a computer system.

At block 1910, a plurality of cell-free DNA fragments from the biological sample are analysed to obtain sequence reads. The sequence reads can include ending sequences corresponding to ends of the plurality of cell-free DNA fragments. As examples, the sequence reads can be obtained using sequencing or probe-based techniques, either of which may including enriching, e.g., via amplification or capture probes.

The sequencing may be performed in a variety of ways, e.g., using massively parallel sequencing or next-generation sequencing, using single molecule sequencing, and/or using double- or single-stranded DNA sequencing library preparation protocols. The skilled person will appreciate the variety of sequencing techniques that may be used. As part of the sequencing, it is possible that some of the sequence reads may correspond to cellular nucleic acids.

The sequencing may be targeted sequencing as described herein. For example, biological sample can be enriched for DNA fragments from a particular region. The enriching can include using capture probes that bind to a portion of, or an entire genome, e.g., as defined by a reference genome.

A statistically significant number of cell-free DNA molecules can be analyzed so as to provide an accurate determination of the fractional concentration. In some embodiments, at least 1,000 cell-free DNA molecules are analyzed. In other embodiments, at least 10,000 or 50,000 or 100,000 or 500,000 or 1,000,000 or 5,000,000 cell-free DNA molecules, or more, can be analyzed.

At block 1920, for each of the plurality of cell-free DNA fragments, a sequence motif is determined for each of one or more ending sequences of the cell-free DNA fragment. The sequence motifs can include N base positions (e.g., 1, 2, 3, 4, 5, 6, etc.). As examples, the sequence motif can be determined by analyzing the sequence read at an end corresponding to the end of the DNA fragment, correlating a signal with a particular motif (e.g., when a probe is used), and/or aligning a sequence read to a reference genome, e.g., as described in FIG. 1.

For example, after sequencing by a sequencing device, the sequence reads may be received by a computer system, which may be communicably coupled to a sequencing device that performed the sequencing, e.g., via wired or wireless communications or via a detachable memory device. In some implementations, one or more sequence reads that include both ends of the nucleic acid fragment can be received. The location of a DNA molecule can be determined by mapping (aligning) the one or more sequence reads of the DNA molecule to respective parts of the human genome, e.g., to specific regions. In other embodiments, a particular probe (e.g., following PCR or other amplification) can indicate a location or a particular end motif, such as via a particular fluorescent color. The identification can be that the cell-free DNA molecule corresponds to one of a set of sequence motifs.

At block 1930, relative frequencies of a set of one or more sequence motifs corresponding to the ending sequences of the plurality of cell-free DNA fragments is determined. A relative frequency of a sequence motif can provide a proportion of the plurality of cell-free DNA fragments that have an ending sequence corresponding to the sequence motif. The set of one or more sequence motifs can be identified using a reference set of one or more reference samples. The fractional concentration of clinically-relevant DNA need not be known for a reference sample, although genotypic differences may be determined so that differences between the end motifs of the clinically-relevant DNA and the other DNA (e.g., healthy DNA, maternal DNA, or DNA of a subject how received a transplanted organ) may be identified. Particular end motifs can be selected on the basis of the differences (e.g., to select the end motifs with the highest absolute or percentage difference). Examples of relative frequencies are described throughout the disclosure.

In some implementations, the sequence motifs include N base positions, where the set of one or more sequence motifs include all combinations of N bases. In some example, N can be an integer equal to or greater than two or three. The set of one or more sequence motifs can be a top M (e.g., 10) most frequent sequence motifs occurring in the one or more calibration samples or other reference sample not used for calibrating the fractional concentration.

At block 1940, an aggregate value of the relative frequencies of the set of one or more sequence motifs is determined. Example aggregate values are described throughout the disclosure, e.g., including an entropy value (a motif diversity score), a sum of relative frequencies, and a multidimensional data point corresponding to a vector of counts for a set of motifs (e.g., a vector 256 counts for 245 motifs of possible 4-mers or 64 counts for 64 motifs of possible 3-mers). When the set of one or more sequence motifs includes a plurality of sequence motifs, the aggregate value can include a sum of the relative frequencies of the set.

As an example, when the set of one or more sequence motifs includes a plurality of sequence motifs, the aggregate value can include a sum of the relative frequencies of the set. As another example, the aggregate value can correspond to a variance in the relative frequencies. For instance, the aggregate value can include an entropy term. The entropy term can include a sum of terms, each term including a relative frequency multiplied by a logarithm of the relative frequency. As another example, the aggregate value can include a final or intermediate output of a machine learning model, e.g., clustering model.

At block 1950, a classification of the fractional concentration of clinically-relevant DNA in the biological sample is determined by comparing the aggregate value to one or more calibration values. The one or more calibration values can be determined from one or more calibration samples whose fractional concentration of clinically-relevant DNA are known (e.g., measured). The comparison can be to a plurality of calibration values. The comparison can occur by inputting the aggregate value into a calibration function fit to the calibration data that provides a change in the aggregate value relative to a change in the fractional concentration of the clinically-relevant DNA in the sample. As another example, the one or more calibration values can correspond to one or more aggregate values of the relative frequencies of the set of one or more sequence motifs that are measured using cell-free DNA fragments in the one or more calibration samples.

A calibration value can be calculated as an aggregate value for each calibration sample. A calibration data point may be determined for each sample, where the calibration data point includes the calibration value and the measured fractional concentration for the sample. These calibration data points can be used in method 1900, or can be used to determine the final calibration data points (e.g., as defined via a functional fit). For example, a linear function could be fit to the calibration values as a function of fractional concentration. The linear function can define the calibration data points to be used in method 1900. The new aggregate value of a new sample can be used as an input to the function as part of the comparison to provide an output fractional concentration. Accordingly, the one or more calibration values can be a plurality of calibration values of a calibration function that is determined using fractional concentrations of clinically-relevant DNA of a plurality of calibration samples.

As another example, the new aggregate value can be compared to an average aggregate value for samples having a same classification of fractional concentrations (e.g., in a same range), and if the new aggregate value is closer to this average than a calibration value to the average for another classification, the new sample can be determined to have a same concentration as the closest calibration value. Such a technique may be used when clustering is performed. For example, the calibration value can be a representative value for a cluster that corresponds to a particular classification of the fractional concentration.

The determination of calibration data point can include measuring a fractional concentration, e.g., as follows. For each calibration sample of the one or more calibration samples, the fractional concentration of clinically-relevant DNA can be measured in the calibration sample. The aggregate value of the relative frequencies of the set of one or more sequence motifs can be determined by analyzing cell-free DNA fragments from the calibration sample as part of obtaining a calibration data point, thereby determining one or more aggregate values. Each calibration data point can specify the measured fractional concentration of clinically-relevant DNA in the calibration sample and the aggregate value determined for the calibration sample. The one or more calibration values can be the one or more aggregate values or be determined using the one or more aggregate values (e.g., when using a calibration function). The measurement of the fractional concentration can be performed in various ways as described herein, e.g., by using an allele specific to the clinically-relevant DNA.

In various embodiments, measuring a fractional concentration of clinically-relevant DNA can be performed using a tissue-specific allele or epigenetic marker, or using a size of DNA fragments, e.g., as described in US Patent Publication 2013/0237431. Tissue-specific epigenetic markers can include DNA sequences that exhibit tissue-specific DNA methylation patterns in the sample.

In various embodiments, the clinically-relevant DNA can be selected from a group consisting of fetal DNA, tumor DNA, DNA from a transplanted organ, and a particular tissue type (e.g., from a particular organ). The clinically-relevant DNA can be of a particular tissue type, e.g., the particular tissue type is liver or hematopoietic. When the subject is a pregnant female, the clinically-relevant DNA can be placental tissue, which corresponds to fetal DNA. As another example, the clinically-relevant DNA can be tumor DNA derived from an organ that has cancer.

Generally, it is preferred for the one or more calibration values determined from one or more calibration samples to be generated using a similar assay as used for the biological (test) sample for which the fractional concentration is being measured. For example, a sequencing library can be generated in a same manner. Two example processing techniques are GeneRead (www.qiagen.com/us/shop/sequencing/generead-size-selection-kit/#orderinginformation) and SPRI (solid phase reversible immobilization, AMPure bead, www.beckman.hk/reagents_depr/genomic_depr/cleanup-and-size-selection/pcr). GeneRead can remove the short DNA, which are predominantly tumor fragments, which can affect the relative frequencies of the end motifs for the wildtype and mutant fragments, as well as for the fetal and transplant cases.

*E. Determining gestational age*

As described above in FIGS. 7A, 7B, and 8-10, the fetal-specific fragment motifs can be used to infer the gestational age.

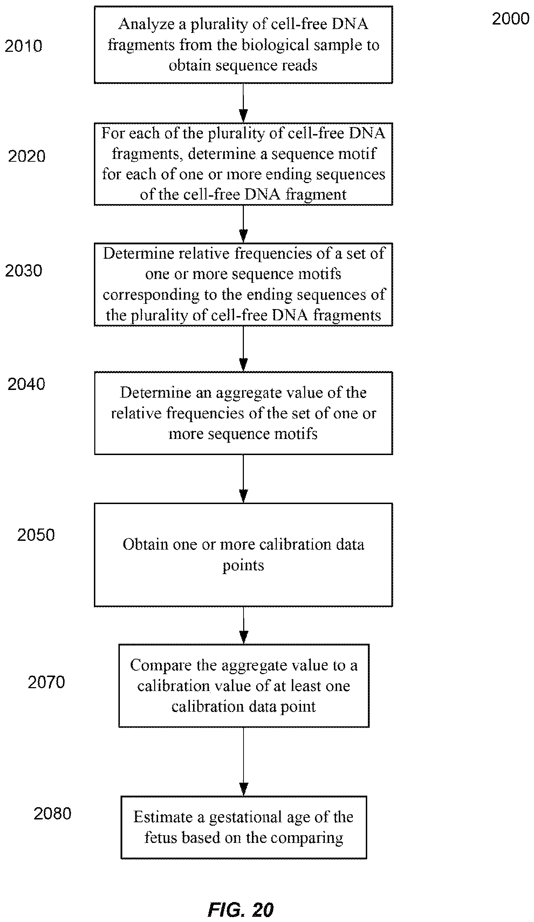

FIG. 20 is a flowchart illustrating a method 2000 of determining a gestational age of a fetus by analyzing a biological sample from a female subject pregnant with a fetus according to embodiments of the present disclosure. The biological sample includes cell-free DNA molecules from the female subject and the fetus.

At block 2010, a plurality of cell-free DNA fragments from the biological sample are analyzed to obtain sequence reads. The sequence reads can include ending sequences corresponding to ends of the plurality of cell-free DNA fragments. Block 2010 may be performed in a similar manner as block 1910 of FIG. 19.

Before, after, or as part of the analyzing, the plurality of cell-free DNA fragments can be identified as being derived from the fetus, e.g., as described above for FIGS. 2 and 5A. This can filter the DNA fragments for ones that are fetal or most likely fetal. As examples, the plurality of cell-free DNA fragments can be identified using a fetal-specific allele or a fetal-specific epigenetic marker. As another example, for each of the sequence reads, a likelihood that the sequence read corresponds to the fetus can be determined based on an ending sequence of the sequence read including a sequence motif of the set of one or more sequence motifs. Other criteria can also be used, e.g., as described in section II.E. The likelihood can be compared to a threshold, and the sequence read can be identified as being derived from the fetus when the likelihood exceeds the threshold. Further details on enriching a sample for clinically-relevant DNA can be found in section IV.

At block 2020, for each of the plurality of cell-free DNA fragments, a sequence motif is determined for each of one or more ending sequences of the cell-free DNA fragment. Block 2020 may be performed in a similar manner as block 2020 of FIG. 19.

At block 2030, relative frequencies of a set of one or more sequence motifs corresponding to the ending sequences of the plurality of cell-free DNA fragments are determined. A relative frequency of a sequence motif can provide a proportion of the plurality of cell-free DNA fragments that have an ending sequence corresponding to the sequence motif. Block 2030 may be performed in a similar manner as block 1930 of FIG. 19.

At block 2040, an aggregate value of the relative frequencies of the set of one or more sequence motifs is determined. Block 2040 may be performed in a similar manner as block 1940 of FIG. 19.

At block 2050, one or more calibration data points are obtained. Each calibration data point can specify a gestational age (e.g., trimester as described in the figures above) corresponding to an aggregate value. As described above, the one or more calibration data points can be determined from a plurality of calibration samples with known gestational ages and including cell-free DNA molecules. In some implementations, the one or more calibration data points can be a plurality of calibration data points that form a calibration function that approximates measured aggregate values determined from the cell-free DNA molecules in the plurality of calibration samples with known gestational ages.

At block 2060, the aggregate value is compared to a calibration value of at least one calibration data point. For example, a new aggregate value of a new sample can be compared to the average for the 3$^{rd}$ trimester as determined in FIG. 8A. As another example, the calibration value of the at least one calibration data point can correspond to the aggregate value measured using the cell-free DNA molecules in at least one of the plurality of calibration samples. The comparison of the aggregate value can be to a plurality of calibration values, e.g., each corresponding to one of the plurality of calibration samples. The comparison can occur by inputting the aggregate value into a function fit (calibration function) to the calibration data that provides a change in the aggregate value relative to gestational age. The comparison can be performed in a similar manner described for method 1900, e.g., in relation to block 1950.

At block 2070, a gestational age of the fetus is estimated based on the comparing. For example, if the new aggregate value is closest to the 3$^{rd}$ trimester average (or other calibration value used), then the new sample can be determined to be in the 3$^{rd}$ trimester. As another example, the new aggregate value can be compared to a calibration function (e.g., linear function) that is fit to the data in FIG. 8A or other similar figures. The function can output the gestational age, e.g., as the Y value of the linear function. Other examples provided herein for the use of a calibration function can also be used in the context of determining a gestational age.

**III. PHENOTYPE APPROACHES**

Using genotypic based analyses for pregnant subjects, cancer subjects as well as liver transplantation, the presence of plasma DNA end motifs bore the relationship with the tissue of origin. We reasoned that, in cancer patients, the tumor DNA released into the blood circulation, thus altering the original normal presentation of plasma DNA end motifs. However, we do not exclude the possibility that other aspects of the pathobiology of cancer e.g., the tumor microenvironment (infiltrating T cells, B cells, neutrophils etc.) would generate different end motifs, exerting influence on the landscape of end motifs. Thus, the analysis of plasma DNA end motifs between cancer subjects and non-cancers control subjects would reveal the power of classifying HCC from control subjects.

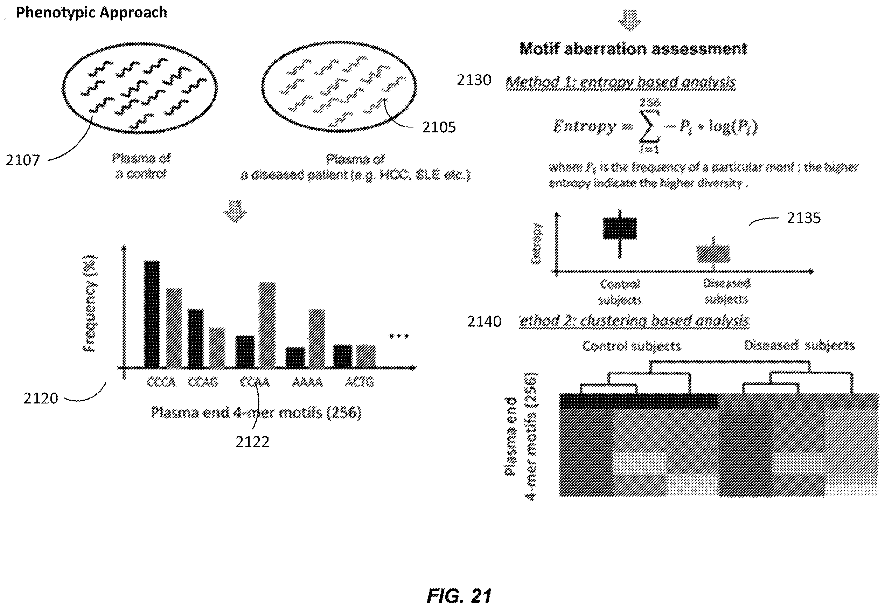

FIG. 21 shows a schematic of a phenotypic approach for plasma DNA end motif analysis according to embodiments of the present disclosure. FIG. 21 has similarities to FIGS. 2 and 12, e.g., that relative frequencies can be plotted, a variance value (e.g., entropy) can be determined, and clustering can be performed.

In FIG. 21, end motifs (e.g., 4-mers) deduced from plasma DNA molecules are used and compared between cancer and control subjects, thereby obviating the restriction of genotypic markers and making it broadly applicable in many clinical scenarios, for example, detection of autoimmune disease (e.g. systemic lupus erythematosus, SLE) and transplantation. Using the phenotypic approach with the use of all sequenced plasma DNA fragments, the entropy and clustering analysis could be performed in very similar analytical procedures as it was done in genotypic difference based approach. In this context, the entropy analysis and clustering analysis would be compared between control and diseased subjects.

The diseased molecules 2105 are from one or more subjects that is determined to have the disease. The control molecules 2107 are from one or more subjects that does not have the disease. The relative frequencies for a set of end motifs are determined for the two pools of molecules. Bar plot 1220 provides a relative frequency (%) that each 4-mer occurs as an end motif for control and diseased sequences. Such relative frequencies can be determined as described above for bar plot 220 of FIG. 2. As one can see, an end motif 2122 has a significant difference of relative frequencies between DNA fragments of the different tissue types. Such a difference can be used for various purposes, e.g., to classify a new sample as diseased or not diseased, or some other level of the disease.

To capture the landscape difference in end motifs between tumor and shared DNA molecules, an entropy-based analysis 2130 can be used, similar to FIG. 2. Plot 2135 shows entropy values for the control subjects and the diseased subjects. The difference in the entropy or other variance metric can provide a classification of a level of pathology relating to the disease.

In yet another embodiment, a clustering-based analysis 2140 can be performed, similar to the fetal

analysis in FIG. 2 and the tumor analysis in FIG. 12. A classification for a level of pathology can be determined based on a new sample belonging to a reference cluster whose classification is known.

Accordingly, in one example of an aggregate value of relative frequencies, each individual can be characterized by a vector comprising 256 frequencies regarding 4-mer end motifs (i.e. a 256-dimensional vector). In other examples, the standard deviation (SD), the coefficient of variation (CV), interquartile range (IQR) or a certain percentile cutoff (e.g. 95th or 99th percentile) among different motif frequencies can be used for assessing the landscape changes of end motif patterns between disease and control groups. Other examples of aggregate values are also provided in other sections and are applicable here.

*A. Oncology*

In some embodiments, the disease (pathology) can be cancer. Thus, some embodiments can classify a level of cancer.

### 1. Differences in relative frequencies in ranked order

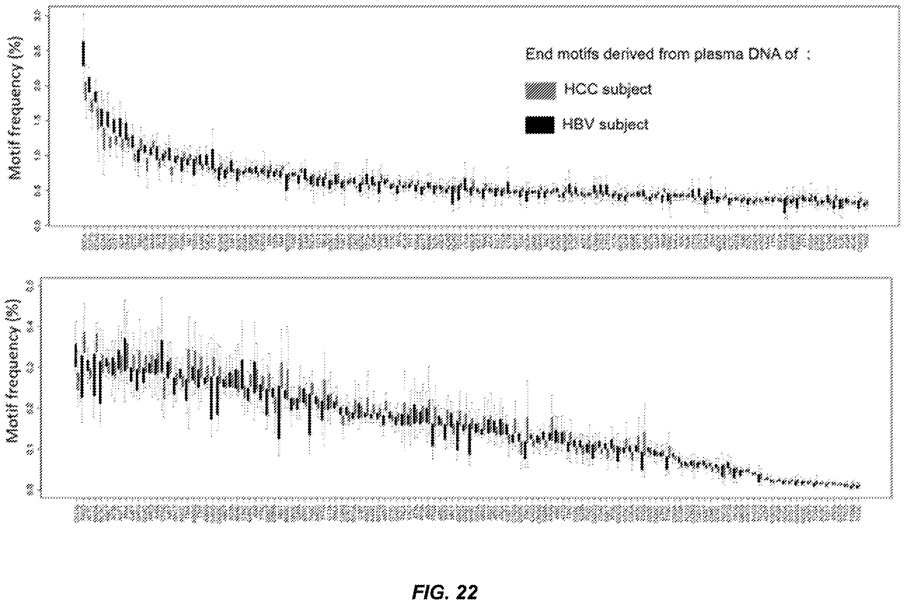

FIG. 22 shows an example for the frequency profile of 4-mer end motifs between hepatocellular carcinoma (HCC) and hepatitis B virus (HBV) subjects with the use of all plasma DNA molecules according to embodiments of the present disclosure. FIG. 22 compares the frequencies of 256 end motifs in an HCC patient with one HBV subject. As with similar plots, the vertical axis is motif frequency and the horizontal axis corresponds to respective end motifs. In FIG. 22, we ranked the motifs in an ascending order based on the mean of motif frequency in non-HCC subjects. The bottom plot continues the top plot, but at a different scale for ease of illustration.

There were a number of end motifs showing aberrations in the HCC patient. For example, compared with the HBV subject, the top 10 ranked end motifs (TGGG, TAAA, AAAA, GAAA, GGAG, TAGA, GCAG, TGGT, GCTG, and GAGA) that showed an increase of its frequency in the HCC patient had a mean 1.22 fold change, with a range of 1.12-1.35 fold change; and the top 10 ranked end motifs (CCCA, CCAG, CCAA, CCCT, CCTG, CCAC, CCAT, CCCC, CCTC, and CCTT) that showed a decrease in its frequency in HCC patients had a mean 1.23 fold change, with a range of 1.16-1.29 fold change. Such sets of top motifs showing an increase (or decrease as a separate set) of its frequency in the HCC group relative to a non-cancer group can be used to classify a new subject regarding cancer. As another example, a ranking process could choose all those motifs showing an increase in HCC, and then rank those motifs according to AUC between HCC and non-HCC subjects in a descending order. Then choose the top 10 motifs based on AUC values.

To test the diagnostic potential by using the plasma DNA end motifs, we sequenced 20 healthy control subjects (Control), 22 chronic hepatitis B carriers (HBV), 12 cirrhosis subjects (Cirr), 24 early-stage HCC (eHCC), 11 immediate-stage HCC (iHCC), and 7 advanced-stage HCC (aHCC) with a median paired-reads of 215 million (range: 97-1,681 million).

FIG. 23A shows a boxplot for the combined frequencies of top 10 plasma DNA 4-mer end motifs for various subjects having different levels of cancer according to embodiments of the present disclosure. The top 10 plasma DNA 4-mer end motifs were selected based on data in FIG. 22, i.e., based on frequency in HBV subject. The combined frequency is a sum of the frequencies of the 10 end motifs for a given subject. We found that the combined frequency of the top 10 ranked end motifs was significantly reduced in HCC patients compared with non-cancer subjects (p-value < 0.0001). Importantly, using this end motif analysis, 58.3% of eHCC patients could be identified at a specificity of 95%. Further, different stages of cancer can be detected. For example, the advanced HCC has substantially lower values than eHCC and iHCC.

FIG. 23B shows a Receiver Operating Characteristic (ROC) curve of the combined frequencies of top 10 plasma DNA 4-mer end motifs between HCC and non-cancer subjects according to embodiments of the present disclosure. The area under the curve (AUC) of ROC curve was found to be 0.91, showing that the plasma DNA end motifs indeed bore the clinical potential of distinguishing the HCC from non-cancer subjects. In another embodiment, a combined frequency of seven end motifs having a largest separation between the HCC subjects and non-HCC subjects provides an AUC of 0.92.

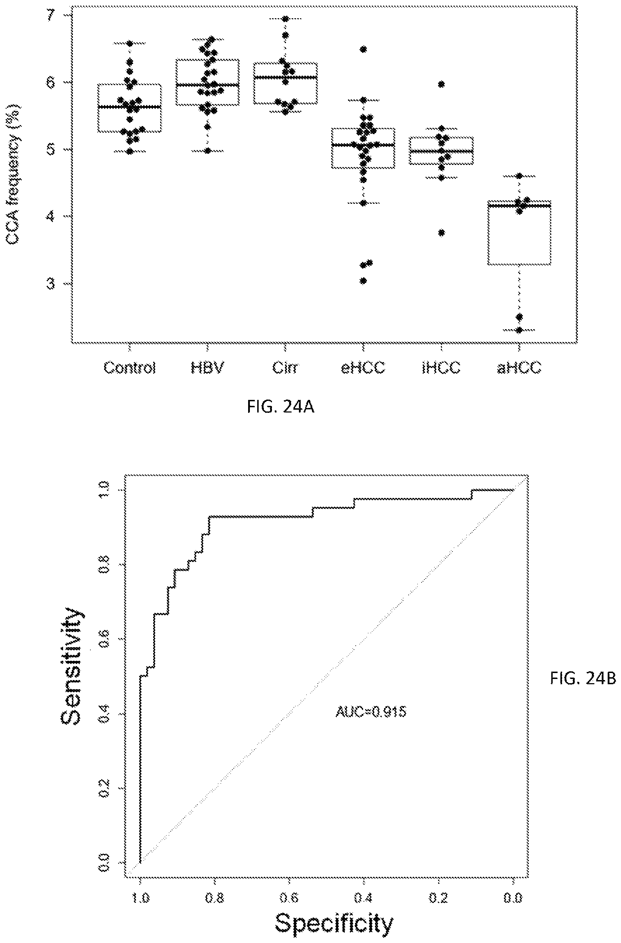

FIG. 24A shows a boxplot of the frequency of CCA motif across different groups according to embodiments of the present disclosure. The most frequent 3-mer motif (CCA) in non-HCC group was shown to be significantly lower in HCC group (p-value < 0.0001). FIG. 24B shows an ROC curve between non-HCC and HCC groups using the most frequent 3-mer motif (CCA) present in non-HCC subjects according to embodiments of the present disclosure. The AUC was found to be 0.915. A most frequent 4-mer (CCCA) also provides a similar AUC of 0.91.

### 2. Use of entropy (motif diversity score)

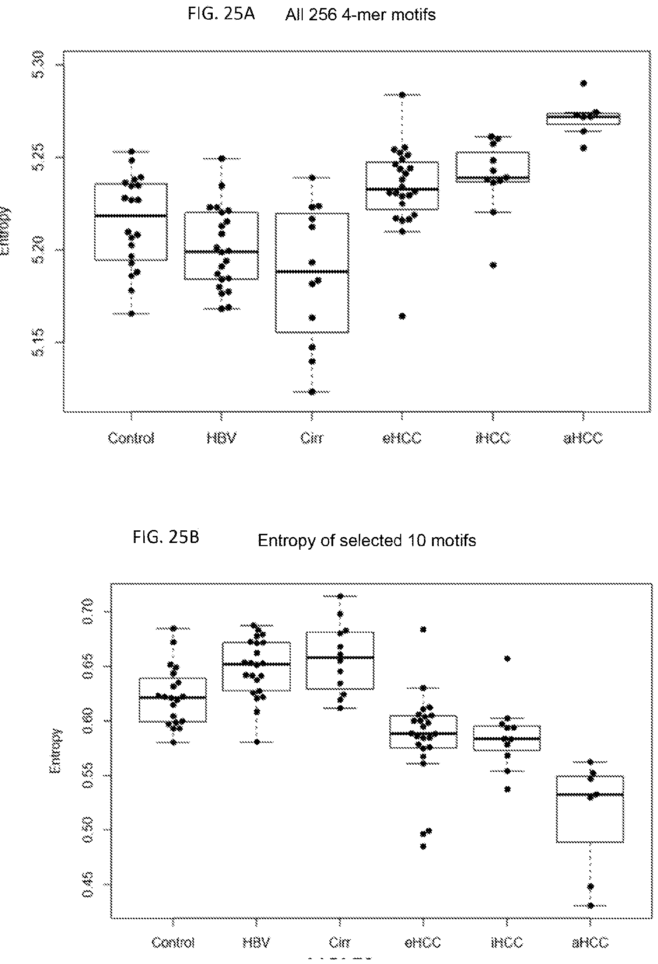

FIG. 25A shows a boxplot of entropy values across different groups using 256 4-mer end motifs according to embodiments of the present disclosure. All 256 motifs of 4-mers were used. As shown in FIG. 25A, the entropy values were significantly increased (p-values < 0.0001) in HCC patients (mean: 5.242; range: 5.164 - 5.29) compared with non-HCC subjects (mean: 5.203; range: 5.124 - 5.253). Importantly, using this end motif analysis, 41.7% of eHCC patients could be identified at a specificity of 95%. Entropy increased generally in the HCC, IHCC, and advanced stage HCC group compared to non-HCC group. Further, different stages of cancer can be detected. For example, the advanced HCC has substantially higher values than eHCC and iHCC.

FIG. 25B shows a boxplot of entropy values across different groups using 10 4-mer end motifs according to embodiments of the present disclosure. Here, the HCC subjects have an entropy that is decreased relative to the non-HCC subjects. Thus, the set of end motifs used can alter the relationship from an increase to a decrease. For example, using the top 10 motifs, there is a reduction in entropy in the HCC group. Either way, there is a diagnostic power between HCC and non HCC group, as well as advanced HCC relative to earlier stages of HCC.

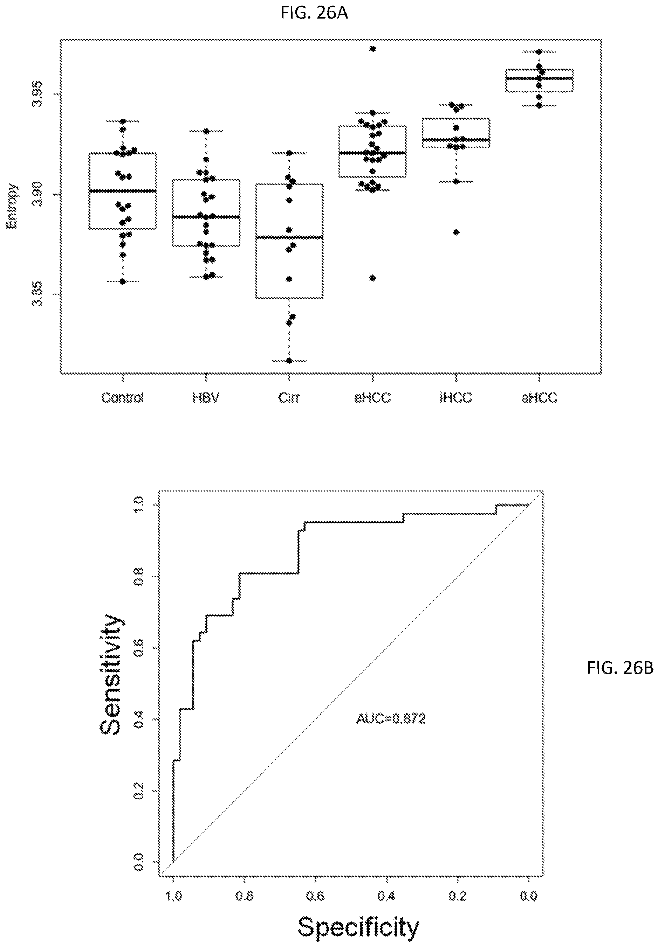

FIG. 26A shows a boxplot of entropy values using 3-mer motifs across different groups according to embodiments of the present disclosure. The entropy of HCC subjects using 3-mer motifs (a total of 64 motifs) was found to be significantly higher (p-value < 0.0001) than that of non-HCC subjects. FIG. 26B shows an ROC curve using the entropy of 64 3-mer motifs between non-HCC and HCC groups according to embodiments of the present disclosure. The AUC was found to be 0.872.

As explained above, a higher entropy value indicates a higher diversity in the end motif. As a further illustration of an ability of embodiments that use a motif diversity score to discriminate between various cancer types and control (e.g., healthy) samples, data from a published study was used.

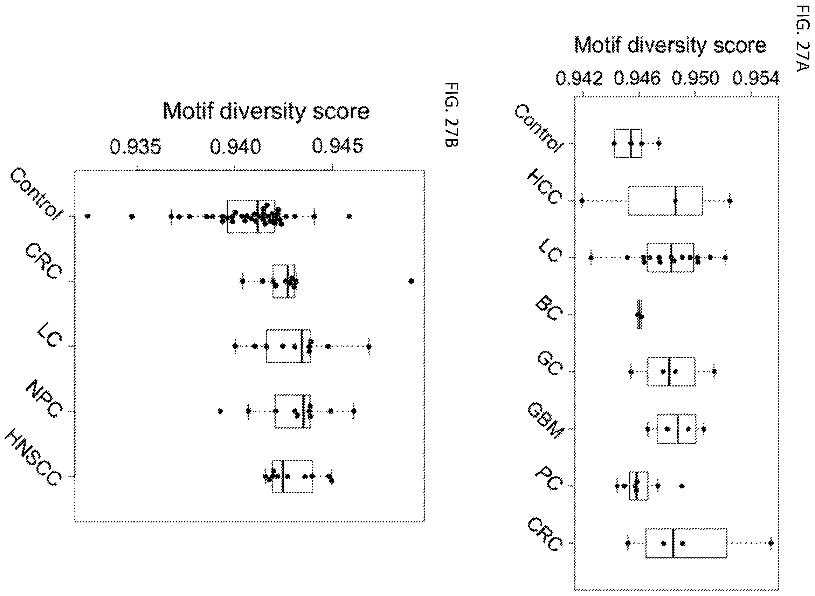

FIGS. 27A and 27B show boxplots of motif diversity scores using 4-mers across different groups according to embodiments of the present disclosure. All 256 4-mers were used to determine the motif diversity scores. The increase of plasma DNA end diversity could be generally observed among various cancer types when we performed MDS analysis using sequencing results of plasma DNA downloaded from a published study (Song et al. 2017), which may reflect the fact that different tumor cells from different anatomical sites would shed their DNA into the blood circulation (Bettegowda et al. 2014). The cancers analyzed were: hepatocellular carcinoma (HCC), lung cancer (LC), breast cancer (BC), gastric cancer (GC), glioblastoma multiforme (GBM), pancreatic cancer (PC), and colorectal cancer (CRC).

To further test the generalizability of MDS changes across different cancer types, we further sequenced an independent cohort with 40 plasma DNA samples of other cancer types, including patients with colorectal cancer (n=10), lung cancer (n=10), nasopharyngeal carcinoma (n=10), and head and neck squamous cell carcinoma (n=10), with a median of 42 million paired-end reads (range: 19 - 65 million). As shown in FIG. 27B, the MDS values in the group of patients with cancer (median: 0.943; range: 0.939-0.949) were significantly higher than the control group without cancer (median: 0.941; range: 0.933- 0.946; p-value < 0.0001, Wilcoxon sum-rank test).

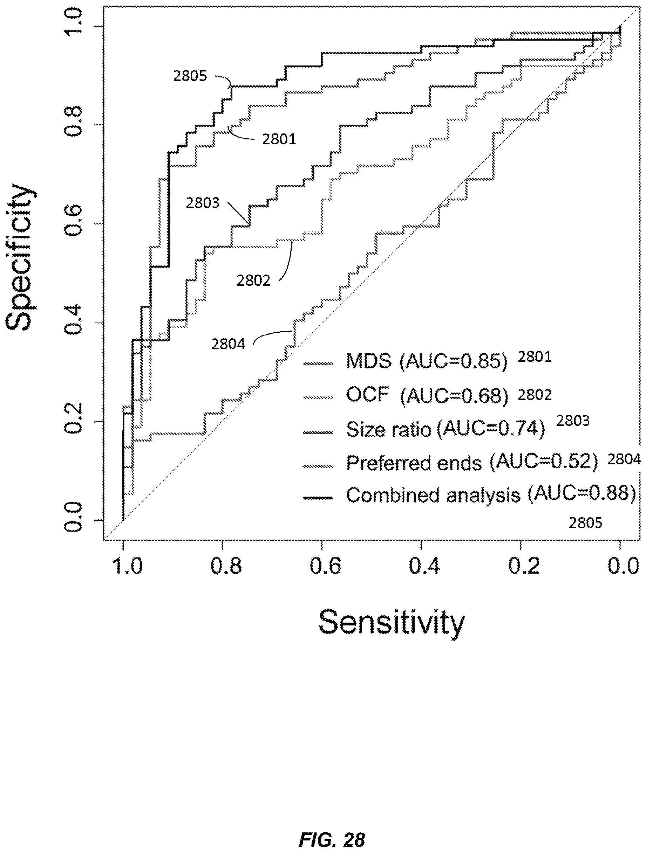

FIG. 28 shows a receiver operating curve for various techniques of discriminating healthy controls from cancer according to embodiments of the present disclosure. We had a total of 129 samples, including healthy controls (n = 38), HBV carriers (n = 17), patients with hepatocellular carcinoma (n = 34), colorectal cancer (n = 10), lung cancer (n = 10), nasopharyngeal carcinoma (n = 10), and head and neck squamous cell carcinoma (n = 10). Interestingly, the MDS based method 2801 (AUC =0.85) appeared to have the best performance, compared with other fragmentomic metrics including fragment size 2803 (AUC = 0.74, p-value = 0.0040; DeLong test) (Yu et al. 2017b), fragment preferred ends 2804 (AUC = 0.52, p-value < 0.0001) (Jiang et al. 2018) and orientation-aware plasma cell-free fragmentation signals, OCF, 2802 (AUC = 0.68, p-value = 0.0013) (Sun et al. 2019). The combined analysis 2805 identified a subject as having cancer if any one of the techniques classified the subject as having cancer.

The accuracy of MDS analysis to discriminate between cancer and non-cancer is maintained relative well for different lengths of motifs. An analysis was performed using MDS for 1-mers to 5-mers.

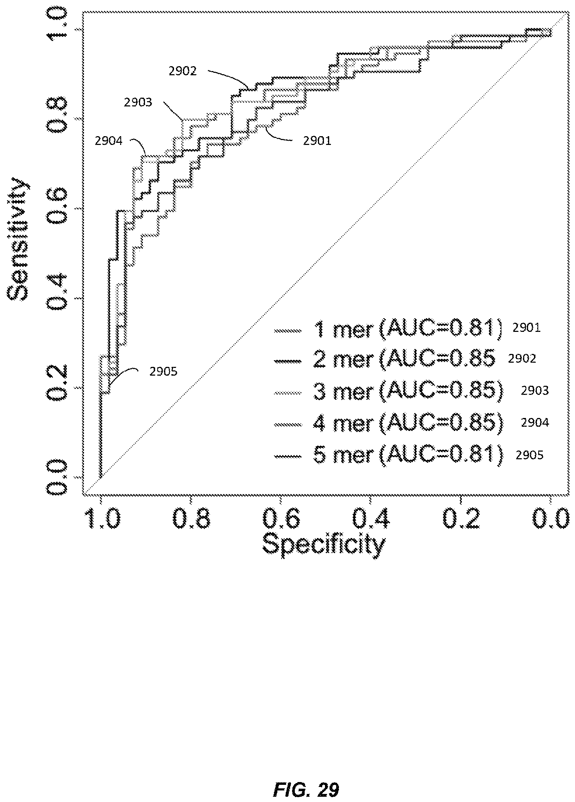

FIG. 29 shows a receiver operating curve for an MDS analysis using various k-mers according to embodiments of the present disclosure. The MDS values deduced from 1 to 5-mer motifs also bore the power of distinguishing patients with and without cancer. The 1-mer analysis 2901 provides 0.81 AUC. The 2-mer analysis 2902 provides 0.85 AUC. The 3-mer analysis 2903 provides 0.85 AUC. The 4-mer analysis 2904 provides 0.85 AUC. The 5-mer analysis 2905 provides 0.81 AUC.

We also explored the effect of tumor DNA fraction on the performance of MDS-based cancer detection according to computer simulation.

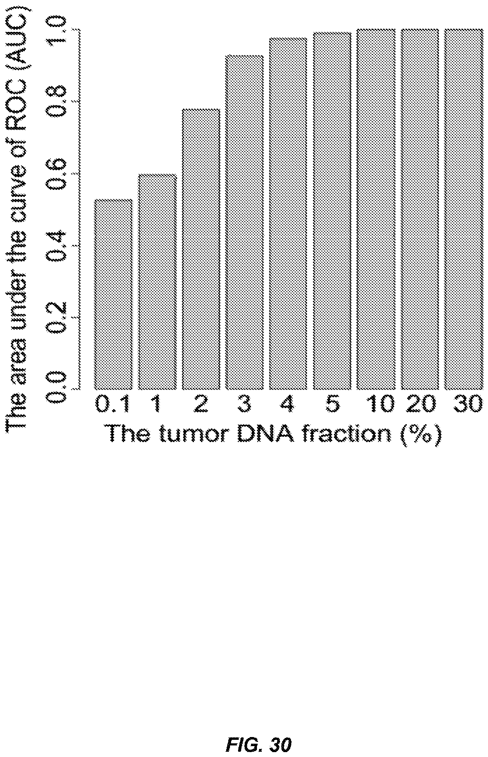

FIG. 30 shows performance of an MDS-based cancer detection for various tumor DNA fractions according to embodiments of the present disclosure. As shown in FIG. 30, the performance of cancer detection progressively improved as the tumor DNA fraction in plasma DNA increased. For example, the area under the curve of ROC (AUC) was only 0.52 for those patients with a tumor DNA fraction of 0.1%, whereas the AUC increased up to 0.9 for those patients with a tumor DNA fraction of 3%, with further increases at higher concentrations, but already nearing a maximum at 5% tumor fraction.

### 3. Machine Learning (SVM, Regression, and Clustering)

To further explore whether a classifier could be built for detecting cancer patients using plasma DNA end motifs, we used the 256 plasma DNA end motifs to build a classifier to differentiate patients with (n=55) cancer and without (n=74) cancer, respectively, using support vector machine (SVM) and logistic regression which took into account the magnitude and direction of each end motif. The SVM analysis identified a hyperplane that best discriminated between cancer and non-cancer patients in a 256 dimension place, where the training data points are the frequencies of each of the 256 motifs of 4-mers. The logistic regression determined coefficients to multiply each of the 256 frequencies, and also determined a cutoff for the resulting output of the logistic function, which can be a weighted sum of the multiplied frequencies or receive as input the weighted sum. Such a logistic function can be a sigmoid function or other activation function, as will be familiar to the skilled person.

To minimize the issue of over-fitting, we adopted the leave-one-out procedure to evaluate its performance by using receiver operating characteristic (ROC) curve analysis. The leave-one-out procedure was performed according to the following steps. Among a sample size of N, we left one sample out as a testing sample, and used the remaining samples (N - 1) to train the classifier based on SVM and logistic regression using the 256 plasma DNA end motifs. Then, we used the trained classifier to determine whether the left-out sample was classified as taken from a subject with or without cancer. We systematically left one sample out as a testing sample to test the classifier trained from the remaining samples. Therefore, we could obtain a predicted result for each sample and the accuracy was calculated from the predicted results.

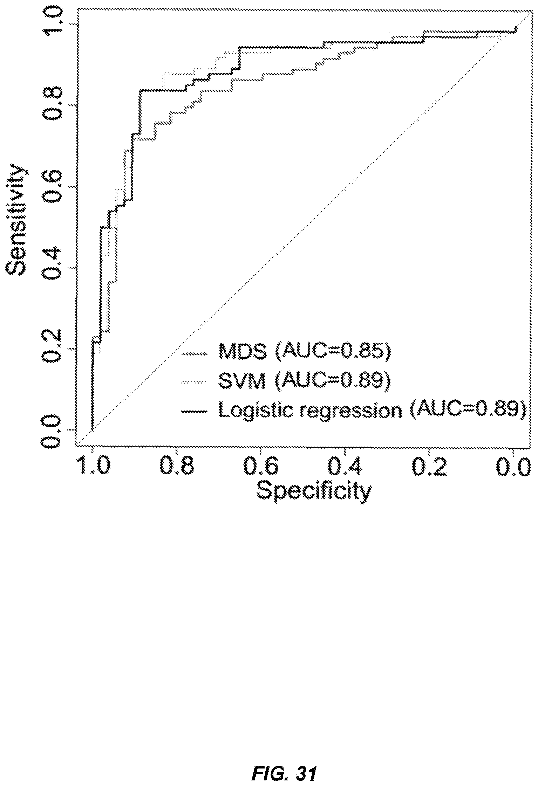

FIG. 31 shows a receiver operating curve for MDS, SVM, and logistic regression analyses according to embodiments of the present disclosure. We observed a small increase in AUC of using the classifiers with 256 end motifs (AUC = 0.89 for both SVM and logistic regression) compared with the MDS based analysis (AUC = 0.85).

As another machine learning technique, we used clustering based on a frequency of end motifs.

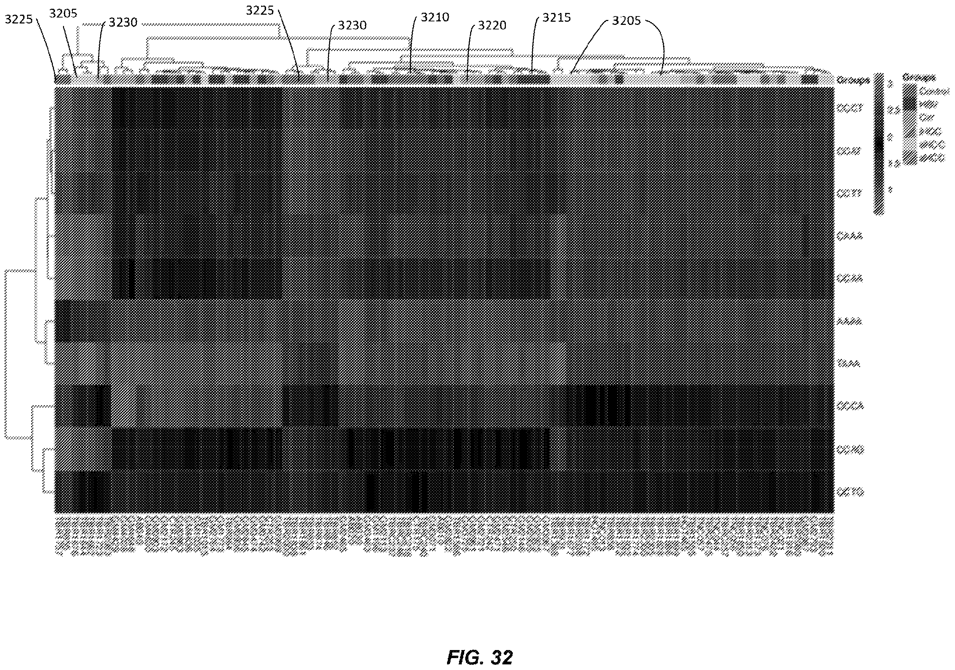

FIG. 32 shows a hierarchical clustering analysis for top 10 ranked end motifs across different groups having different levels of cancer according to embodiments of the present disclosure. As shown, the HCC subjects (eHCC: early-stage HCC 3205 ; iHCC: immediate-stage HCC 3230 ; and aHCC: advanced-stage HCC 3225) are generally clustered together, and the non-HCC (healthy control subjects; HBV: chronic hepatitis B carriers) are generally clustered together. For example, a cluster on the right is the early HCC 3205 (yellow). To the center left is mostly control 3210, HBV 3215, and cirrhosis 3220. The distinct clustering patterns between HCC and non-HCC groups suggested that the end motifs would reflect the disease-associated preference in plasma DNA end motifs and suggested the potential diagnosis power for plasma DNA end motifs. Other clustering techniques could be used besides the connectivity-based hierarchical clustering as the statistical method, such as centroid-based clustering, distribution-based clustering, and density-based clustering.

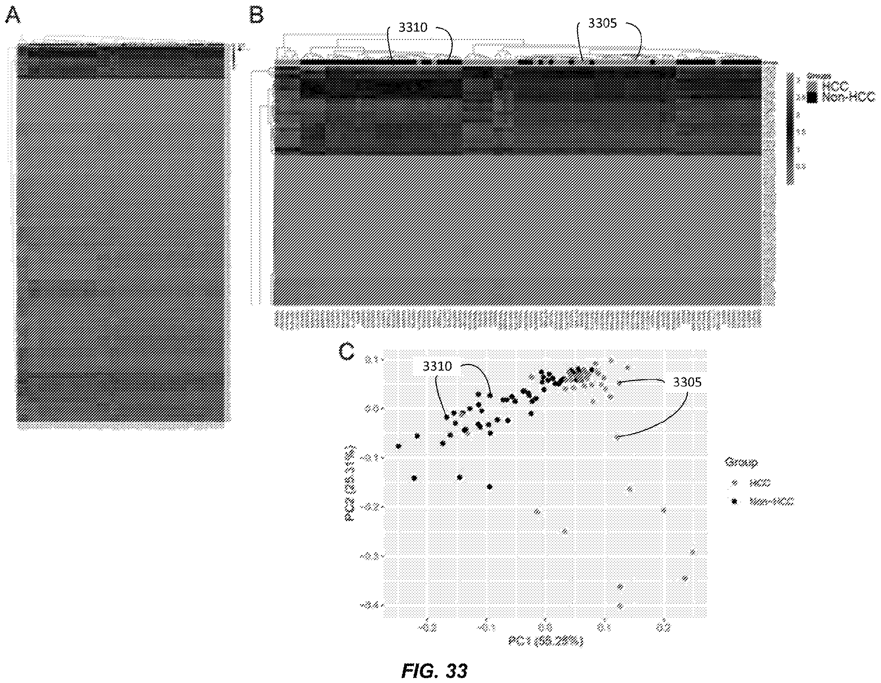

FIGS. 33A-33C shows a hierarchical clustering analysis using all plasma DNA molecules across different groups having different levels of cancer according to embodiments of the present disclosure. FIG. 33A shows a hierarchical clustering analysis based on 256 4-mer end motif frequencies. FIG. 33B shows a zoomed-in visualization for hierarchical clustering analysis based on 256 4-mer end motif frequencies. Each row represents one type of end motif. Each column represents an individual plasma DNA sample. The gradient colors indicate the frequencies of end motifs. Red one represents the highest frequency and green one represents the lowest frequency. FIG. 33C shows a principal component analysis (PCA) for HCC and non-HCC subjects using end motifs. The principal components are a linear combination of the 256 motifs that provide the greatest variance, e.g., in a resulting weighted sum of the frequencies.

Since HCC and non-HCC subjects appeared to form two distinct clusters, the end motifs derived from all plasma DNA molecules would be important metrics to differentiate HCC from non-HCC subjects. FIGS. 33A and 33B show that the HCC subjects 3305 (red) tended to be clustered into one group and non-HCC subjects 3310 (blue) tended to be clustered into another group. In FIG. 33C, the PCA analysis also showed that HCC and non-HCC subjects tended to be clustered into two different groups. PC1 and PC2 correspond to different linear combinations (e.g., weighted averages) of the relative frequencies, which can represent patterns of a given histogram of relative frequencies. FIG. 33C shows that linear combinations (or other transformations) can be performed before performing a clustering or using cutoff values or cutoff planes. Thus, transformed relative frequencies may be used to determine the aggregate value.

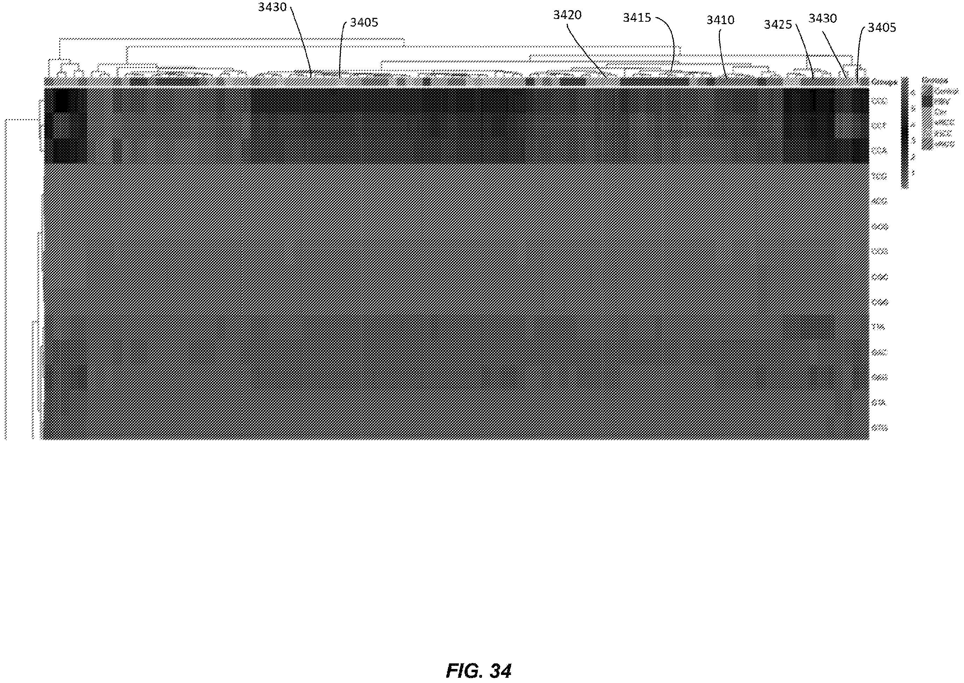

FIG. 34 shows a hierarchical clustering analysis based on 3-mer motifs using all plasma DNA molecules across different groups having different levels of cancer according to embodiments of the present disclosure. For ease of illustration, only a top portion of the heatmap is shown. As shown, the HCC subjects (eHCC: early-stage HCC 3405 ; iHCC: immediate-stage HCC 3430; and aHCC: advanced-stage HCC 3425) are generally clustered together, and the non-HCC (healthy control subjects 3410; HBV 3415: chronic hepatitis B carriers; and cirrhosis 3420) are generally clustered together.

On the basis of these findings, the machine learning (e.g., deep learning) models could be used for training the cancer classifier by making use of 256-dimensional vector comprising the plasma DNA end motifs, including but not limited to support vector machines (SVM), decision tree, naive Bayes classification, logistic regression, clustering algorithm, PCA, singular value decomposition (SVD), t-distributed stochastic neighbor embedding (tSNE), artificial neural network, as well as ensemble methods which construct a set of classifiers and then classify new data points by taking a weighted vote of their predictions. Once the cancer classifier is trained based on "256-dimensional vector based matrix" including a series of cancer patients and non-cancer patients, the probability of being cancer for a new patient would be able to be predicted.

In such uses of machine learning algorithms, the aggregate value can correspond to a probability or a distance (e.g., when using SVMs) that can be compared to a reference value. In other embodiments, the aggregate value can correspond to an output earlier in the model (e.g., an earlier layer in a neural network) that is compared to a cutoff between two classifications or compared to a representative value of a given classification.

*B. Immune disease monitoring*

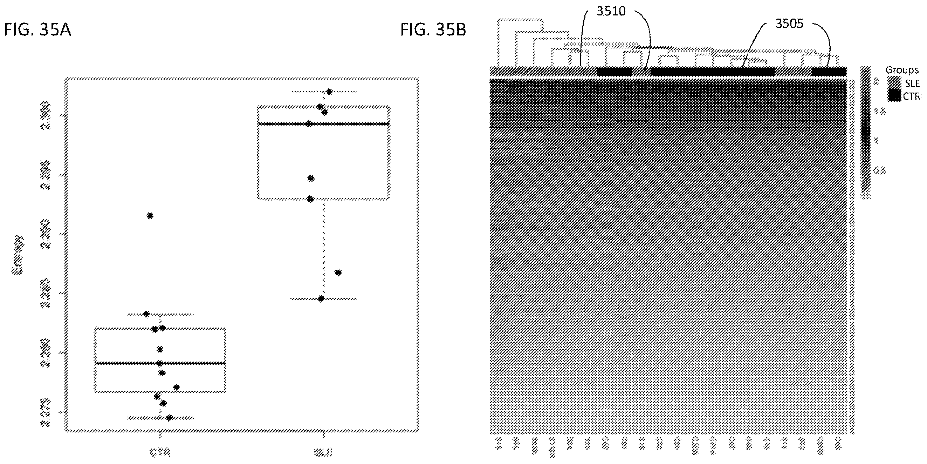

FIG 35A shows an entropy analysis using all plasma DNA molecules between healthy control subjects and SLE patients according to embodiments of the present disclosure. FIG 35B shows a hierarchical clustering analysis using all plasma DNA molecules between healthy control subjects and SLE patients according to embodiments of the present disclosure.

The global landscape aberration analysis for plasma DNA end motifs, including entropy (FIG. 35A, p-value: 0.00014) and clustering analysis (FIG. 35B) illustrated that the SLE patients could be distinguished from healthy control subjects. For example, the entropy increases for subjects with SLE (FIG. 35A). And, two clusters are generally formed on the left (SLE 3510) and the right (control/normal 3505). Thus, the autoimmune disease alters the plasma DNA fragmentation patterns, thereby showing a discriminative power of plasma DNA end motifs between SLE and control subjects.

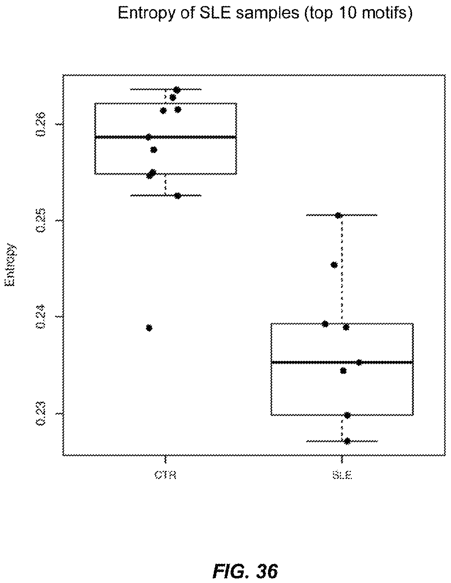

FIG. 36 shows an entropy analysis using plasma DNA molecules having 10 selected end motifs between healthy control subjects and SLE patients according to embodiments of the present disclosure. The motifs having the top 10 highest relative frequencies for a control subject were used. As with other phenotypes, the set of motifs can impact whether the SLE entropy is higher or lower. Given that the 10 motifs were selected as having the highest values for the control, the entropy is higher since the values are similar to each other (i.e., due to the ranking). And, the SLE entropy is lower as there is more variation, e.g., since they are not ranked for an SLE subject. An opposite relationship can exist if the top 10 motifs were selected using an SLE sample. Accordingly, a level of an autoimmune disease (e.g., SLE) can be determined using an aggregate value of relative frequencies.

*C. Synergistic analysis for end motifs and conventional metrics*

We tested whether a combined analysis of plasma DNA end motif and other metrics (copy number aberrations (CNA), hypomethylation, and hypermethylation) would improve the performance of noninvasive cancer detection. For example, a decision tree-based classification could be used for combined analysis.

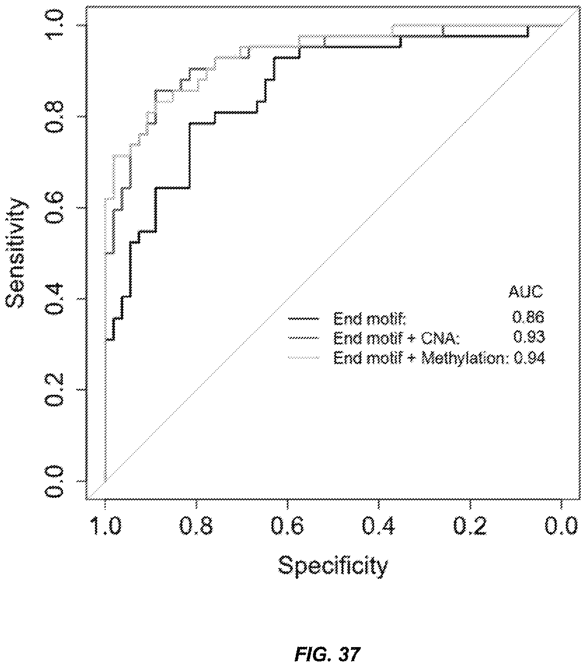

FIG. 37 shows an ROC curve for a combined analysis that includes end motifs and copy number or methylation for HCC and non-HCC subjects according to embodiments of the present disclosure. The end motif analysis uses a motif diversity score determined using all 356 motifs of 4-mers. The combined analysis identify cancer if either analysis resulted in a classification of cancer. The combined analysis of end motif and methylation analysis (AUC: 0.94) or the combined analysis of end motif and CNA analysis (AUC: 0.93) was superior to the analysis only using end motif (AUC: 0.86). The methylation analysis used the number of hypomethylated (defined as methylation density z-score < -3) 1-Mb bins being above that of normal controls, with a cutoff number of aberrant bins discriminating between cancer and non-cancer. The CNA analysis used the number of 1-Mb bins having representation of a z-score that is more than 3 or less than -3, with a cutoff number of aberrant bins discriminating between cancer and non-cancer. Further details for the methylation analysis can be found in U.S. Patent publication 2014/0080715 and for the CNA analysis can be found in U.S. Patent publication U.S. 2013/0040824.

An example decision-tree based classification is described. For example, we can use random forest algorithm to deduce the cutoffs for each metric, including CNA, hypomethylation, hypermethylation, size (e.g., as described in U.S. Patent Publication 2013/0237431), end motifs, and fragmentation patterns (e.g., as described in U.S. Patent Publications 2017/0024513 and 2019/0341127 and U.S. Patent Application 16/519,912). Each metric would have a particular cutoff. Taking one metric (hypomethylation) as example, one case can be classified as cancer or non-cancer depending on whether the metric is below or above the cutoff. One metric represents one node in the decision tree. After a sample travels all nodes in the whole tree, for example, the majority of votes (e.g. the number of nodes indicating cancer is greater than that indicating non-cancer) can provide the final classification.

### D. Example of an alternative way to define the end motif of plasma DNA

To demonstrate the feasibility of using the alternative way to define end motif of plasma DNA, technique 160 in FIG. 1 was adopted to analyse the HCC and non-HCC subjects, which including sequenced 20 healthy control subjects (Control), 22 chronic hepatitis B carriers (HBV), 12 cirrhosis subjects (Cirr), 24 early-stage HCC (eHCC), 11 immediate-stage HCC (iHCC), and 7 advanced-stage HCC (aHCC).

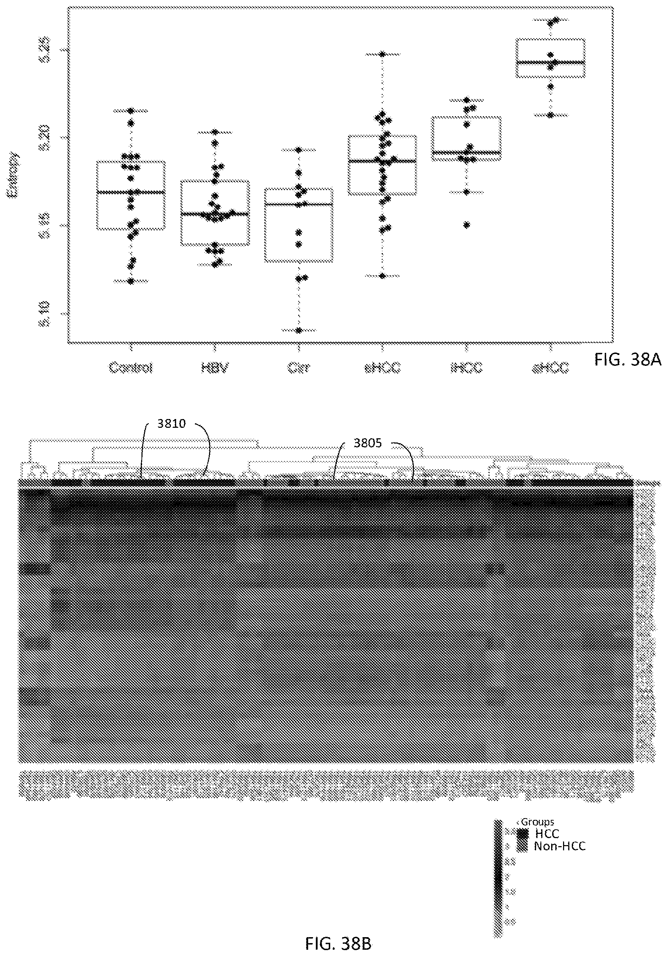

FIG. 38A shows an entropy analysis based on the 4-mer jointly constructed from the ends of sequenced plasma DNA fragments and their adjacent genomic sequences in HCC and non-HCC subjects according to embodiments of the present disclosure. The entropy was determined using all 256 end motifs. As with the analysis that defined a motif using technique 140 of FIG. 1, the entropy of the HCC subjects difference from the non-cancer subjects. And, the advanced HCC shows a substantial difference from the eHCC and iHCC. FIG. 38B shows a clustering analysis based on the 4-mer jointly constructed from the ends of sequenced plasma DNA fragments and their adjacent genomic sequences in HCC subjects 3810 and non-HCC subjects 3805 according to embodiments of the present disclosure.

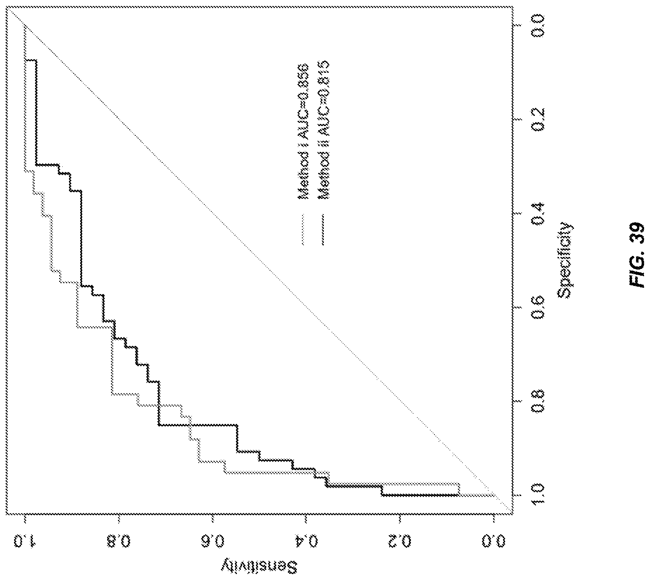

FIG. 39 shows an ROC comparison for techniques 140 and 160 of FIG. 1 used to define the end motif of plasma DNA according to embodiments of the present disclosure. The same subjects as FIG. 38A were used, and an entropy analysis using 4-mers was performed to make the classification. Method (i) corresponds to technique 140, and method (ii) corresponds to technique 160. Compared with technique 140 in FIG. 1, a slightly inferior performance (AUC: 0.815 versus 0.856) was observed with the use of technique 160 in FIG. 1.

### E. Filtering for improved discrimination

Certain criteria can be used to filter specific DNA fragments (besides by end motifs) to provide greater accuracy, e.g., sensitivity and specificity. As examples, the end motif analysis can be restricted to DNA fragments that originate from open chromatin regions of a particular tissue, e.g., as determined by reads aligning entirely within or partially to one of a plurality of open chromatin regions. For example, any read with at least one nucleotide overlapping with an open chromatin region can be defined as a read within an open chromatin region. The typical open chromatin region is about 300 bp according to DNase I hypersensitive site. The size of an open chromatin region can variable, depending on the technique used to define the open chromatin regions, for example, ATAC-seq (Assay for Transposase Accessible Chromatin sequencing) vs. DNaseI-Seq.

As another example, DNA fragments of a particular size can be selected for performing the end motif analysis. As shown below, this can increase the separation of an aggregate value of relative frequencies of end motifs, thereby increasing accuracy.

A further example can use methylation properties of the DNA fragments. Fetal and tumor DNA are generally hypomethylated. Embodiments can determine a methylation metric (e.g., density) of a DNA fragment (e.g., as a proportion or absolute number of site(s) that are methylated on a DNA fragment). And, DNA fragments can be selected for use in the end motif analysis based on the measured methylation densities. For example, a DNA fragment can be used only if the methylation density is above a threshold.

Whether a DNA fragment includes a sequence variation (e.g. base substitution, insertion or deletion) relative to a reference genome can also be used for filtering.

The various filtering criteria can be used in combination together. For example, each criterion may need to be satisfied, or at least a specific number of criteria may need to be satisfied. In another implementation, a probability that a fragment corresponds to clinically-relevant DNA (e.g., fetal, tumor, or transplant) can be determined, and a threshold imposed for the probability, for which a DNA fragment is to satisfy before being used in an end motif analysis. As a further example, a contribution of a DNA fragment to a frequency counter of a particular end motif can be weighted based on the probability (e.g., adding the probability that has a value less than one, instead of adding one). Thus, DNA fragments with particular end motifs would be weighted higher and/or have a higher probability. Such enrichment is described further below.

### 1. End motifs across tissue-specific chromatin regions

Since the different tissues would have preferred fragmentation patterns during apoptosis (Chan et al, Proc Natl Acad Sci USA. 2016;113:E8159-8168; Jiang et al, Proc Natl Acad Sci USA. 2018; doi:10.1073/pnas.1814616115), we further reasoned that the selection of a certain genomic regions for plasma DNA end motif analysis would further improve the discriminative power in classifying the diseased patients and control subjects. Taking the detection of HCC patients as an example, open chromatin regions for blood and liver were used.

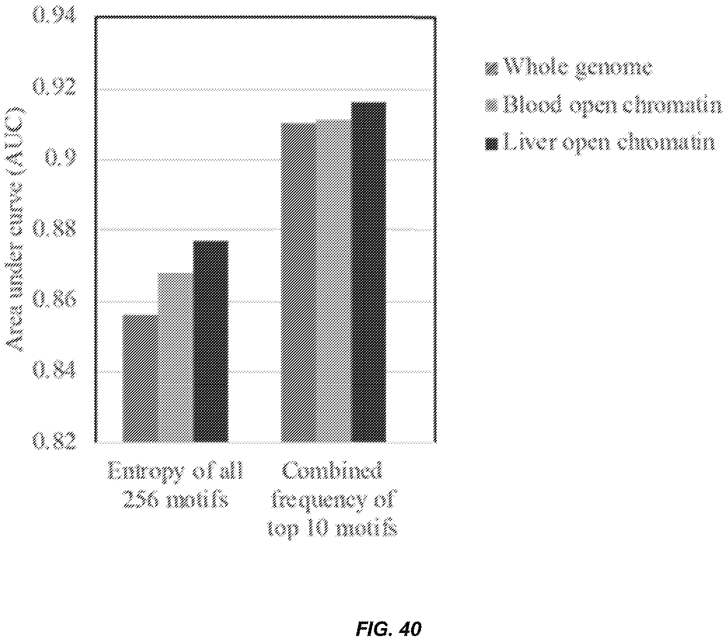

FIG. 40 shows a comparison of accuracies that shows tissue-specific open chromatin regions improves the discriminative power of plasma DNA end motif for HCC and non-cancer patients according to embodiments of the present disclosure. The analysis was performed for the entropy of all 256 motifs using 4-mers and the combined frequency of the top 10 motifs. For the liver open chromatin results, a sequence read was kept (i.e., not filtered out) if the read had at least one nucleotide overlapping with one of the liver open chromatin regions.

The power of end motifs originating from the plasma DNA molecules overlapping with liver open chromatin regions gives rise to the best performance with an AUC of 0.918 with the use of combined frequencies of top 10 ranked motifs. In contrast, the discriminating power of end motifs originating from the plasma DNA molecules for all 256 motifs without any selection was the least AUC of 0.855.

Accordingly, if a particular tissue is being screened for cancer, DNA fragments from an open chromatin of that particular tissue (or at least where ending sequence is in an open chromatin region) can be used to perform the analysis, whereas DNA fragments not in these identified regions are not used. Liver was used here, as the cancer was HCC. The location of the DNA fragments can be determined by aligning the sequence reads to a reference genome, where the open chromatin regions can be identified from literature or databases.

### 2. Size-band based end motif analysis

The frequencies of a certain of end motifs were shown to vary according to the size ranges (size bands) being analyzed, for example, the percentage of CCCA shows this behavior. This implies a size-band based end motif analysis can influence the performance in using plasma DNA end motifs to distinguish cancer patients from non-cancer subjects. To illustrate this possibility, we test a series of size ranges, including but not limited to 50-80 bp, 81-110 bp, 111-140 bp, 141-170 bp, 171-200 bp, 201-230 bp, to investigate how the size band being analyzed would affect the overall diagnostic performance.

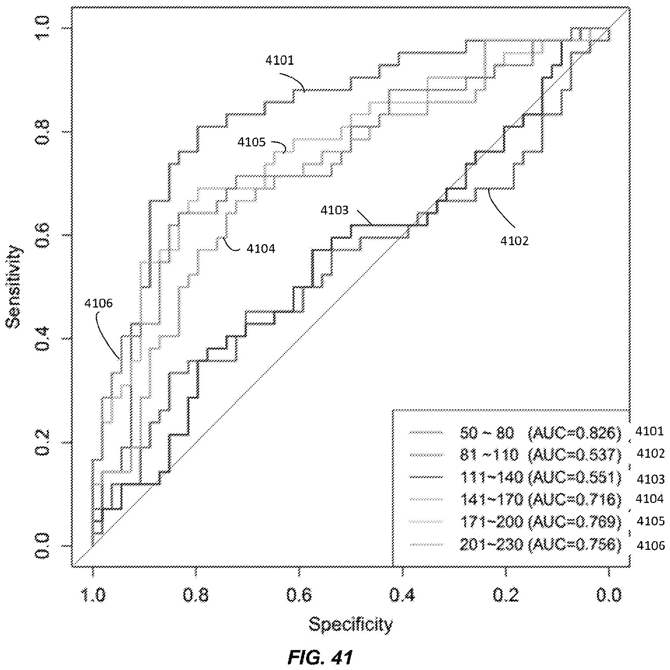

FIG. 41 shows a size-band based plasma DNA end motif analysis according to embodiments of the present disclosure. The classification used motif diversity score (entropy) is determined using 256 motifs for 4-mers. Various ranges are listed in FIG. 41, but other ranges may be used. The 50-80 analysis 4101 provides 0.826 AUC. The 81-110 analysis 4102 provides 0.537 AUC. The 111-140 analysis 4103 provides 0.551 AUC. The 141-170 analysis 4104 provides 0.716 AUC. The 171-200 analysis 4105 provides 0.769 AUC. The 201-230 analysis 4106 provides 0.756 AUC.

Such size ranges may be used for techniques that enrich clinically-relevant DNA. For example, selecting DNA molecules that are 50-80 bases would enrich the a sample for tumor DNA. Multiple disjoint size ranges could be used, as opposed to a single size range. Such enrichment can be a reason that a better AUC occurs for a size range of 50-80 bases vs. 81-110 bases.

The end motifs derived from plasma DNA molecules within the range of 50 to 80 bp appeared to give the best discriminative power of detecting HCC from non-HCC subjects (AUC: 0.83). Accordingly, embodiments can filter DNA fragments to select ones in a particular size range, and then use the selected DNA fragments (reads) to determine the relative frequencies and later operations. As examples, the size filter can be done via physical separation or by determining size using the sequence reads (e.g., length if entire fragment is sequenced or by aligning the paired-ends to a reference). Examples of physical enrichment for short DNA include band cutting upon gel electrophoresis, by collecting eluate at certain retention time upon capillary electrophoresis, after liquid chromatography, or by microfluidics.

### F. Classifying a level of Pathology

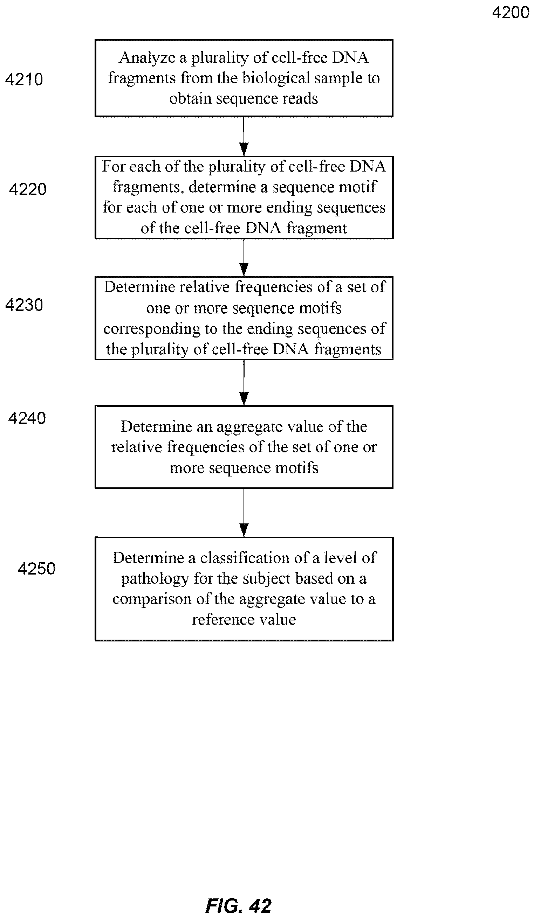

FIG. 42 is a flowchart illustrating a method 4200 of classifying a level of pathology in a biological sample of a subject according to embodiments of the present disclosure. The biological sample including cell-free DNA. Aspects of method 4200 may be performed in a similar manner as method 1900 of FIG. 19 and method 2000 of FIG. 20.

At block 4210, a plurality of cell-free DNA fragments from the biological sample is analyzed to obtain sequence reads. The sequence reads include ending sequences corresponding to ends of the plurality of cell-free DNA fragments. Block 4210 may be performed in a similar manner as block 1910 of FIG. 19.

At block 4220, for each of the plurality of cell-free DNA fragments, a sequence motif is determined for each of one or more ending sequences of the cell-free DNA fragment. Block 4220 may be performed in a similar manner as block 1920 of FIG. 19.

At block 4230, relative frequencies of a set of one or more sequence motifs corresponding to the ending sequences of the plurality of cell-free DNA fragments are determined. A relative frequency of a sequence motif can provide a proportion of the plurality of cell-free DNA fragments that have an ending sequence corresponding to the sequence motif. Block 4230 may be performed in a similar manner as block 1930 of FIG. 19. For example, the set of one or more sequence motifs can include N base positions. The set of one or more sequence motifs can include all combinations of N bases. N can be an integer equal to or greater than three, as well as any other integer.

As another example, the set of one or more sequence motifs can be a top M sequence motifs with a largest difference between two types of DNA as determined in one or more reference samples, e.g., the motifs that all show a largest positive difference (e.g., top 10 or other number) or all who a largest negative difference. M can be an integer equal to or greater than one. For methods 1900 and 2000, the two types of DNA can be the clinically-relevant DNA and the other DNA. For method 4200, the two types of DNA can be from two references samples having different classifications for the level of pathology. As a further example, the set of one or more sequence motifs can be a top M most frequent sequence motifs occurring in one or more reference samples, e.g., as shown in FIG. 22, where the reference sample is a non-cancer sample, such as an HBV sample.

At block 4240, an aggregate value of the relative frequencies of the set of one or more sequence motifs is determined. Block 4240 may be performed in a similar manner as block 1940 of FIG. 19. Examples of an aggregate value are described throughout this disclosure and include entropy, combined frequencies, a difference (e.g., a distance) from a reference pattern of relative frequencies as may be implemented in clustering or using SVMs or a value (e.g., a probability) determined from the difference, or a output in a machine learning model (e.g., an intermediate or final layer in a neural network) that is compared to a cutoff between two classifications or compared to a representative value of a given classification.

When the set of one or more sequence motifs includes a plurality of sequence motifs, the aggregate value may include a sum of the relative frequencies of the set. The sum can be a weighted sum. For example, the aggregate value can include an entropy term, which includes a sum of terms comprising the weighted sum. Each term can include a relative frequency multiplied by a logarithm of the relative frequency. The aggregate value can correspond to a variance in the relative frequencies

In another example, the aggregate value includes a final or intermediate output of a machine learning model. In various implementations, the machine learning model uses clustering, support vector machines, or logistic regression.

At block 4250, a classification of a level of pathology can be determined for the subject based on a comparison of the aggregate value to a reference value. As examples, the pathology can be a cancer or an auto-immune disorder. As examples, the levels can be no cancer, early stage, intermediate stage, or advanced stage. The classification can then select one of the levels. Accordingly, the classification can be determined from a plurality of levels of cancer that include a plurality of stages of cancer. As examples, the cancer can be hepatocellular carcinoma, lung cancer, breast cancer, gastric cancer, glioblastoma multiforme, pancreatic cancer, colorectal cancer, nasopharyngeal carcinoma, and head and neck squamous cell carcinoma. As an example, the auto-immune disorder can be systemic lupus erythematosus.

In further examples, the level of pathology corresponds to a fractional concentration of clinically-relevant DNA associated with the pathology. For instance, the level of pathology can be cancer and the clinically-relevant DNA can be tumor DNA. The reference value can be a calibration value determined from a calibration sample, as described for method 1900.

In some embodiments, the cell-free DNA are filtered to identify the plurality of cell-free DNA fragments. Examples of filtering are provided in the section above. For example, the filtering can be based on a methylation (density or whether a particular site is methylated), size, or a region from which a DNA fragment is derived. The cell-free DNA can be filtered for DNA fragments from open chromatin regions of a particular tissue.

## IV. ENRICHMENT

The preference of DNA fragments from particular tissue to exhibit a particular set of end motifs can be used to enrich a sample for DNA from that particular tissue. Accordingly, embodiments can enrich a sample for clinically-relevant DNA. For example, only DNA fragments having a particular ending sequence may be sequenced, amplified, and/or captured using an assay. As another example, filtering of sequence reads can be performed, e.g., in a similar manner as described in section III.E.

### A. Physical enrichment

Physical enrichment may be performed in various ways, e.g., via targeted sequencing or PCR, as may be performed using particular primers or adapters. If a particular end motif of an ending sequence is detected, then an adaptor can be added to the end of the fragment. Then, when sequencing is performed, only DNA fragments with the adapter will be sequenced (or at least predominantly sequenced), thereby providing targeted sequencing.

As another example, primers that hybridize to the particular set of end motifs can be used. Then, sequencing or amplification can be performed using these primers. Capture probes corresponding to the particular end motifs can also be used to capture DNA molecules with those end motifs for further analysis. Some embodiments can ligate a short oligonucleotide to the end of a plasma DNA molecule. Then, a probe can be designed such that it would only recognize a sequence that is partially the end motif and partially the ligated oligonucleotide

Some embodiments can use CRISPR-based diagnostic technology, e.g. using a guide RNA to localise a site corresponding to a preferred end motif for the clinically-relevant DNA and then a nuclease to cut the DNA fragment, as may be done using Cas-9 or Cas-12. For example, an adapter can be used to recognize the end motif, and then CRISPR/Cas9 or Cas-12 can be used to cut the end motif/adaptor hybrid and create a universal recognisable end for further enrichment of the molecules with the desired ends.

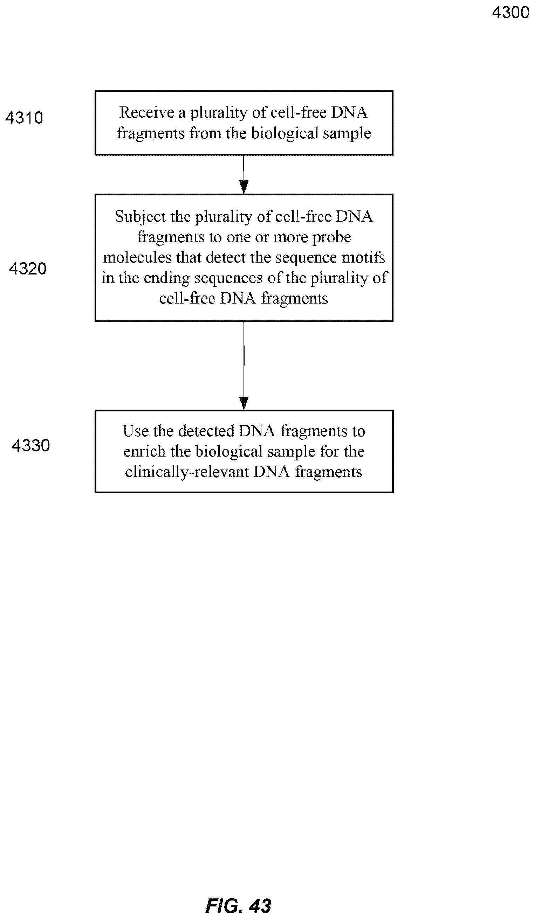

FIG. 43 is a flowchart illustrating a method 4300 of enriching a biological sample for clinically-relevant DNA according to embodiments of the present disclosure. The biological sample includes the clinically-relevant DNA molecules and other DNA molecules that are cell-free. Method 4300 can use particular assays to perform the enrichment.

At block 4310, a plurality of cell-free DNA fragments from the biological sample is received. The clinically-relevant DNA fragments (e.g., fetal or tumor) have ending sequences that include sequence motifs that occur at a relative frequency greater than the other DNA (e.g., maternal DNA, healthy DNA, or blood cells). As examples, data from FIGS. 3 and 13 can be used). Thus, the sequence motifs can be used to enrich for the clinically-relevant DNA.

At block 4320, the plurality of cell-free DNA fragments is subjected to one or more probe molecules that detect the sequence motifs in the ending sequences of the plurality of cell-free DNA fragments. Such use of probe molecules can result in obtaining detected DNA fragments. In one example, the one or more probe molecules can include one or more enzymes that interrogate the plurality of cell-free DNA fragments and that append a new sequence that is used to amplify the detected DNA fragments. In another example, the one or more probe molecules can be attached to a surface for detecting the sequence motifs in the ending sequences by hybridization.

At block 4330, the detected DNA fragments are used to enrich the biological sample for the clinically-relevant DNA fragments. As an example, using the detected DNA fragments to enrich the biological sample for the clinically-relevant DNA fragments can includes amplifying the detected DNA fragments. As another example, the detected DNA fragments can be captured, and non-detected DNA fragments can be discarded.

*B. In Silico enrichment*

The in silico enrichment can use various criteria to select or discard certain DNA fragments. Such criteria can include end motifs, open chromatin regions, size, sequence variation, methylation and other epigenetic characteristics. Epigenetic characteristics include all modifications of the genome that do not involve a change in DNA sequence. The criteria can specify cutoffs, e.g., requiring certain properties, such as a particular size range, methylation metric above or below a certain amount, combination of methylation status of more than one CpG sites (e.g., a methylation haplotype (Guo et al, Nat Genet. 2017; 49: 635-42)), etc., or having a combined probability above a threshold. Such enrichment can also involve weighting DNA fragments based on such a probability.

As examples, the enriched sample can be used to classify a pathology (as described above), as well as to identify tumor or fetal mutations or for tag-counting for amplification/deletion detection of a chromosome or chromosomal region. For instance, if a particular end motif or a set of end motifs are associated with liver cancer (i.e., a higher relative frequency than for non-cancer or other cancers), then embodiments for performing cancer screening can weight such DNA fragments higher than DNA fragments not having this preferred one or this preferred set of end motifs.

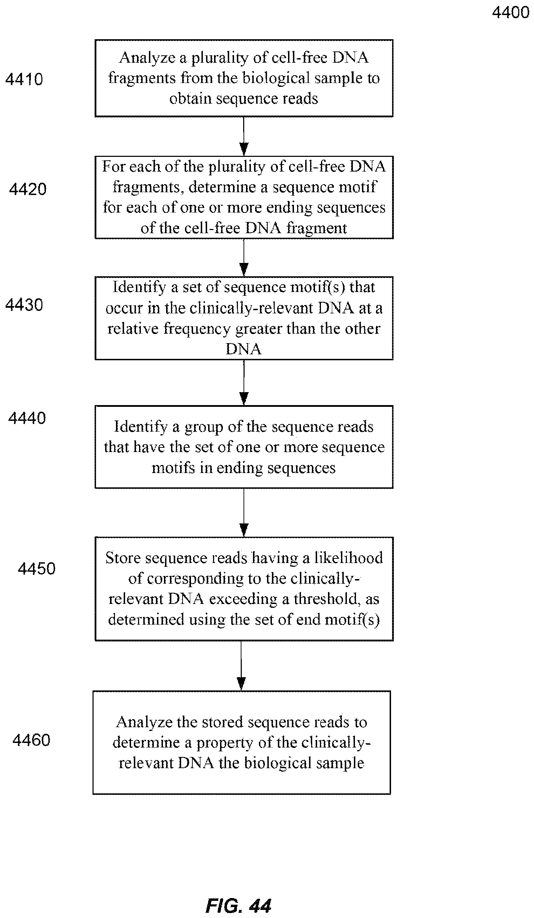

FIG. 44 is a flowchart illustrating a method 4400 of enriching a biological sample for clinically-relevant DNA according to embodiments of the present disclosure. The biological sample includes the clinically-relevant DNA molecules and other DNA molecules that are cell-free. Method 4400 can use particular criteria of sequence reads to perform the enrichment.

At block 4410, a plurality of cell-free DNA fragments from the biological sample is analyzed to obtain sequence reads. The sequence reads include ending sequences corresponding to ends of the plurality of cell-free DNA fragments. Block 4410 may be performed in a similar manner as block 1910 of FIG. 19.

At block 4420, for each of the plurality of cell-free DNA fragments, a sequence motif is determined for each of one or more ending sequences of the cell-free DNA fragment. Block 4420 may be performed in a similar manner as block 1920 of FIG. 19.

At block 4430, a set of one or more sequence motifs that occur in the clinically-relevant DNA at a relative frequency greater than the other DNA is identified. The set of sequence motif(s) can be identified by genotypic or phenotypic techniques described herein. Calibration or references samples may be used to rank and select sequence motifs that are selective for the clinically-relevant DNA.

At block 4440, a group of the sequence reads that have the set of one or more sequence motifs in ending sequences is identified. This can be viewed as a first stage of filtering.

At block 4450, sequence reads having a likelihood of corresponding to the clinically-relevant DNA exceeding a threshold can be stored. The likelihood can be determined using the set of end motif(s). For instance, for each sequence read of the group of the sequence reads, a likelihood that the sequence read corresponds to the clinically-relevant DNA can be determined based on an ending sequence of the sequence read including a sequence motif of the set of one or more sequence motifs. The likelihood can be compared to a threshold. As an example, the threshold can be determined empirically. For instance, various thresholds can be tested for samples that a concentration of the clinically-relevant DNA can be measured for a group of sequence reads. An optimal threshold can maximize the concentration while maintaining a certain percentage of the total number of sequence reads. The threshold could be determined by one or more given percentiles (5th, 10th, 90th, or 95th) of the concentrations of one or more end motifs present in the healthy controls or in control groups exposed to similar etiological risk factors but without diseases. The threshold could be a regression or probabilistic score.

The sequence read can be stored in memory (e.g., in a file, table, or other data structure) when the likelihood exceeds the threshold, thereby obtaining stored sequence reads. Sequence reads having a likelihood below the threshold can be discarded or not stored in the memory location of the reads that are kept, or a field of a database can include a flag indicating the read had a lower threshold so that later analysis can exclude such reads. As examples, the likelihood can be determined using various techniques, such as odds ratio, z-scores, or probability distributions.

At block 4460, the stored sequence reads can be analyzed to determine a property of the clinically-relevant DNA the biological sample, e.g., as described herein, such as described in other flowcharts. Methods 1900, 2000, and 4200 are such examples. For instance, the property of the clinically-relevant DNA the biological sample can be a fractional concentration of the clinically-relevant DNA. As another example, the property can be a level of pathology of a subject from whom the biological sample was obtained, where the level of pathology is associated with the clinically-relevant DNA. As another example, the property can be a gestational age of a fetus of a pregnant female from whom the biological sample was obtained.

Other criteria can be used to determine the likelihood. Sizes of the plurality of cell-free DNA fragments can be measured using the sequence reads. The likelihood that a particular sequence read corresponds to the clinically-relevant DNA can be further based on a size of the cell-free DNA fragment corresponding to the particular sequence read.

Methylation can also be used. Thus, embodiments can measure one or more methylation statuses at one or more sites of a cell-free DNA fragment corresponding to a particular sequence read. The likelihood that the particular sequence read corresponds to the clinically-relevant DNA can be further based on the one or more methylation statuses. As a further example, whether a read is within an identified set of open chromatin regions can be used as a filter.

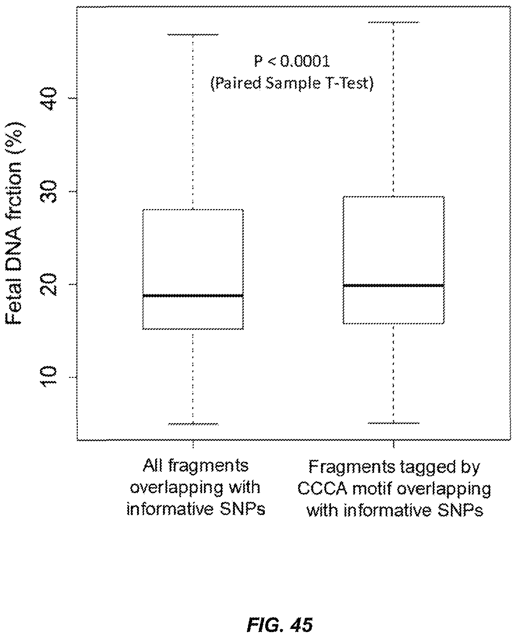

FIG. 45 shows an example plot illustrating an increase in fetal DNA fraction using the CCCA end motif according to embodiments of the present disclosure. The vertical axis is the fetal DNA fraction for the tested samples. The two sets of data are for (1) all fragments overlapping with informative SNPs (i.e., one with a fetal-specific allele) and (2) fragments having a CCCA end motif and overlapping with the informative SNPs. Thus, the data on the left provides the actual fetal DNA fraction in the whole sample, and the data on the right provides the data for an in silico enriched sample. In this example, the likelihood can be determined to be above the threshold when the ending motif is CCCA. More motifs can be used in a similar manner, e.g., as a group indicating the likelihood is above a threshold.

The median relative increase of fetal DNA fraction is 3.2% (IQR: 1.3-6.4%). The relative increase of fetal DNA fraction is defined by (b-a)/a*100, where a is the original fetal DNA fraction calculated by all fragments overlapping with informative SNPs where the mother is homozygous and the fetus is heterozygous, and b is the fetal DNA fraction calculated by the fragments tagged by CCCA motif that is enriched in fetal DNA molecules.

For any of the methods described herein, the sequence motif for each of one or more ending sequences of the cell-free DNA fragment can be performed using a reference genome (e.g., via technique 160 of FIG. 1). Such a technique can include: aligning one or more sequence reads corresponding to the cell-free DNA fragment to a reference genome, identifying one or more bases in the reference genome that are adjacent to the ending sequence, and using the ending sequence and the one or more bases to determine the sequence motif.

## V. EXAMPLE SYSTEMS

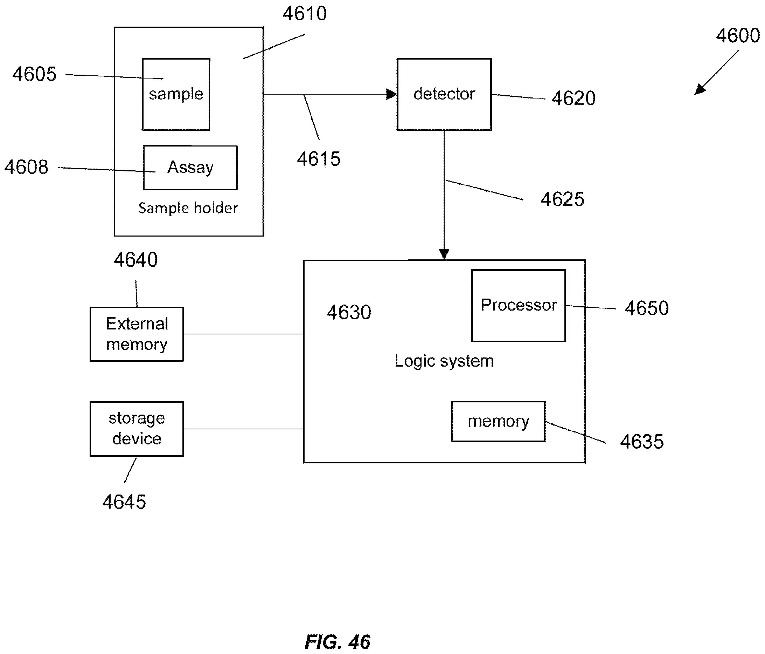

FIG. 46 illustrates a measurement system 4600 according to an embodiment of the present invention. The system as shown includes a sample 4605, such as cell-free DNA molecules within a sample holder 4610, where sample 4605 can be contacted with an assay 4608 to provide a signal of a physical characteristic 4615. An example of a sample holder can be a flow cell that includes probes and/or primers of an assay or a tube through which a droplet moves (with the droplet including the assay). Physical characteristic 4615 (e.g., a fluorescence intensity, a voltage, or a current), from the sample is detected by detector 4620. Detector 4620 can take a measurement at intervals (e.g., periodic intervals) to obtain data points that make up a data signal. In one embodiment, an analog-to-digital converter converts an analog signal from the detector into digital form at a plurality of times. Sample holder 4610 and detector 4620 can form an assay device, e.g., a sequencing device that performs sequencing according to embodiments described herein. A data signal 4625 is sent from detector 4620 to logic system 4630. Data signal 4625 may be stored in a local memory 4635, an external memory 4640, or a storage device 4645.

Logic system 4630 may be, or may include, a computer system, ASIC, microprocessor, etc. It may also include or be coupled with a display (e.g., monitor, LED display, etc.) and a user input device (e.g., mouse, keyboard, buttons, etc.). Logic system 4630 and the other components may be part of a stand-alone or network connected computer system, or they may be directly attached to or incorporated in a device (e.g., a sequencing device) that includes detector 4620 and/or sample holder 4610. Logic system 4630 may also include software that executes in a processor 4650. Logic system 4630 may include a computer readable medium storing instructions for controlling measurement system 4600 to perform any of the methods described herein. For example, logic system 4630 can provide commands to a system that includes sample holder 4610 such that sequencing or other physical operations are performed. Such physical operations can be performed in a particular order, e.g., with reagents being added and removed in a particular order. Such physical operations may be performed by a robotics system, e.g., including a robotic arm, as may be used to obtain a sample and perform an assay.

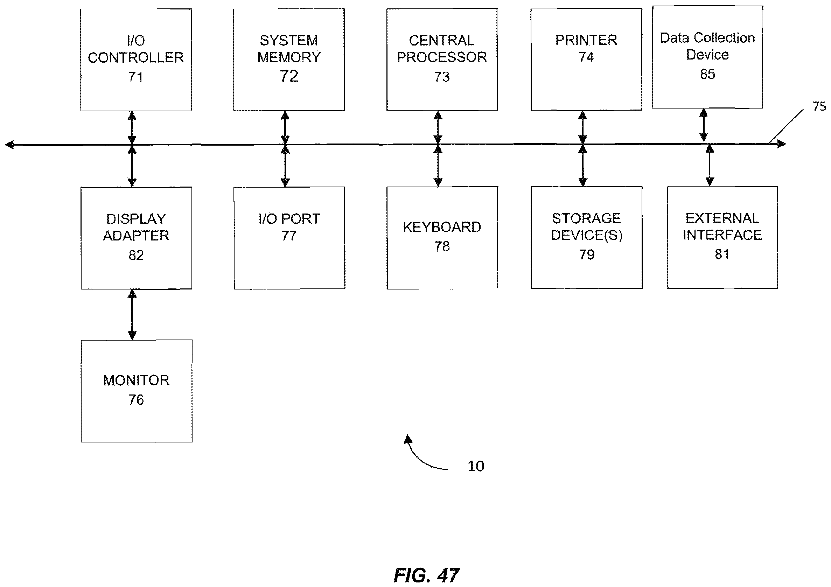

Any of the computer systems mentioned herein may utilize any suitable number of subsystems. Examples of such subsystems are shown in FIG. 47 in computer system 10. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in FIG. 47 are interconnected via a system bus 75. Additional subsystems such as a printer 74, keyboard 78, storage device(s) 79, monitor 76 (e.g., a display screen, such as an LED), which is coupled to display adapter 82, and others are shown. Peripherals and input/output (I/O) devices, which couple to I/O controller 71, can be connected to the computer system by any number of means known in the art such as input/output (I/O) port 77 (e.g., USB, FireWire®). For example, I/O port 77 or external interface 81 (e.g. Ethernet, Wi-Fi, etc.) can be used to connect computer system 10 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software with a generally programmable processor in a modular or integrated manner. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present invention using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a harddrive or a floppy disk, or an optical medium such as a compact disk (CD) or DVD (digital versatile disk) or Blu-ray disk, flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

The specific details of particular embodiments may be combined in any suitable manner without departing from the scope of embodiments of the invention. However, other embodiments of the invention may be directed to specific embodiments relating to each individual aspect, or specific combinations of these individual aspects.

The above description of example embodiments of the present disclosure has been presented for the purposes of illustration and description. It is not intended to be exhaustive or to limit the disclosure to the precise form described, and many modifications and variations are possible in light of the teaching above.

A recitation of "a", "an" or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover, reference to a "first" or a "second" component does not limit the referenced component to a particular location unless expressly stated. The term "based on" is intended to mean "based at least in part on."

**Claims**

1. A method of estimating a fractional concentration of clinically-relevant DNA in a biological sample of a subject, the biological sample including the clinically-relevant DNA and other DNA that are cell-free, the method comprising:

   analyzing a plurality of cell-free DNA fragments from the biological sample to obtain sequence reads, wherein the sequence reads include ending sequences corresponding to ends of the plurality of cell-free DNA fragments;
   for each of the plurality of cell-free DNA fragments, determining a sequence motif for each of one or more ending sequences of the cell-free DNA fragment;
   determining relative frequencies of a set of one or more sequence motifs corresponding to the ending sequences of the plurality of cell-free DNA fragments, wherein a relative frequency of a sequence motif provides a proportion of the plurality of cell-free DNA fragments that have an ending sequence corresponding to the sequence motif;
   determining an aggregate value of the relative frequencies of the set of one or more sequence motifs; and
   determining a classification of the fractional concentration of clinically-relevant DNA in the biological sample by comparing the aggregate value to one or more calibration values determined from one or more calibration samples whose fractional concentration of clinically-relevant DNA are known.

2. The method of claim 1, wherein the clinically-relevant DNA is selected from a group consisting of fetal DNA, tumor DNA, DNA from a transplanted organ, and a particular tissue type.

3. The method of claim 1, wherein the clinically-relevant DNA is of a particular tissue type.

4. The method of claim 3, wherein the particular tissue type is liver or hematopoietic.

5. The method of claim 1, wherein the one or more calibration values are a plurality of calibration values of a calibration function that is determined using fractional concentrations of clinically-relevant DNA of a plurality of calibration samples.

6. The method of claim 1, wherein the one or more calibration values correspond to one or more aggregate values of the relative frequencies of the set of one or more sequence motifs that are measured using cell-free DNA fragments in the one or more calibration samples.

**7.** The method of claim 1, further comprising:
for each calibration sample of the one or more calibration samples:

measuring the fractional concentration of clinically-relevant DNA in the calibration sample; and
determining the aggregate value of the relative frequencies of the set of one or more sequence motifs by analyzing cell-free DNA fragments from the calibration sample as part of obtaining a calibration data point, thereby determining one or more aggregate values, wherein each calibration data point specifies the measured fractional concentration of clinically-relevant DNA in the calibration sample and the aggregate value determined for the calibration sample, and wherein the one or more calibration values are the one or more aggregate values or are determined using the one or more aggregate values.

**8.** The method of any one of claims 1-7, wherein the set of one or more sequence motifs include N base positions, wherein the set of one or more sequence motifs include all combinations of N bases, and wherein N is an integer equal to or greater than three.

**9.** The method of any one of claims 1-7, wherein the set of one or more sequence motifs are a top M sequence motifs with a largest difference between two types of DNA as determined in one or more reference samples, M being an integer equal to or greater than one.

**10.** The method of any one of claims 1-7, wherein the set of one or more sequence motifs are a top M most frequent sequence motifs occurring in one or more reference samples, M being an integer equal to or greater than one.

**11.** The method of any one of claims 8-10, wherein the set of one or more sequence motifs includes a plurality of sequence motifs, and wherein the aggregate value includes a sum of the relative frequencies of the set.

**12.** The method of any one of claims 1-7, wherein the aggregate value corresponds to a variance in the relative frequencies.

**13.** The method of any one of claims 1-7, wherein the aggregate value includes a final or intermediate output of a machine learning model.

**14.** The method of any one of claims 1-7, wherein determining the sequence motif for each of one or more ending sequences of a cell-free DNA fragment includes:

aligning one or more sequence reads corresponding to the cell-free DNA fragment to a reference genome;
identifying one or more bases in the reference genome that are adjacent to the ending sequence; and
using the ending sequence and the one or more bases to determine the sequence motif.

**15.** A computer program comprising a plurality of instructions that, when executed, control a computer system to perform the method of any one of claims 1-14.

**Patentansprüche**

**1.** Verfahren zum Schätzen einer fraktionellen Konzentration von klinisch relevanter DNA in einer biologischen Probe eines Subjekts, wobei die biologische Probe die klinisch relevante DNA und andere DNA, die zellfrei sind, umfasst, wobei das Verfahren Folgendes umfasst:

Analysieren einer Vielzahl von zellfreien DNA-Fragmenten aus der biologischen Probe, um Sequenzablesungen zu erhalten, wobei die Sequenzablesungen Endsequenzen umfassen, die Enden der Vielzahl von zellfreien DNA-Fragmenten entsprechen;
für jedes der Vielzahl von zellfreien DNA-Fragmenten, Bestimmen eines Sequenzmotivs für jede von einer oder mehreren Endsequenzen des zellfreien DNA-Fragments;
Bestimmen relativer Häufigkeiten eines Satzes von einem oder mehreren Sequenzmotiven, die den Endsequenzen der Vielzahl von zellfreien DNA-Fragmenten entsprechen, wobei eine relative Häufigkeit eines Sequenzmotivs einen Anteil der Vielzahl von zellfreien DNA-Fragmenten bereitstellt, die eine Endsequenz aufweisen, die dem Sequenzmotiv entspricht;
Bestimmen eines Gesamtwerts der relativen Häufigkeiten des Satzes von einem oder mehreren Sequenzmotiven; und
Bestimmen einer Klassifizierung der fraktionellen Konzentration von klinisch relevanter DNA in der biologischen Probe durch Vergleichen des Gesamtwerts mit einem oder mehreren Kalibrierungswerten, die aus einer oder mehreren Kalibrierungsproben bestimmt werden, deren fraktionelle Konzentration von klinisch relevanter DNA bekannt ist.

**2.** Verfahren nach Anspruch 1, wobei die klinisch relevante DNA ausgewählt ist aus einer Gruppe bestehend aus fetaler DNA, Tumor-DNA, DNA aus einem transplantierten Organ und einem bestimmten Gewebetyp.

3. Verfahren nach Anspruch 1, wobei die klinisch relevante DNA von einem bestimmten Gewebetyp ist.

4. Verfahren nach Anspruch 3, wobei der bestimmte Gewebetyp Leber oder hämatopoetisch ist.

5. Verfahren nach Anspruch 1, wobei der eine oder die mehreren Kalibrierungswerte eine Vielzahl von Kalibrierungswerten einer Kalibrierungsfunktion sind, die unter Verwendung von fraktionellen Konzentrationen klinisch relevanter DNA einer Vielzahl von Kalibrierungsproben bestimmt wird.

6. Verfahren nach Anspruch 1, wobei der eine oder die mehreren Kalibrierungswerte einem oder mehreren Gesamtwerten der relativen Häufigkeiten des Satzes von einem oder mehreren Sequenzmotiven entsprechen, die unter Verwendung zellfreier DNA-Fragmente in der einen oder den mehreren Kalibrierungsproben gemessen werden.

7. Verfahren nach Anspruch 1, ferner umfassend:

für jede Kalibrierungsprobe der einen oder mehreren Kalibrierungsproben:
Messen der fraktionellen Konzentration klinisch relevanter DNA in der Kalibrierungsprobe; und
Bestimmen des Gesamtwerts der relativen Häufigkeiten des Satzes von einem oder mehreren Sequenzmotiven durch Analysieren zellfreier DNA-Fragmente aus der Kalibrierungsprobe als Teil des Erhaltens eines Kalibrierungsdatenpunkts, wodurch ein oder mehrere Gesamtwerte bestimmt werden, wobei jeder Kalibrierungsdatenpunkt die gemessene fraktionelle Konzentration von klinisch relevanter DNA in der Kalibrierungsprobe und den für die Kalibrierungsprobe bestimmten Gesamtwert spezifiziert, und wobei der eine oder die mehreren Kalibrierungswerte der eine oder die mehreren Gesamtwerte sind oder unter Verwendung des einen oder der mehreren Gesamtwerte bestimmt werden.

8. Verfahren nach einem der Ansprüche 1-7, wobei der Satz aus einem oder mehreren Sequenzmotiven N Basenpositionen umfasst, wobei der Satz aus einem oder mehreren Sequenzmotiven alle Kombinationen von N Basen umfasst und wobei N eine ganze Zahl gleich oder größer als drei ist.

9. Verfahren nach einem der Ansprüche 1-7, wobei der Satz aus einem oder mehreren Sequenzmotiven die obersten M Sequenzmotive mit dem größten Unterschied zwischen zwei DNA-Typen ist, wie in einer oder mehreren Referenzproben bestimmt, wobei M eine ganze Zahl gleich oder größer als 1 ist.

10. Verfahren nach einem der Ansprüche 1-7, wobei der Satz aus einem oder mehreren Sequenzmotiven die obersten M häufigsten Sequenzmotive ist, die in einer oder mehreren Referenzproben auftreten, wobei M eine ganze Zahl gleich oder größer als 1 ist.

11. Verfahren nach einem der Ansprüche 8-10, wobei der Satz aus einem oder mehreren Sequenzmotiven eine Vielzahl von Sequenzmotiven umfasst und wobei der Gesamtwert eine Summe der relativen Häufigkeiten des Satzes umfasst.

12. Verfahren nach einem der Ansprüche 1-7, wobei der Gesamtwert einer Varianz in den relativen Häufigkeiten entspricht.

13. Verfahren nach einem der Ansprüche 1-7, wobei der Gesamtwert eine End- oder Zwischenausgabe eines maschinellen Lernmodells umfasst.

14. Verfahren nach einem der Ansprüche 1-7, wobei die Bestimmung des Sequenzmotivs für jede von einer oder mehreren Endsequenzen eines zellfreien DNA-Fragments Folgendes umfasst:

Ausrichten einer oder mehrerer Sequenzablesungen, die dem zellfreien DNA-Fragment entsprechen, an einem Referenzgenom;
Identifizieren einer oder mehrerer Basen in dem Referenzgenom, die an die Endsequenz angrenzen; und
Verwenden der Endsequenz und der einen oder mehreren Basen, um das Sequenzmotiv zu bestimmen.

15. Computerprogramm, das eine Vielzahl von Anweisungen umfasst, die bei Ausführung ein Computersystem steuern, um das Verfahren nach einem der Ansprüche 1-14 auszuführen.

**Revendications**

1. Méthode d'estimation d'une concentration fractionnelle d'ADN cliniquement pertinent dans un échantillon biologique d'un sujet, l'échantillon biologique comprenant l'ADN cliniquement pertinent et d'autres ADN qui sont acellulaires, la méthode comprenant :

l'analyse d'une pluralité de fragments d'ADN acellulaire issus de l'échantillon biologique afin d'obtenir des lectures de séquences, dans laquelle les lectures de séquences incluent des séquences d'extrémité correspondant à des extrémités de la pluralité de fragments d'ADN acellulaire ;

pour chacun de la pluralité de fragments d'ADN acellulaire, déterminer un motif de séquence pour chacune d'une ou de plusieurs séquences d'extrémité du fragment d'ADN acellulaire ;

la détermination de fréquences relatives d'un ensemble d'un ou de plusieurs motifs de séquence correspondant aux séquences d'extrémité de la pluralité de fragments d'ADN acellulaire, dans laquelle une fréquence relative d'un motif de séquence fournit une proportion de la pluralité de fragments d'ADN acellulaire qui ont une séquence d'extrémité correspondant au motif de séquence ;

la détermination d'une valeur agrégée des fréquences relatives de l'ensemble d'un ou de plusieurs motifs de séquence ; et

la détermination d'une classification de la concentration fractionnelle d'ADN cliniquement pertinent dans l'échantillon biologique par comparaison de la valeur agrégée à une ou plusieurs valeurs d'étalonnage déterminées à partir d'un ou de plusieurs échantillons d'étalonnage dont la concentration fractionnelle d'ADN cliniquement pertinent est connue.

2. Méthode selon la revendication 1, dans laquelle l'ADN cliniquement pertinent est sélectionné dans un groupe constitué d'ADN fœtal, d'ADN tumoral, d'ADN issu d'un organe transplanté, et d'un type de tissu particulier.

3. Méthode selon la revendication 1, dans laquelle l'ADN cliniquement pertinent est d'un type de tissu particulier.

4. Méthode selon la revendication 3, dans laquelle le type de tissu particulier est hépatique ou hématopoïétique.

5. Méthode selon la revendication 1, dans laquelle les une ou plusieurs valeurs d'étalonnage sont une pluralité de valeurs d'étalonnage d'une fonction d'étalonnage qui est déterminée à l'aide de concentrations fractionnelles d'ADN cliniquement pertinent d'une pluralité d'échantillons d'étalonnage.

6. Méthode selon la revendication 1, dans laquelle les une ou plusieurs valeurs d'étalonnage correspondent à une ou plusieurs valeurs agrégées des fréquences relatives de l'ensemble d'un ou de plusieurs motifs de séquence qui sont mesurées à l'aide de fragments d'ADN acellulaire dans les un ou plusieurs échantillons d'étalonnage.

7. Méthode selon la revendication 1, comprenant en outre :

pour chaque échantillon d'étalonnage des un ou de plusieurs échantillons d'étalonnage :

la mesure de la concentration fractionnelle d'ADN cliniquement pertinent dans l'échantillon d'étalonnage ; et

la détermination de la valeur agrégée des fréquences relatives de l'ensemble d'un ou de plusieurs motifs de séquence par l'analyse de fragments d'ADN acellulaires issus de l'échantillon d'étalonnage dans le cadre de l'obtention d'un point de données d'étalonnage, ce qui permet de déterminer une ou plusieurs valeurs agrégées, dans laquelle chaque point de données d'étalonnage spécifie la concentration fractionnelle mesurée d'ADN cliniquement pertinent dans l'échantillon d'étalonnage et la valeur agrégée déterminée pour l'échantillon d'étalonnage, et dans laquelle les une ou plusieurs valeurs d'étalonnage sont les ou plusieurs valeurs agrégées ou sont déterminées à l'aide des une ou plusieurs valeurs agrégées.

8. Méthode selon l'une quelconque des revendications 1 à 7, dans laquelle l'ensemble d'un ou de plusieurs motifs de séquence inclut N positions de base, dans laquelle l'ensemble d'un ou de plusieurs motifs de séquence inclut toutes les combinaisons de N bases, et dans laquelle N est un nombre entier égal ou supérieur à trois.

9. Méthode selon l'une quelconque des revendications 1 à 7, dans laquelle l'ensemble d'un ou de plusieurs motifs de séquence est constitué de M premiers motifs de séquence ayant la plus grande différence entre deux types d'ADN tels que déterminés dans un ou plusieurs échantillons de référence, M étant un nombre entier supérieur ou égal à un.

10. Méthode selon l'une quelconque des revendications 1 à 7, dans laquelle l'ensemble d'un ou de plusieurs motifs de séquence est constitué de M motifs de séquence les plus fréquents présents dans un ou plusieurs échantillons de référence, M étant un nombre entier supérieur ou égal à un.

11. Méthode selon l'une quelconque des revendications 8 à 10, dans laquelle l'ensemble d'un ou de plusieurs motifs de séquence inclut une pluralité de motifs de séquence, et dans laquelle la valeur agrégée inclut une somme des fréquences relatives de l'ensemble.

12. Méthode selon l'une quelconque des revendications 1 à 7, dans laquelle la valeur agrégée correspond à une variance des fréquences relatives.

13. Méthode selon l'une quelconque des revendications 1 à 7, dans laquelle la valeur agrégée inclut un résultat final ou intermédiaire d'un modèle d'apprentissage automatique.

**14.** Méthode selon l'une quelconque des revendications 1 à 7, dans laquelle la détermination du motif de séquence pour chacune d'une ou de plusieurs séquences d'extrémité d'un fragment d'ADN acellulaire inclut :

l'alignement d'une ou de plusieurs lectures de séquence correspondant au fragment d'ADN acellulaire sur un génome de référence ;
l'identification d'une ou de plusieurs bases dans le génome de référence qui sont adjacentes à la séquence d'extrémité ; et
l'utilisation de la séquence d'extrémité et des une ou plusieurs bases pour déterminer le motif de séquence.

**15.** Programme informatique comprenant une pluralité d'instructions qui, lorsqu'elles sont exécutées, commandent un système informatique pour qu'il réalise le procédé de l'une quelconque des revendications 1 à 14.

Plasma DNA | 110

↓

Paired-end sequencing | 120

↓

Alignment to reference genome | 130

↓

Technique 140 : 4-mer on the ends of a fragment

Sequenced fragment 141

142 ╱ ╲ 144

5' CCCA TCGA 3'

Genome 145

Motif being analyzed

Technique 160 : 4-mer jointly constructed from 2-mer on the ends of a fragment plus 2-mer on the genomic regions adjacent to ends of a fragment

Sequenced fragment 161

162 ╱ ╲ 164

5' CGCC CCGA 3'

Genome 165

Motif being analyzed

*FIG. 1*

# Genotypic difference based approach

Mother   Fetus

Genotypes   A A    A B

Maternal plasma

207    205

Shared sequences    Fetal sequences

220    Frequency (%) — Plasma end 4-mer motifs (256): CCCA, CCAG, CCAA, AAAA, ACTG · · ·

222

# Motif aberration assessment

230   : *entropy based analysis*

$$Entropy = \sum_{i=1}^{256} - P_i * \log(P_i)$$

where $P_i$ is the frequency of a particular motif ; the higher entropy indicate the higher diversity .

Entropy — Shared sequences, Fetal sequences

235

240   : *clustering based analysis*

individuals characterized by end motifs derived from shared sequences     individuals characterized by end motifs derived from fetal-specific sequences

Plasma end 4-mer motifs (256)

242    244

*FIG. 2*

FIG. 3

*FIG. 4*

(All 256 motifs)

FIG. 5A

(Selected 10 motifs)

FIG. 5B

FIG. 6B

610

Heatmap of fetal-specific and shared fragments' all motifs in first-trimester

620

FIG. 6A

EP 4 300 500 B1

Fetal-specific sequences

Shared sequences

All 256 motifs

FIG. 7A    Shared fragments

Fetal-specific fragments    FIG. 7B

FIG. 7C

p-value = 0.74    Shared fragments

10 motifs

p-value = 0.063    Fetal-specific fragments

FIG. 7D

FIG. 8A    Entropy with all fragments

FIG. 8B    Entropy of Y chromosome

FIG. 9

EP 4 300 500 B1

FIG. 10

**FIG. 11**

FIG. 12

FIG. 13

EP 4 300 500 B1

FIG. 14

FIG. 15A

End motifs derived from:
Mutant sequences
Shared sequences

FIG. 15B

Combined 8 motif frequency

Entropy barplot for the deep sequencing case
(HCC case)

FIG. 16A

Entropy barplot for the deep sequencing case
(HCC case top 10 motifs)

FIG. 16B

FIG. 17

FIG. 18A

FIG. 18B

1900

1910 | Analyze a plurality of cell-free DNA fragments from the biological sample to obtain sequence reads

1920 | For each of the plurality of cell-free DNA fragments, determine a sequence motif for each of one or more ending sequences of the cell-free DNA fragment

1930 | Determine relative frequencies of a set of one or more sequence motifs corresponding to the ending sequences of the plurality of cell-free DNA fragments

1940 | Determine an aggregate value of the relative frequencies of the set of one or more sequence motifs

1950 | Compare the aggregate value to one or more calibration values to determine a classification of the fractional concentration of clinically-relevant DNA

**FIG. 19**

2000

2010 | Analyze a plurality of cell-free DNA fragments from the biological sample to obtain sequence reads

2020 | For each of the plurality of cell-free DNA fragments, determine a sequence motif for each of one or more ending sequences of the cell-free DNA fragment

2030 | Determine relative frequencies of a set of one or more sequence motifs corresponding to the ending sequences of the plurality of cell-free DNA fragments

2040 | Determine an aggregate value of the relative frequencies of the set of one or more sequence motifs

2050 | Obtain one or more calibration data points

2070 | Compare the aggregate value to a calibration value of at least one calibration data point

2080 | Estimate a gestational age of the fetus based on the comparing

**FIG. 20**

FIG. 21

FIG. 22

FIG. 23A

FIG. 23B

FIG. 24A

FIG. 24B

FIG. 25A    All 256 4-mer motifs

FIG. 25B    Entropy of selected 10 motifs

FIG. 26A

FIG. 26B

EP 4 300 500 B1

FIG. 27A

FIG. 27B

60

FIG. 28

*FIG. 29*

*FIG. 30*

*FIG. 31*

*FIG. 32*

FIG. 33

FIG. 34

FIG. 35A

FIG. 35B

Entropy of SLE samples (top 10 motifs)

*FIG. 36*

FIG. 37

FIG. 38A

FIG. 38B

*FIG. 39*

FIG. 40

*FIG. 41*

4200

4210 — Analyze a plurality of cell-free DNA fragments from the biological sample to obtain sequence reads

4220 — For each of the plurality of cell-free DNA fragments, determine a sequence motif for each of one or more ending sequences of the cell-free DNA fragment

4230 — Determine relative frequencies of a set of one or more sequence motifs corresponding to the ending sequences of the plurality of cell-free DNA fragments

4240 — Determine an aggregate value of the relative frequencies of the set of one or more sequence motifs

4250 — Determine a classification of a level of pathology for the subject based on a comparison of the aggregate value to a reference value

*FIG. 42*

4300

4310 — Receive a plurality of cell-free DNA fragments from the biological sample

4320 — Subject the plurality of cell-free DNA fragments to one or more probe molecules that detect the sequence motifs in the ending sequences of the plurality of cell-free DNA fragments

4330 — Use the detected DNA fragments to enrich the biological sample for the clinically-relevant DNA fragments

*FIG. 43*

4400

4410 | Analyze a plurality of cell-free DNA fragments from the biological sample to obtain sequence reads

4420 | For each of the plurality of cell-free DNA fragments, determine a sequence motif for each of one or more ending sequences of the cell-free DNA fragment

4430 | Identify a set of sequence motif(s) that occur in the clinically-relevant DNA at a relative frequency greater than the other DNA

4440 | Identify a group of the sequence reads that have the set of one or more sequence motifs in ending sequences

4450 | Store sequence reads having a likelihood of corresponding to the clinically-relevant DNA exceeding a threshold, as determined using the set of end motif(s)

4460 | Analyze the stored sequence reads to determine a property of the clinically-relevant DNA the biological sample

FIG. 44

FIG. 45

**FIG. 46**

| I/O CONTROLLER 71 | SYSTEM MEMORY 72 | CENTRAL PROCESSOR 73 | PRINTER 74 | Data Collection Device 85 |

75

| DISPLAY ADAPTER 82 | I/O PORT 77 | KEYBOARD 78 | STORAGE DEVICE(S) 79 | EXTERNAL INTERFACE 81 |

MONITOR 76

10

**FIG. 47**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62782316
- WO 2018081130 A
- US 20140100121 A
- US 20130237431 A
- US 20140080715 A
- US 20130040824 A
- US 20170024513 A
- US 20190341127 A
- US 519912

**Non-patent literature cited in the description**

- **SUN et al.** *Proc Natl Acad Sci USA.*, 2015, vol. 112, E5503-12
- **LEHMANN-WERMAN et al.** *Proc Natl Acad Sci USA.*, 2016, vol. 113, E1826-34
- **MOSS et al.** *Nat Commun.*, 2018, vol. 9, 5068
- **LO et al.** *Sci Transl Med.*, 2010, vol. 2, 61-91
- **JIANG et al.** *Proc Natl Acad Sci USA.*, 2015, vol. 112, E1317-25
- **CHAN et al.** *Proc Natl Acad Sci USA.*, 2016, vol. 113, E8159-8168
- **JIANG et al.** *Proc Natl Acad Sci USA.*, 2018
- **CHANDRANANDA et al.** *BMC Med Genomics*, 2015, vol. 8, 29
- **BAILEY.** *Bioinformatics*, 2011, vol. 27, 1653-9
- **LO et al.** *Am J Hum Genet.*, 1998, vol. 62, 768-775
- **LUN et al.** *Clin Chem.*, 2008, vol. 54, 1664-1672
- **SCHREIBER et al.** *Proc Natl Acad Sci USA.*, 2013, vol. 110, 18910-18915
- **FLUSBERG et al.** *Nat Methods.*, 2010, vol. 7, 461-465
- **CHANDRANANDA et al.** *MC Med Genomics*, 2015, vol. 8, 29
- **GUO et al.** *Nat Genet.*, 2017, vol. 49, 635-42